# EUROPEAN PATENT SPECIFICATION

(11) **EP 2 859 002 B1**
(45) Date of publication and mention of the grant of the patent: **30.12.2020**
(21) Application number: 13800272.0
(22) Date of filing: 07.06.2013
(51) Int. Cl.: C07D 295/033, C07F 7/18

(54) **SILICON-BASED CROSS COUPLING AGENTS AND METHODS OF THEIR USE**
SILIKONBASIERTE KREUZKUPPLUNGSMITTEL UND VERFAHREN ZU DEREN ANWENDUNG
AGENTS DE COUPLAGE À BASE DE SILICIUM ET LEURS PROCÉDÉS D'UTILISATION

(30) Priority: 08.06.2012 US 201261657309 P; 01.04.2013 US 201361807192 P
(43) Date of publication of application: 15.04.2015
(73) Proprietor: The Trustees of The University of Pennsylvania, Philadelphia, PA 19104 (US)
(72) Inventor: HOYE, Adam, T., Philadelphia, PA 19128 (US); KIM, Won-suk, Seoul 130-101 (KR); MARTINEZ-SOLORIO, Dionicio, Bala Cynwyd, PA 19004 (US); SMITH, Amos, B., Merion, PA 19066 (US); SANCHEZ, Luis, Philadelphia, PA 19104 (US); TONG, Rongbiao, Hong Kong (CN); NGUYEN, Minh, Huu, Philadelphia, PA19104 (US)
(74) Representative: Dörries, Hans Ulrich
(86) International application number: PCT/US2013/044689
(87) International publication number: WO 2013/185021

(56) References cited:
- US-A- 4 447 628
- US-A1- 2009 069 577
- YOUSEF M. HIJJI ET AL: "Rearrangement of o-(chloromethyldimethylsilyl)phenylmethoxi de: evidence for an apical position of the migrating group in a trigonal bipyramid intermediate", CHEM. COMMUN., 1998, pages 1213-1214, XP002745162,
- YOSHIAKI NAKAO ET AL: "Cross-Coupling Reactions through the Intramolecular Activation of Alkyl(triorgano)silanes", ANGEW. CHEM. INT. ED., vol. 49, 2010, pages 4447-4450, XP002745163,
- ERIC M. SIMMONS ET AL: "Iridium-Catalyzed Arene Ortho -Silylation by Formal Hydroxyl-Directed C-H Activation", JOURNAL OF THE AMERICAN CHEMICAL SOCIETY, vol. 132, no. 48, 15 November 2010 (2010-11-15), pages 17092-17095, XP55103551, ISSN: 0002-7863, DOI: 10.1021/ja1086547
- SHI TANG ET AL: "Nickel-catalysed cross-coupling reaction of aryl(trialkyl)silanes with aryl chlorides and tosylates", CHEMICAL COMMUNICATIONS, vol. 47, no. 1, 2011, pages 307-309, XP55216813, ISSN: 1359-7345, DOI: 10.1039/C0CC02173C
- AMOS B. SMITH ET AL: "Unification of Anion Relay Chemistry with the Takeda and Hiyama Cross-Coupling Reactions: Identification of an Effective Silicon-Based Transfer Agent", JOURNAL OF THE AMERICAN CHEMICAL SOCIETY, vol. 134, no. 10, 14 March 2012 (2012-03-14), pages 4533-4536, XP55216795, ISSN: 0002-7863, DOI: 10.1021/ja2120103
- MARCUCCIO SEBASTIAN M ET AL: "Improved Hiyama cross-coupling reactions using HOMSi(R)1reagents: a novel application of a palladacycle", TETRAHEDRON LETTERS, PERGAMON, GB, vol. 52, no. 52, 29 October 2011 (2011-10-29), pages 7178-7181, XP028596966, ISSN: 0040-4039, DOI: 10.1016/J.TETLET.2011.10.126
- KENTA SHIMIZU ET AL: "Polyarylene Synthesis by Cross-Coupling with HOMSi Reagents", CHEM. LETT., vol. 42, 22 December 2012 (2012-12-22), pages 45-47, XP002745164,
- WATARU ANDO ET AL: "Evidence of the formation of oxasilacyclopropane from the reaction of silylene with ketone", JOURNAL OF THE AMERICAN CHEMICAL SOCIETY, vol. 99, no. 19, September 1977 (1977-09), pages 6447-6449, XP55216774, ISSN: 0002-7863, DOI: 10.1021/ja00461a050
- Yohsuke Yamamoto ET AL: "Oo Printed in Great Britain Pergamon Press plc INTRAMOLECULAR CYCLIZATION OF o-SILYLBENZYL ALCOHOLS WITH ELIMINATION OF HYDROCARBON VIA HYPERVALENT SILICON INTERMEDIATES: EFFECT OF STRUCTURE ON THE SELECTIVITY FOR ELIMINATION", , 1989, pages 725-728, XP55216767, Retrieved from the Internet: URL:http://www.sciencedirect.com/science/a rticle/pii/S0040403901802938/pdf?md5=851cd f430cb7a6814ac409f5dc5628d1&pid=1-s2.0-S00 40403901802938-main.pdf [retrieved on 2015-09-29]
- SCOTT M. SIEBURTH ET AL: "An intramolecular Diels-Alder reaction of vinylsilanes", THE JOURNAL OF ORGANIC CHEMISTRY, vol. 57, no. 20, 1992, pages 5279-5281, XP55216766, ISSN: 0022-3263, DOI: 10.1021/jo00046a002
- DATABASE CAPLUS [Online] CHEMICAL ABSTRACTS SERVICE, COLUMBUS, OHIO, US; KANG, KYUNG-TAE ET AL: "Synthesis of 4-substituted 1,1-di-tert- butylbenzosilacyclobutenes", XP002745165, retrieved from STN Database accession no. 1985:615377 & KANG, KYUNG-TAE ET AL: "Synthesis of 4-substituted 1,1-di-tert- butylbenzosilacyclobutenes", CHEMISTRY LETTERS , (5), 617-20 CODEN: CMLTAG; ISSN: 0366-7022, 1985, DOI: 10.1246/CL.1985.617 10.1246/CL.1985.617
- DATABASE CAPLUS [Online] CHEMICAL ABSTRACTS SERVICE, COLUMBUS, OHIO, US; CHEN, JINSHUI ET AL: "Synthesis of biaryls and oligoarenes using aryl[2-(hydroxymethyl)phenyl]dimethylsilan es", XP002745166, retrieved from STN Database accession no. 2010:756389 & CHEN, JINSHUI ET AL: "Synthesis of biaryls and oligoarenes using aryl[2-(hydroxymethyl)phenyl]dimethylsilan es", BULLETIN OF THE CHEMICAL SOCIETY OF JAPAN , 83(5), 554-569 CODEN: BCSJA8; ISSN: 0009-2673, 2010, DOI: 10.1246/BCSJ.20090325 10.1246/BCSJ.20090325
- HANDY, C. ET AL.: 'Recent advances in siloxane-based aryl-aryl coupling reactions: focus on heteroaromatic systems' TETRAHEDRON vol. 61, no. 744, 08 September 2005, pages 12201 - 12225, XP027860659
- SURRY, DS ET AL.: 'Biaryl PhosphineLigands in Palladium-Catalyzed Amination' ANGEW CHEM INT ED ENGL. vol. 47, no. 34, 2008, pages 6338 - 6361, XP055038146

## Description

### REFERENCE TO RELATED APPLICATIONS

This application claims the benefit of U.S. Provisional Application No. 61/657,309, filed June 8, 2012 and U.S. Provisional Application No. 61/807,192, filed April 1, 2013.

### GOVERNMENT RIGHTS

At least a portion of this work was supported by a grant from the National Institutes of Health through Grant GM-29028. Pursuant to 35 U.S.C. § 202, the government may have certain rights in the invention.

### TECHNICAL FIELD

The present invention is directed to silicon-based cross-coupling agents and methods of using them in cross-coupling reactions.

### BACKGROUND

Cross-coupling reactions ("CCRs") of organometallic/main group reagents with, for example, organic halides permit the construction of carbon-carbon and carbon-nitrogen bonds. Although CCRs are atom-efficient processes, the known CCRs have drawbacks, including the undesired formation of homo-coupled products and the use of toxic metals. As such, new methods for the cross coupling of organic compounds to form new carbon-carbon bonds and new carbon-nitrogen bonds are needed.

Cyclic silicon compounds and their use in the context of cross-coupling reactions are disclosed in J. Am. Chem. Soc., 2012, 134(10), 4533-4536, Chem. Comm. 2011, 47(1), 307-309, Tet. Lett. 2011, 52, 7178-7181 and in US2009/069577.

### SUMMARY

The present invention is directed to compounds of formula I: 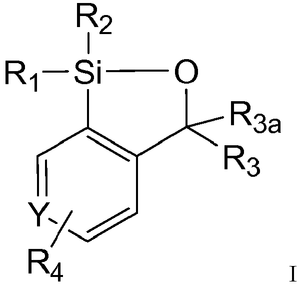
wherein Y is CH or N;
R₁ and R₂ are independently; methyl, propyl, or isopropyl, optionally wherein R₁ and R₂ are each methyl or isopropyl;
R₃ is
   aryl substituted with one or more nitro, diC₁₋₆alkylamino; C₁₋₆alkoxy, or C₁₋₆alkyl;
   heteroaryl optionally substituted with one or more nitro, diC₁₋₆alkylamino; C₁₋₆alkoxy, or C₁₋₆alkyl;
   C₁₋₁₀ straight or branched-chain alkyl optionally substituted with one halogen, nitro, C₁₋₆alkoxy, or aryl;
   a polymer; or
   a resin support;
R₃ₐ is H or CF₃; and
at least one R₄, wherein each R₄ is independently hydrogen, halogen, nitro, C₁₋₆alkoxy, C₁₋₆alkyl, aryl, or a resin support; and wherein R₃ is a polymer or a resin support, and/or R₄ is a resin support.

Use of the compounds of formula I as silicon-based cross-coupling agents in the formation of carbon-carbon and carbon-nitrogen bonds is also described.

### DETAILED DESCRIPTION OF ILLUSTRATIVE EMBODIMENTS

The present invention is directed to compounds of formula I for use as silicon-based cross-coupling agents in the formation of carbon-carbon and carbon-nitrogen bonds: 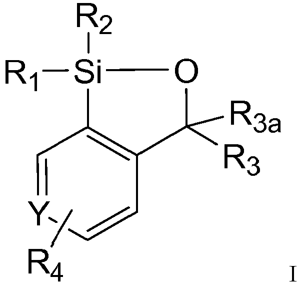 wherein
Y is CH or N;
R₁ and R₂ are independently; methyl, propyl, or isopropyl, optionally wherein R₁ and R₂ are each methyl or isopropyl;
R₃ is
   aryl substituted with one or more nitro, diC₁₋₆alkylamino, C₁₋₆alkoxy, or C₁₋₆alkyl;
   heteroaryl optionally substituted with one or more nitro, diC₁₋₆alkylamino, C₁₋₆alkoxy, or C₁₋₆alkyl;
   C₁₋₁₀ straight or branched-chain alkyl optionally substituted with one halogen, nitro, C₁₋₆alkoxy, or aryl;
   a polymer; or
   a resin support;
R₃ₐ is H or CF₃; and
at least one R₄, wherein each R₄ is independently hydrogen, halogen, nitro, C₁₋₆alkoxy, C₁₋₆alkyl, aryl, or a resin support; and wherein R₃ is a polymer or a resin support, and/or R₄ is a resin support.

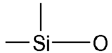

The reactivity of the compound of formula I can be attenuated by manipulation of R₁ and R₂. For example, in preferred embodiments, R₁ and R₂ are independently methyl, *n*-propyl, or isopropyl. Preferably, R₁ and R₂ are independently methyl or isopropyl. In exemplary embodiments, R₁ and R₂ are each methyl. In other embodiments, R₁ and R₂ are each isopropyl.

The reactivity of the compound of formula I can be attenuated by manipulation of R₃. For example, in some embodiments, R₃ is C₁₋₁₀, preferably C₁₋₆, straight or branched-chain alkyl optionally substituted with one halogen or C₁₋₆alkoxy. Preferably, R₃ is C₁₋₄ straight or branched-chain alkyl optionally substituted with one halogen or C₁₋₆alkoxy. More preferably, R₃ is C₁₋₄ straight or branched-chain alkyl. In exemplary embodiments, R₃ is *n*-butyl, isobutyl, *sec*-butyl, or *tert*-butyl*.* In most preferred embodiments, R₃ is n-butyl.

In other embodiments, R₃ is aryl, preferably phenyl, substituted with one or more nitro, diC₁₋₆alkylamino, C₁₋₆alkoxy, or C₁₋₆alkyl. Preferably, R₃ is phenyl substituted with one or more diC₁₋₆alkylamino, C₁₋₆alkoxy, or C₁₋₆alkyl.

In still other embodiments, R₃ is heteroaryl, preferably pyridyl, optionally substituted with one or more nitro, diC₁₋₆alkylamino, C₁₋₆alkoxy, or C₁₋₆alkyl. Preferably, R₃ is pyridyl optionally substituted with one or more diC₁₋₆alkylamino, C₁₋₆alkoxy, or C₁₋₆alkyl. In exemplary embodiments, R₃ is pyridyl.

In other preferred embodiments, R₃ is a polymer. Preferably, this polymer acts as a solid support for the siloxane transfer agent. Such polymers are known in the art *per se.* Preferred polymers are described herein.

In still other embodiments, R₃ is a resin support.

In the invention, R₃ₐ is H or CF₃. Preferably, R₃ₐ is H.

The reactivity of the compound of formula I can be attenuated by manipulation of R₄. The compounds of formula I can include one, two, three, or four R₄ groups, which can each be the same or different. R₄ is preferably hydrogen, halogen, nitro, C₁₋₆alkoxy, C₁₋₆alkyl, or aryl. More preferably, R₄ is hydrogen, halogen, for example F, C₁₋₆alkoxy, or C₁₋₆alkyl. In exemplary embodiments, R₄ is hydrogen. In other embodiments, R₄ is halogen, for example, F, Cl, or Br, with F being particularly preferred. R₄ can also be a resin support.

In those embodiments wherein the compound of formula I include a resin support, the resin support is at either R₃ or R₄. That is, in certain embodiments wherein R₃ is a resin support, each R₄ is independently hydrogen, halogen, nitro, C₁₋₆alkoxy, C₁₋₆alkyl, or aryl. In those embodiments wherein R₄ is a resin support, R₃ is H; aryl optionally substituted with one or more halogen, nitro, diC₁₋₆alkylamino, C₁₋₆alkoxy, or C₁₋₆alkyl; heteroaryl optionally substituted with one or more halogen, nitro, diC₁₋₆alkylamino, C₁₋₆alkoxy, or C₁₋₆alkyl; C₁₋₁₀ straight or branched-chain alkyl optionally substituted with one or more halogen, nitro, C₁₋₆alkoxy, or aryl; or a polymer.

It is preferred that the aromatic moiety of the compounds of formula I is phenyl, that is, wherein Y is CH or CR₄. In such embodiments, preferred compounds of formula I include, for example: 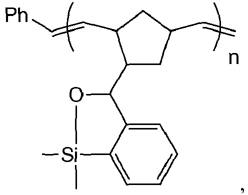 wherein n is about 150 to about 300, preferably about 200 or 250, 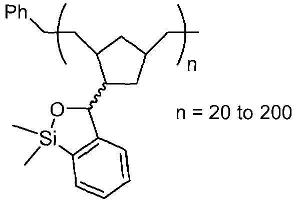 "polymer-supported siloxane transfer agent (saturated)," for example, having 20 to 200 repeating units, 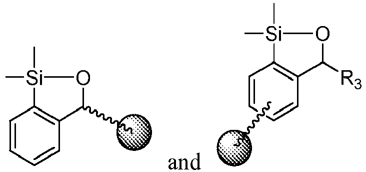 "resin-supported siloxane transfer agent."

Exemplary embodiments of the invention wherein R₃ is a polymer include compounds of the formula: 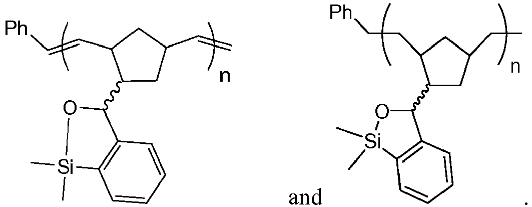 wherein n is independently about 20 to 300, preferably 20 to 200, or 150 to about 300. Most preferably, n is about 200 or 250.

Exemplary embodiments of the invention wherein R₃ or R₄ is a resin support include:

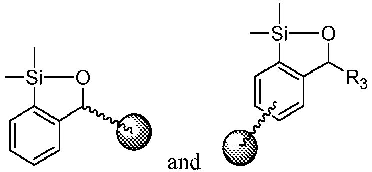

In other embodiments, the aromatic moiety of the compounds of formula I is pyridyl, that is, wherein Y is N.

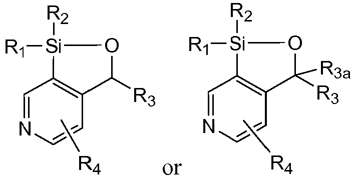

Based on its physico-chemical properties, a compound of formula I including a pyridyl group may facilitate removal of the cross-coupling transfer agent from the products of the cross-coupling reaction mixutre by treatment of the crude reaction mixture with Bronsted or Lewis acids upon workup.

In accordance to the present invention, and as described above and below, the siloxane motif maybe incorporated into polymers using monomers derived from the silicon transfer agent. This polymeric material may improve the ease of purification following the cross-coupling reaction, and may make the transfer agent easier to handle by altering its physical properties.

The invention is also directed to methods of cross-coupling compounds of formula NuLi with a compound of formula E-X to form a compound of formula Nu-E. These methods comprise contacting the compound of formula NuLi with the compound of formula E-X in the presence of a supported siloxane compound of the invention as described herein, a catalyst or a catalyst system, and an ethereal solvent, for a time and under conditions sufficient to produce the compound of formula Nu-E. In these methods, Nu is an aryl compound or an alkenyl compound; E is an aryl compound or an alkenyl compound; and X is iodo or bromo or X is iodo, chloro, or bromo.

The invention is also directed to methods of cross-coupling compounds of formula Nu-Li with a compound of formula E-X to form a compound of formula Nu-E. These methods comprise contacting the compound of formula NuLi with the compound of formula E-X in the presence of a siloxane compound of the invention as described herein, a catalyst or a catalyst system, and an ethereal solvent, for a time and under conditions sufficient to produce the compound of formula Nu-E. In these methods, Nu is an aryl compound or an alkeneyl compound; E is a disubstituted amine, and X is -O-benzoyl.

As used herein, "aryl compound" refers to an organic compound comprising a phenyl or naphthyl group that is optionally substituted with one or more substitutents. Examples of substitutents include alkyl, aryl, halogen, nitro, cyano, ester, alkoxy, siloxy.

As used herein, "alkenyl compound" refers to an organic compound comprising a carbon-carbon double bond that is optionally substituted with one or more substitutents. Examples of substitutents include alkyl, aryl, halogen, nitro, cyano, ester, alkoxy, siloxy.

As used herein, "C₁₋₁₀ straight or branched-chain alkyl" refers to an aliphatic hydrocarbon including from 1 to 10 carbon atoms. Examples include methyl, ethyl, n-propyl, iso-propyl, n-butyl, iso-butyl, tert-butyl, n-penyl, iso-pentyl, n-hexyl.

As used herein, "diC₁₋₆alkylamino" refers to an amino moiety substituted with two C₁₋₆alkyl group. The C₁₋₆alkyl groups can be the same or different. Preferred diC₁₋₆alkylamino groups for use in the invention include dimethylamino, diethylamino, methylethylamino, diisopropylamino.

As used herein, "disubstituted amine" refers to a nitrogen atom having two substituents, which are the same or different. Disubstituted amine also refers to compounds wherein the two substituents are joined to form a ring. Examples of disubstituted amines include (Bn)₂N-, (Et)₂N-,

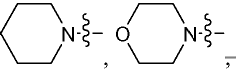

As used herein, "aryl" refers to an aromatic 6-13 membered mono- or bi-cyclic ring such as phenyl or naphthyl.

As used herein, "halogen" refers to iodo, bromo, chloro, or fluoro.

As used herein, "heteroaryl" refers to a mono-or bicyclic aromatic ring structure including carbon atoms as well as up to four heteroatoms selected from nitrogen, oxygen, and sulfur. Heteroaryl rings can include a total of 5, 6, 9, or 10 ring atoms. Preferred heteroaryl groups include pyridyl and pyrimidinyl.

As used herein, "resin support" refers to solid supports used in, for example, combinatorial chemistry. "Wang resins" and "Merrifield resin" are examples of such resins. Resin supports and their use and incorporation are known to those skilled in the art, *per se.*

The compounds of formula I can be used in organic synthesis, most preferably as silicon-based cross-coupling agents. In exemplary embodiments, the compound of formula I is used in the presence of, for example, an organo-lithium compound and an organo-halogen compound to form the resulting cross-coupled organic compound. *See, e.g.,* Scheme 1 for an example using a silicon compound not according to the present invention. Preferably, the organo-lithium compound is an aryl-lithium or alkenyl-lithium compound. The organo-halogen compound is preferably an aryl-halogen compound or an alkenyl-halogen compound. In such embodiments, the halogen is preferably iodo, chloro, or bromo. The resulting cross-coupled organic compounds are thus aryl-aryl compounds, aryl-alkenyl, or alkenyl-alkenyl compounds.

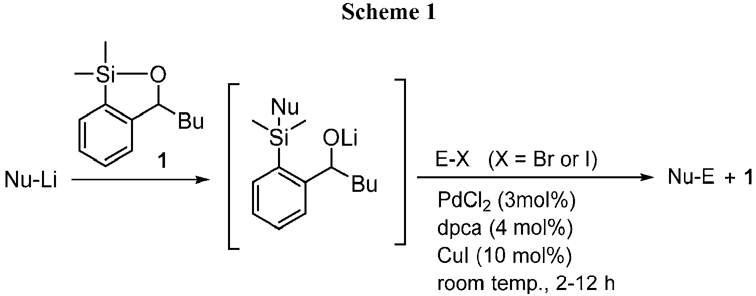

In such cross-coupling reactions, the compounds of formula I can be used in stoichiometric amounts, that is, one molar equivalent, as compared to either the organo-lithium or organo-halogen compound. In some embodiments, the compounds of formula I are used in greater than stoichiometric amounts, that is, greater than one molar equivalent as compared to either the organo-lithium or organo-halogen compound. In such embodiments, up to three molar equivalents of the compound of formula I, as compared to either the organo-lithium or organo-halogen compound, can be used. Within the scope of the invention, it is envisioned that 1.1, 1.2, 1.3, 1.4, 1.5, 1.6, 1.8, 2.0, 2.2, 2.5, or 2.8 molar equivalents of the compound of formula I, as compared to either the organo-lithium or organo-halogen compound, can be used. It is preferred to use 1.6, 1.8, or 2.0 molar equivalents of the compound of formula I. Most preferred is 1.8 molar equivalents of the compound of formula I. The amount of the compound of formula I required for cross-coupling a particular organo-lithium compound with a particular organo-halogen compound can be determined without undue experimentation by someone of skill in the art.

Alternatively, the compound of formula I can be used in catalytic amounts, that is, less than one molar equivalent, as compared to either the organo-lithium or organo-halogen compound.

In some embodiments, cross-coupling of an organo-lithium compound with an organo-halogen compound in the presence of the silicon-based cross-coupling agent compounds of formula I of the invention requires the use of a catalyst or catalyst system. Preferred catalysts or catalyst systems comprise palladium compounds, copper compounds, nickel compounds, or mixtures thereof, optionally in the presence of a compound such as cyclohexyl-(2-diphenylphosphanyl-benzylidene)-amine (dpca), XPhos (2-dicyclohexylphosphino-2',4',6'-triisopropylbiphenyl), or Johnphos ((2-biphenyl)di-*tert*-butylphosphine):

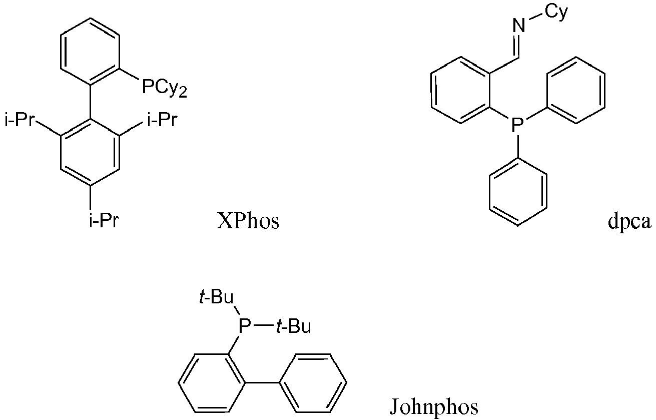

Preferably, the palladium compound is PdCl₂, Pd(PPh₃)₄, or Pd(OAc)₂. The copper compound is preferably a copper halide, for example, CuI. A preferred catalyst system for use in the methods of the invention comprises PdCl₂, CuI, and dpca. Another preferred catalyst system includes Pd(OAc)₂ and XPhos. Yet another catalyst system includes CuI and dpca. Another catalyst system include CuI and Johnphos.

Those of skill in the art can readily determine the mole percentage of the catalyst or each component of the catalyst system for use in the methods of the invention. For example, the catalyst or component of the catalyst system can be present in an amount of from about 1 mol% to about 15 mol%. Alternatively, the catalyst or component of the catalyst system can be present in an amount of from about 3 mol% to about 10 mol%.

In embodiments incorporating the use of a palladium compound as the catalyst or component of the catalyst system, the palladium compound is present in an amount of from about 1 mol% to about 5 mol% or to about 10 mol%. Preferably, the palladium compound, for example, PdCl₂, is present at about 3 mol%.

In embodiments incorporating the use of a copper compound as the catalyst or component of the catalyst system, the copper compound is present in an amount of from about 0.1 mol% to about 10 mol%. In other embodiments, the copper compound is present in an amount of from about 1 mol% to about 10 mol%. Alternatively, the copper compound is present in an amount of from about 5 mol% to about 12 mol%. More preferably, the copper compound is present in an amount of from about 9 mol% to about 11 mol%. Most preferably, for example when the copper compound is CuI, it is present at about 10 mol%.

In those embodiments incorporating dpca in the catalyst systems of the invention, the dpca is present from about 1 mol% to about 10 mol%. Preferably, the dpca is present in an amount from about 2 mol% to about 8 mol%. Particularly preferred are embodiments wherein the dpca is present at about 4 mol%. In those embodiments employing dpca, the dpca is used in conjunction with PdCl₂. Dpca can also be used in conjunction with CuI. Preferably, the dpca is present in an amount greater than the amount of PdCl₂ or CuI. For example, the dpca is present at about 4 mol% and the PdCl₂ is present at about 3 mol%.

In those embodiments incorporating XPhos in the catalyst systems of the invention, the XPhos is present from about 1 mol% to about 50 mol%. Preferably, the XPhos is present in an amount from about 10 mol% to about 30 mol%. Particularly preferred are embodiments wherein the XPhos is present at about 20 mol%.

In embodiments incorporating Johnphos in the catalyst systems of the invention, the Johnphos is present from about 1 mol% to about 20 mol%. Preferably, the XPhos is present in an amount from about 5 mol% to about 15 mol%. Particularly preferred are embodiments wherein the XPhos is present at about 10 mol%.

Preferred solvents for use in the cross-coupling reactions of the invention include ethereal solvents, for example tetrahydrofuran (THF), tetrahydropyran (THP), diisopropyl ether and diethyl ether, with THF being particularly preferred.

Siloxanes of the invention can be prepared according to the methods described herein. Two exemplary methods are shown in Schemes 2A and 2B (although the compounds prepared below are not according to the present invention). Other siloxanes within the scope of the invention can be prepared similarly.

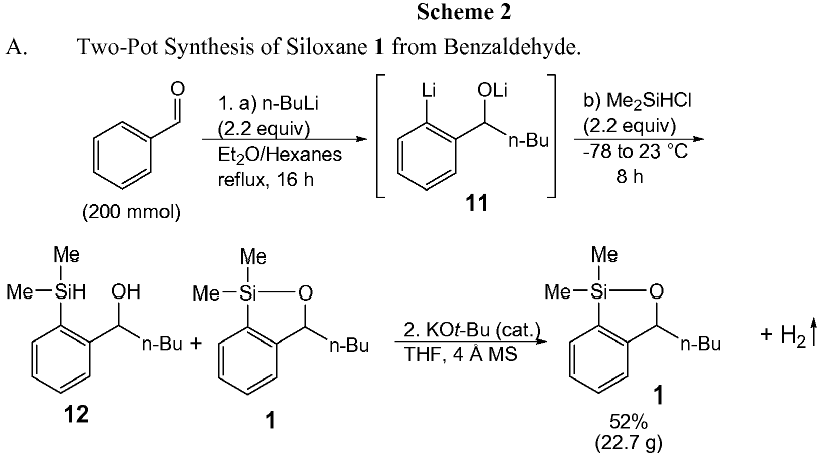

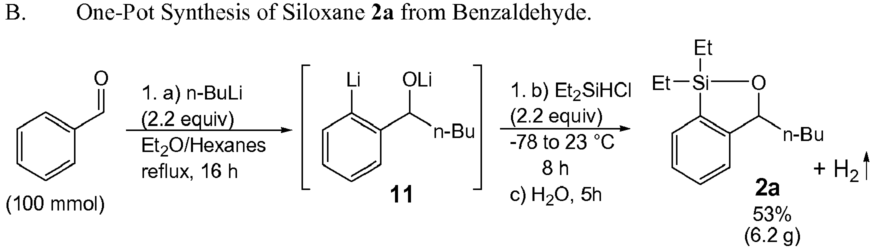

Cyclic silicon compounds have been successfully employed to cross-couple a variety of organo-lithium and organo-halogen compounds, while limiting the amount of homo-coupled product. For example, Scheme 3 depicts the results of several experiments where the organo-halogen compound was 4-iodoanisole and the organo-lithium compound was phenyl lithium.

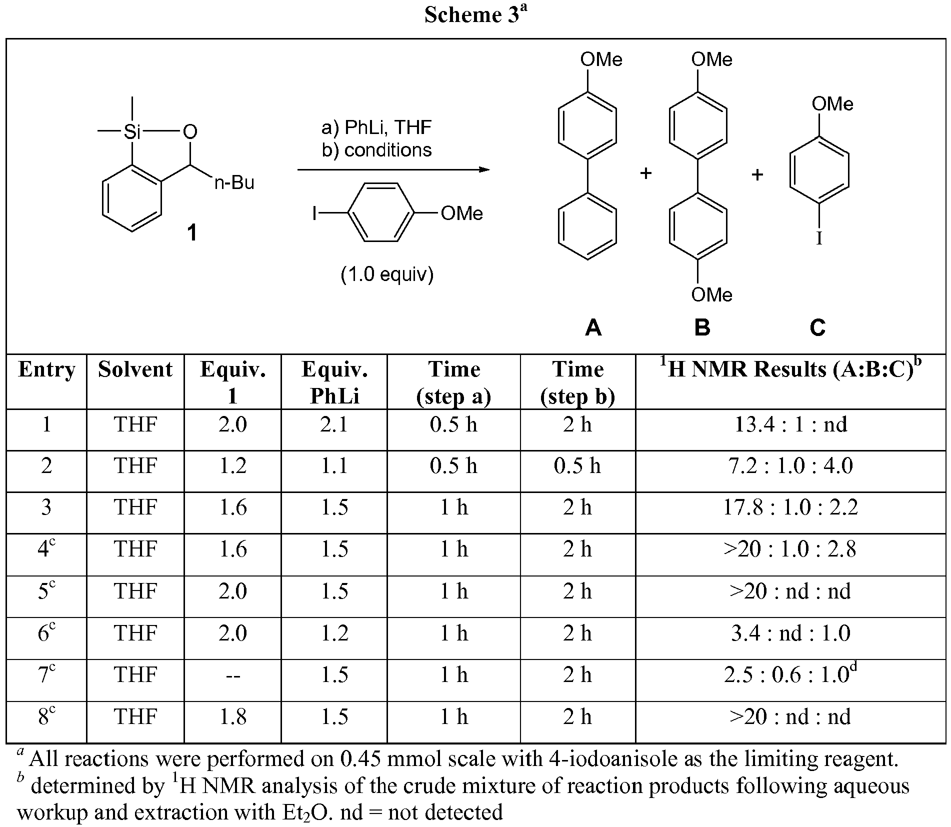

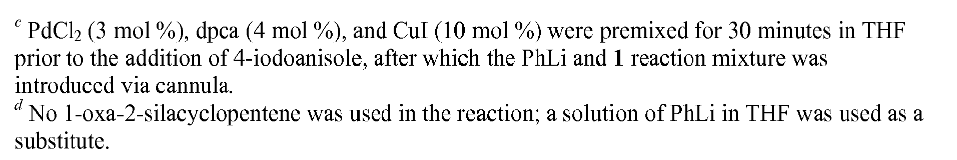

Good conversion of the aryl iodide occurred when steps a and b were allowed to proceed for longer times (see entry 3). A significant increase in the efficiency of the process was observed when the catalyst system of PdCl₂, dpca, and CuI was premixed for about 30 minutes in THF at room temperature prior to introduction of the aryl halide, which was followed by immediate addition of a mixture of PhLi and **1** in THF (see entry 4). Using this protocol in conjunction with the use of 2.0 molar equivalents of compound **1** led to complete conversion of 4-iodoanisole with no detectable homocoupled product. Notably, significant homocoupling and catalyst decomposition was observed in the absence of silicon cross coupling agent **1** (see entry 7). The best results for these reagents, as shown in Scheme 3, resulted using 1.8 molar equivalents of compound **1** and 1.5 molar equivalents of PhLi.

As shown in Scheme 4, organo-halides other than 4-iodoanisole can be used in cross-coupling reactions of the invention using the silicon cross-coupling agents.

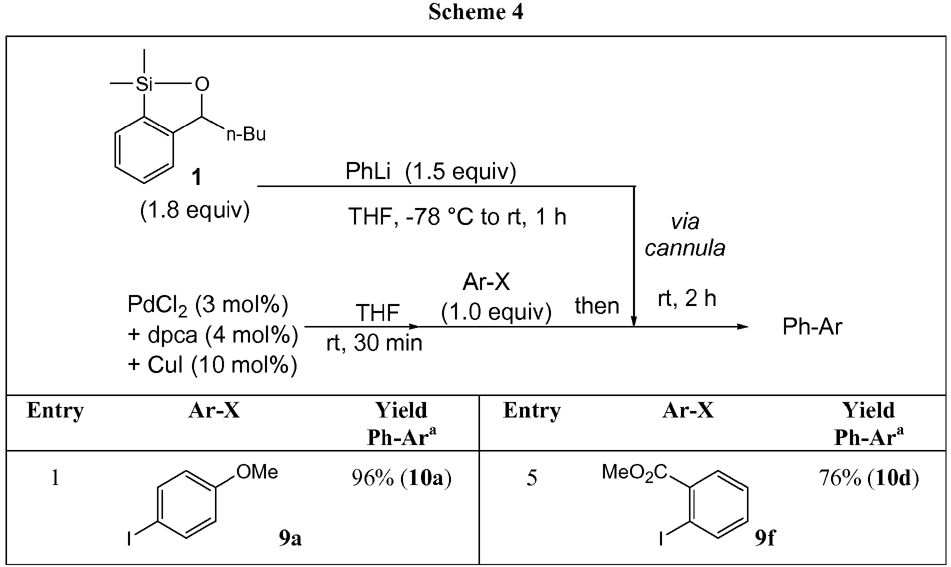

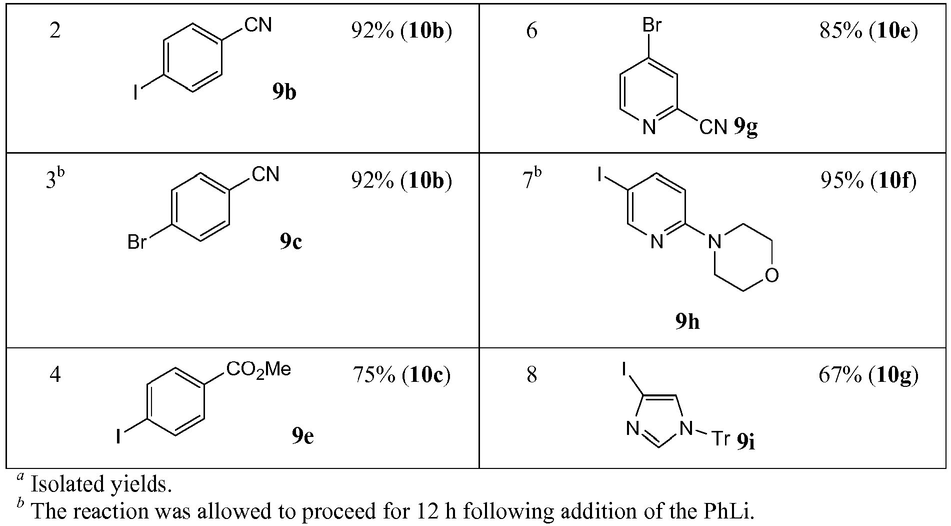

As demonstrated in Scheme 4, electron-rich and electron-deficient substrates were well-tolerated in the reaction, as were a variety of common functional groups, for example, ester, nitriles, and azoheterocycles, to provide the biaryl compounds **10a-g** in good yields. In all cases, silicon cross-coupling agent **1**, which is a compound of formula I of the invention, emerged from the reaction intact, as observed by ¹H NMR analysis of the crude reaction mixtures. For products possessing polarities similar to that of compound **1**, an oxidative Tamao-Fleming workup (Simmons, E.M. et al., J. Am. Chem. Soc. 2010, 132, 17092; Tamao, K. et al. Organometallics 1983, 2, 1694; Fleming, I. et al., J. Chem. Soc. Chem. Commun. 1984, 29) of the initial reaction mixture was used to convert **1** to the corresponding diol, which could be easily separated by column chromatography.

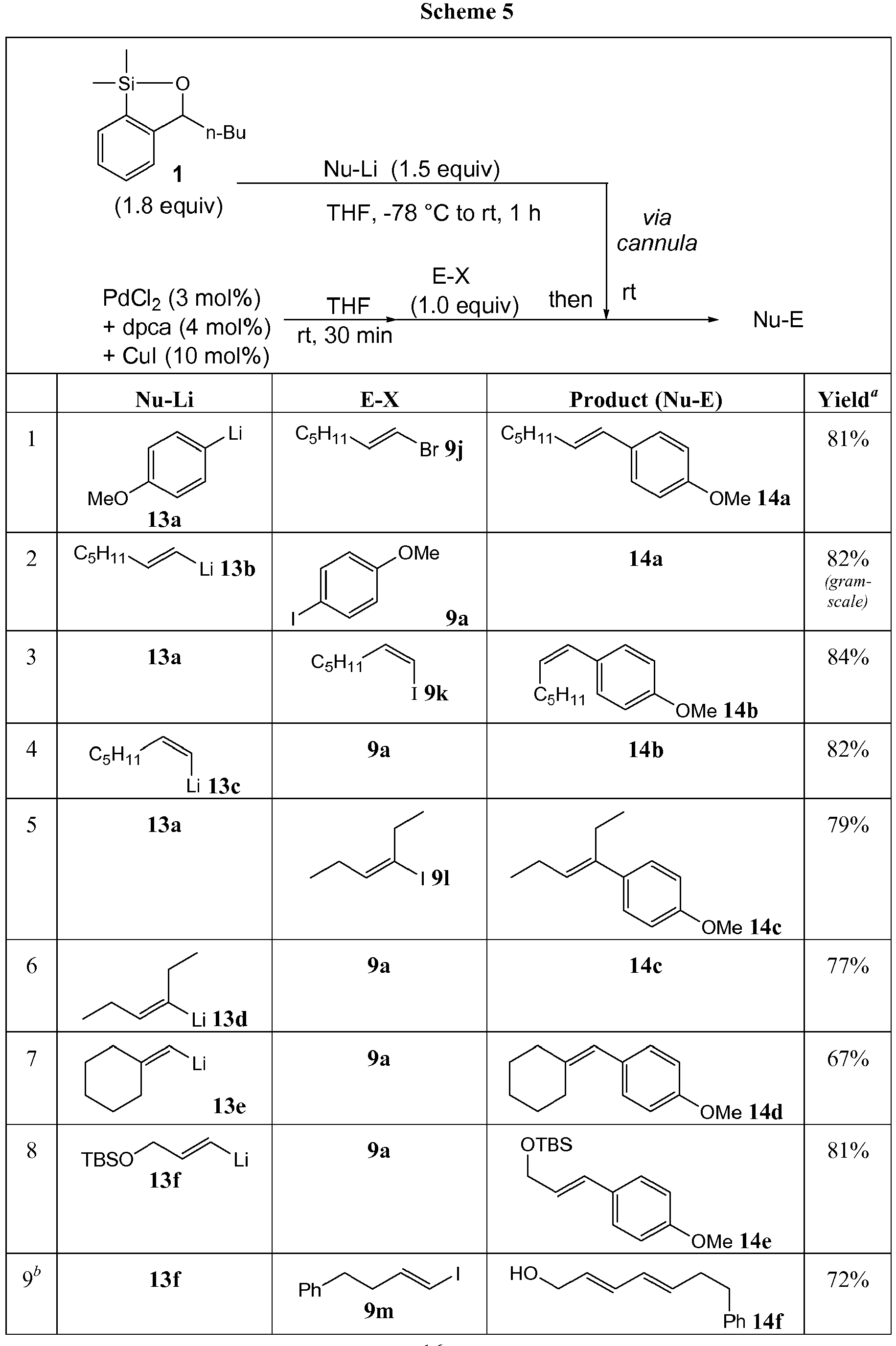

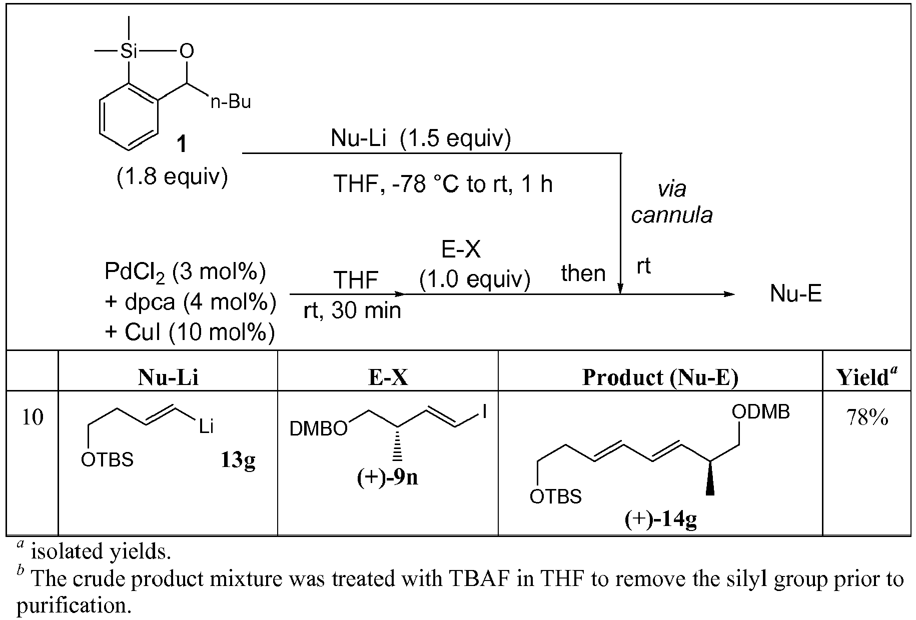

The methods can also be extended to alkenyl substrates. As shown in Scheme 5 above, vinyl halides **9j-l** were good coupling partners with **13a**, forming styrenes **14a-c** with retention of the alkene geometry (see entries 1, 3, and 5). Notably, the roles of the coupling partners could be reversed by using the corresponding vinyllithium and aryl halide to access identical coupling products in comparable yields (see entries 2, 4, and 6), demonstrating the flexibility of the methods of the invention with respect to the choice of nucleophilic and electrophilic components. Geminal and vicinal substitution patterns were tolerated in the cross coupling process from silicon, providing coupled products **14c** and **14d.** Also noteworth was the successful vinyl-vinyl couplings of **13f** and **9m** and between **13g** and (+)**-9n** to provide dienes **14f** and (+)-**14g.**

Scheme 6 details examples demonstrating the scope and utility of the methods using of cyclic silicon compounds as silicon cross coupling agents.

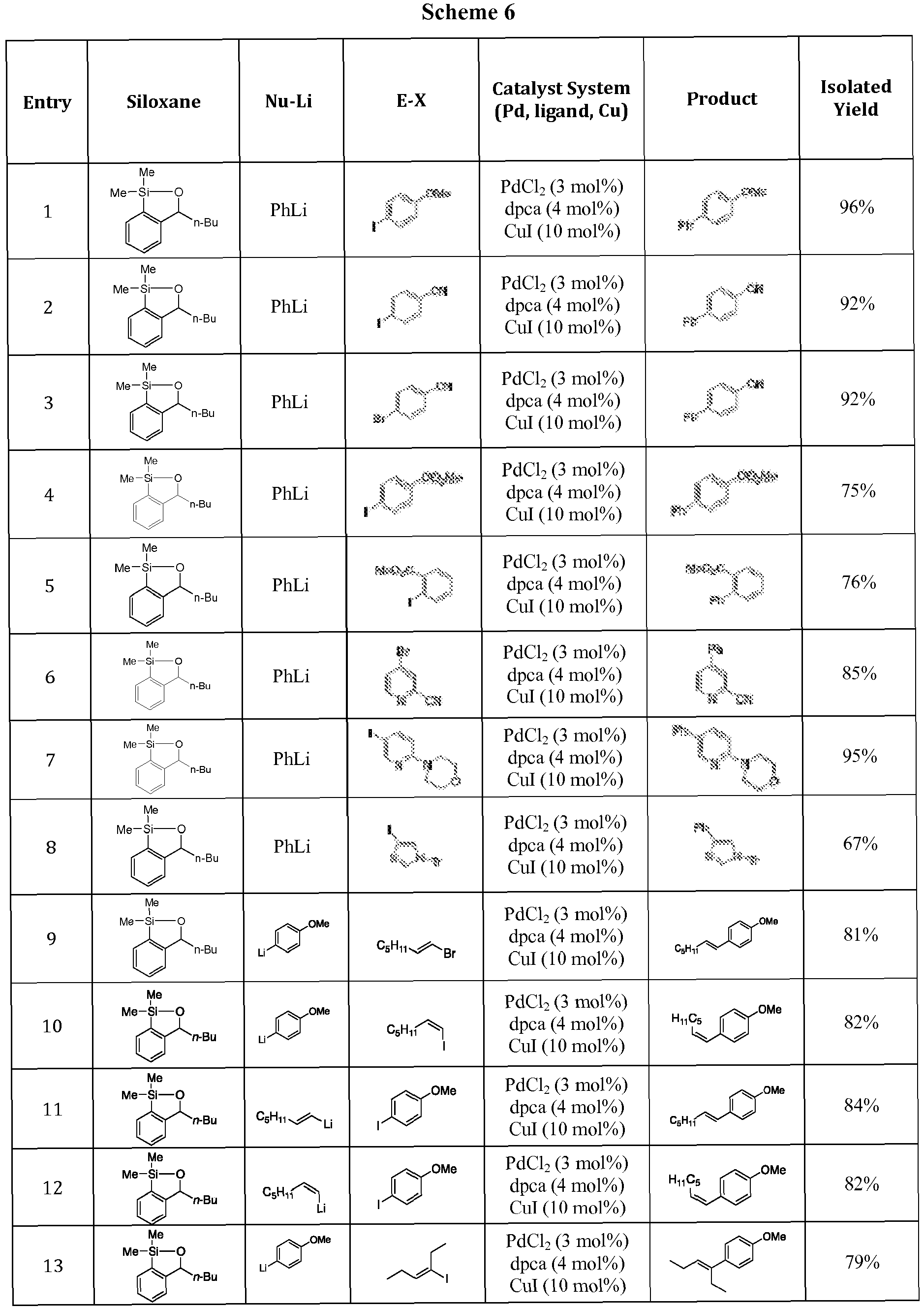

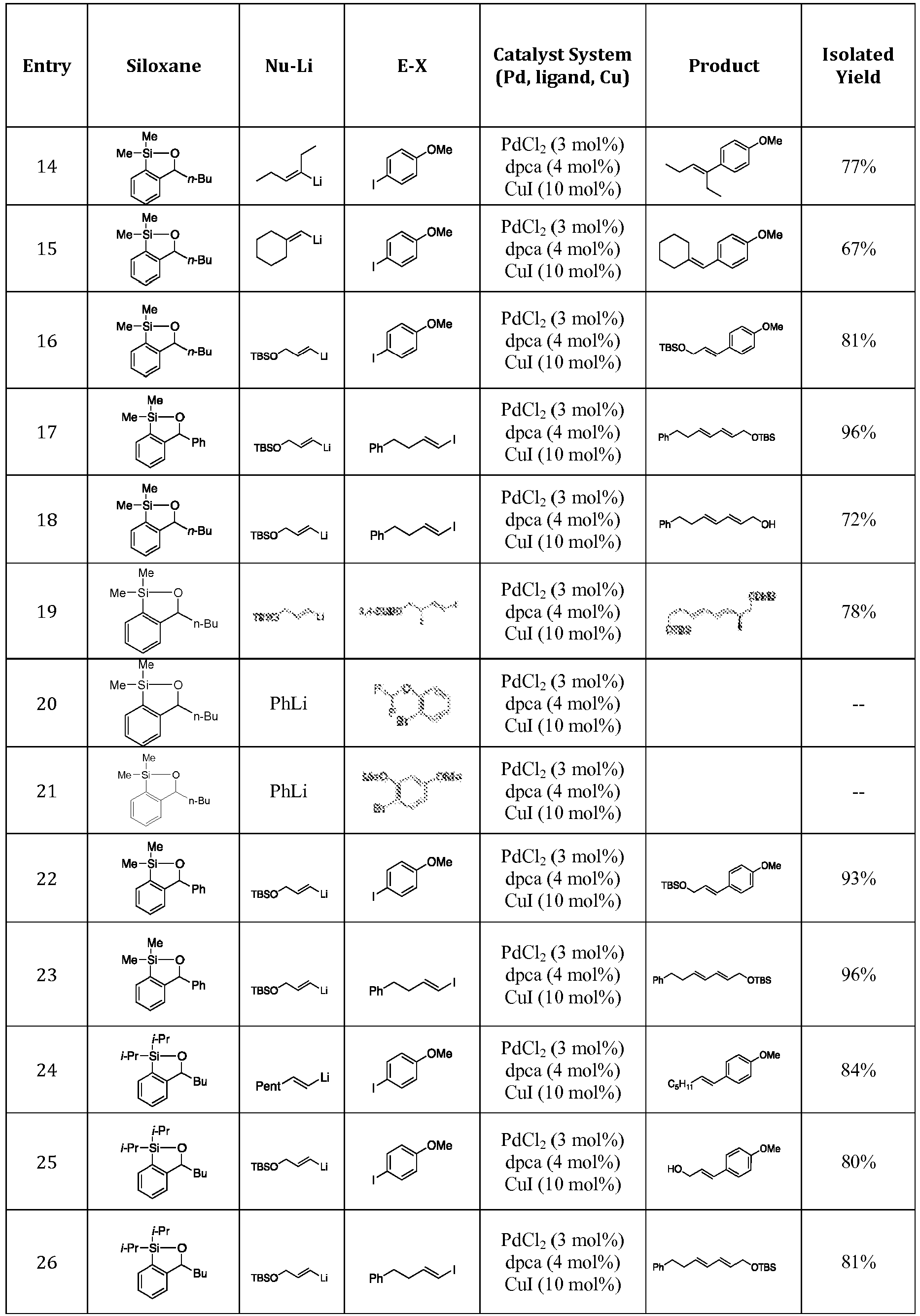

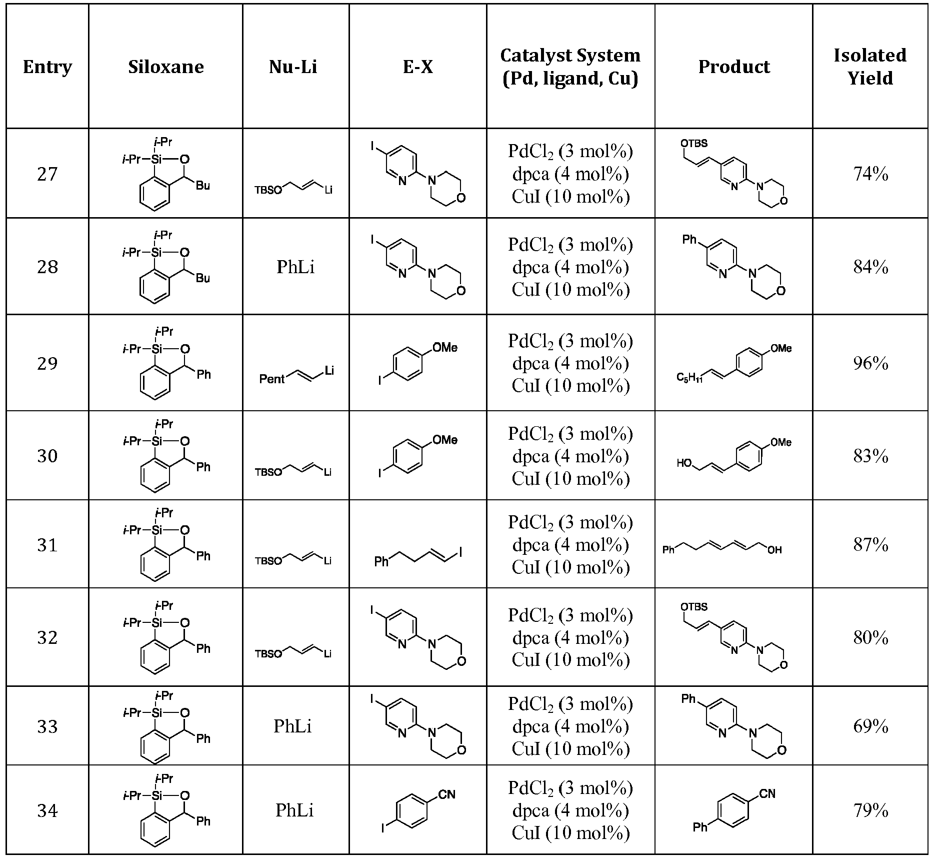

As can be seen in Scheme 7, the synthetic utility of the cross-coupling method may be expanded to include the use of sp3-hybridized organolithium species and alkyl halides. By employing Pd- or Ni-catalysis, it may be possible access sp2-sp3 coupled products. Additionally, sp3-sp3 cross-couplings involving secondary alkyl halides may also be possible. It is preferred, in some examples, that iso-propyl group or groups are present on the silicon in order to avoid competitive transfer of primary alkyl groups from the activated silicon species (e.g., methyl).

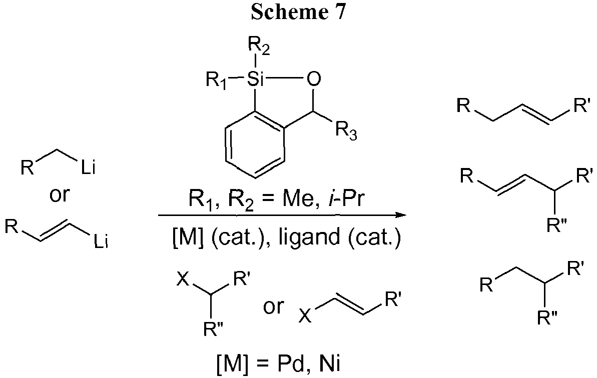

Additional examples of cross-coupling reactions performed using siloxanes as described herein are set forth in Scheme 8.

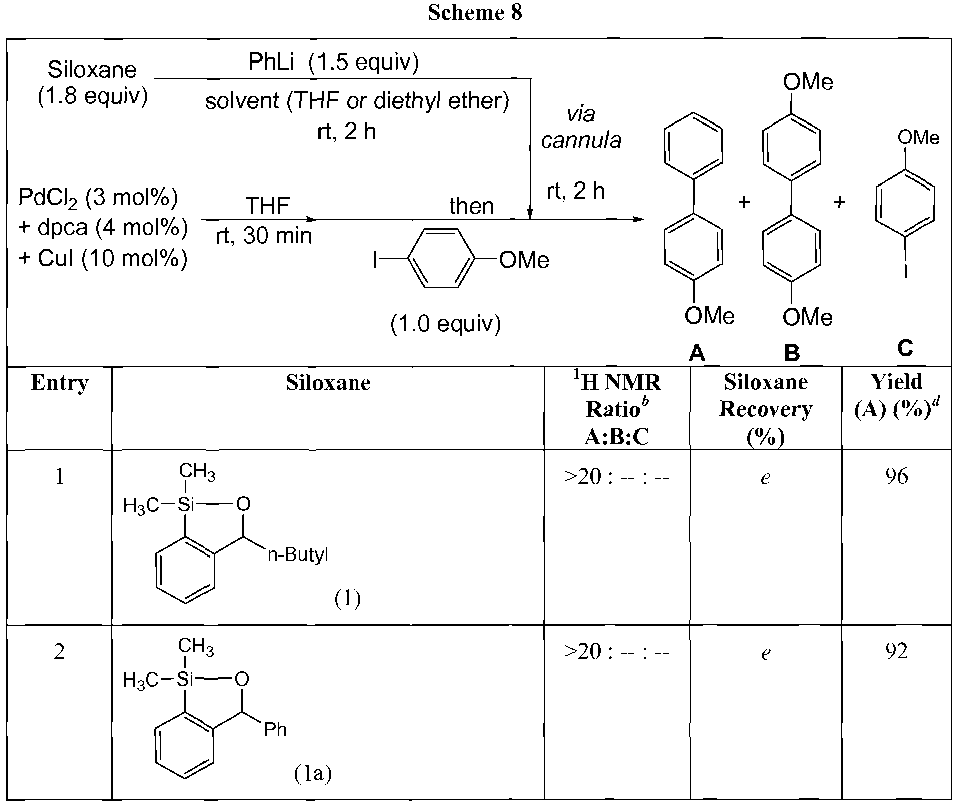

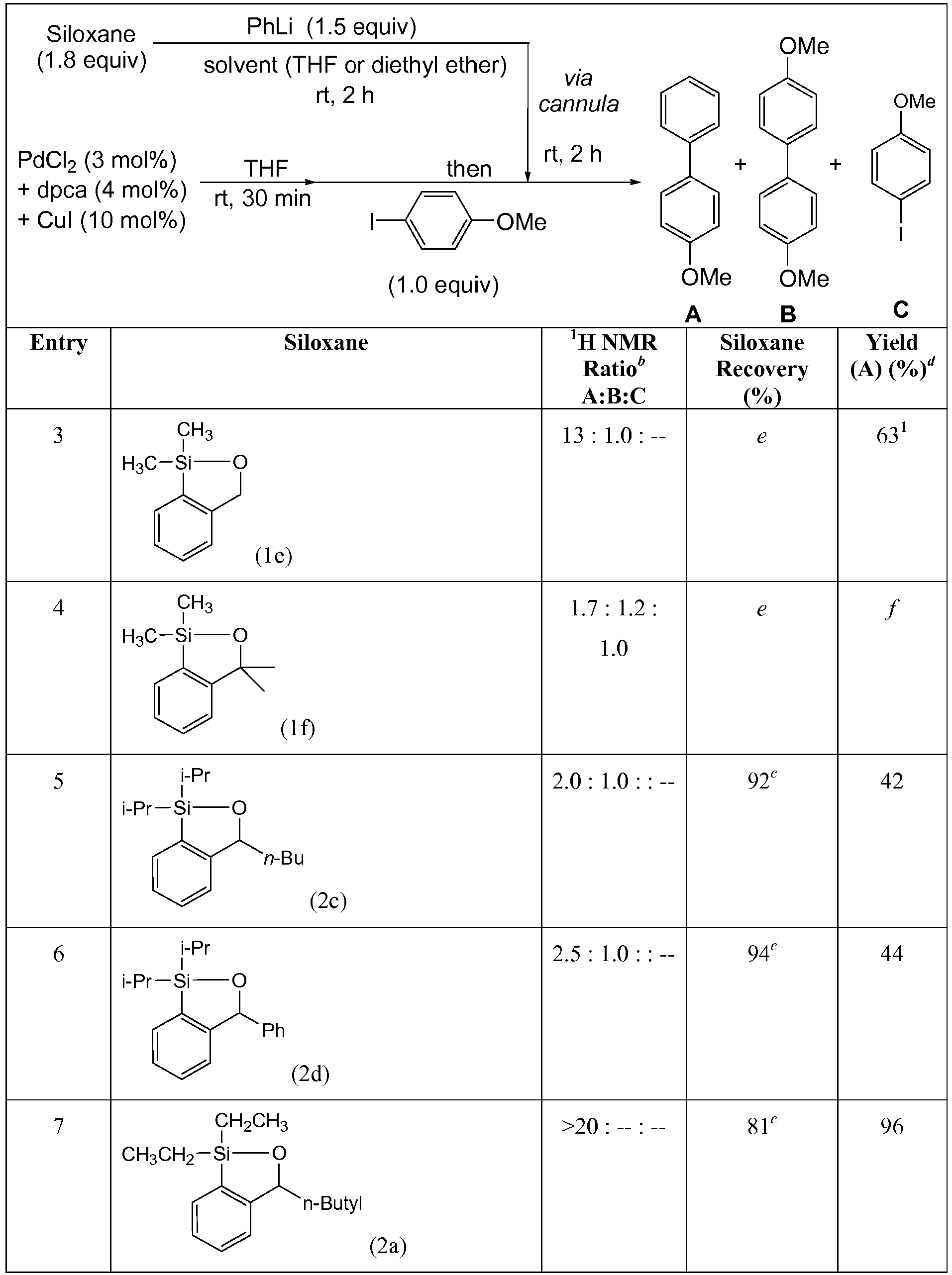

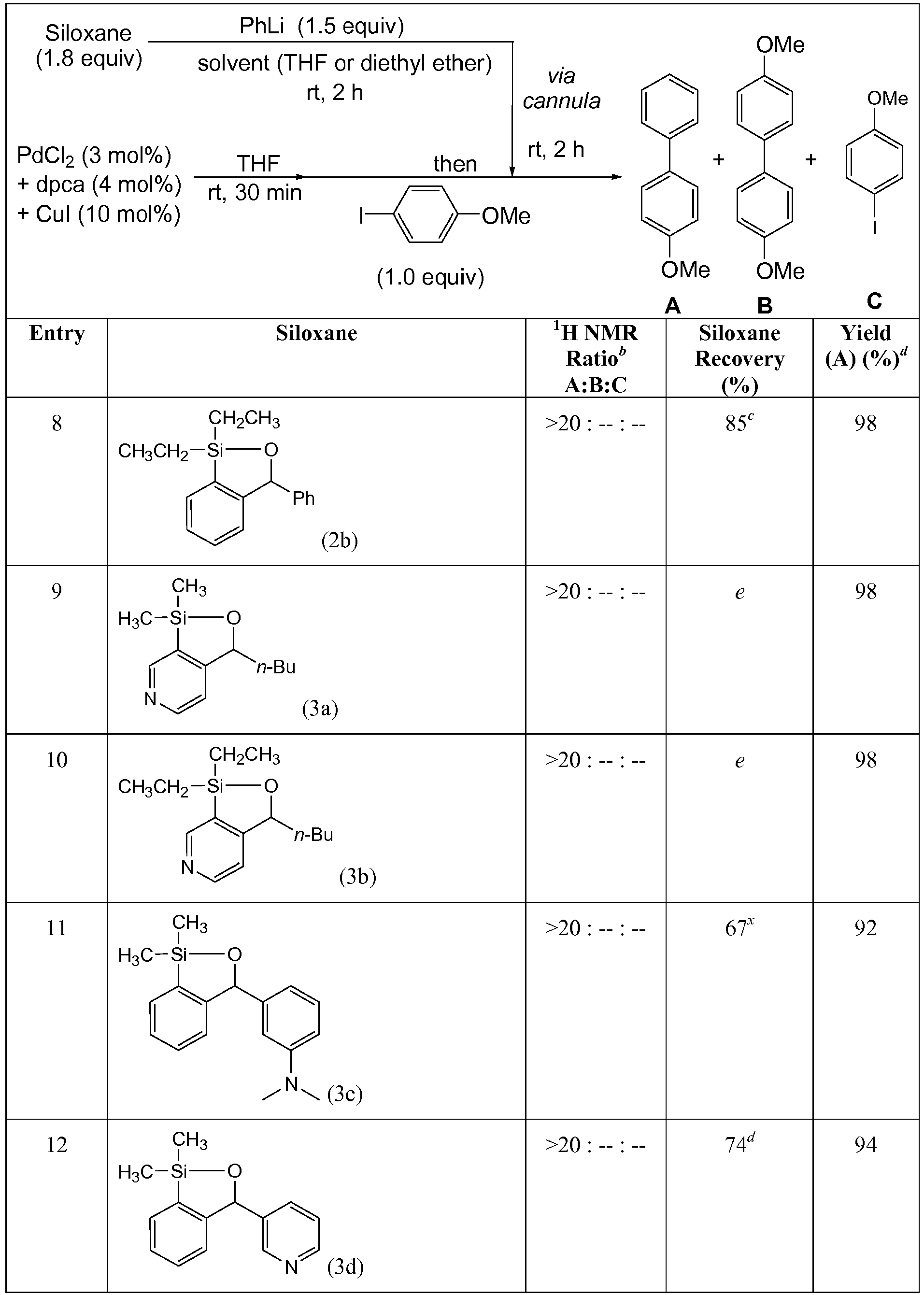

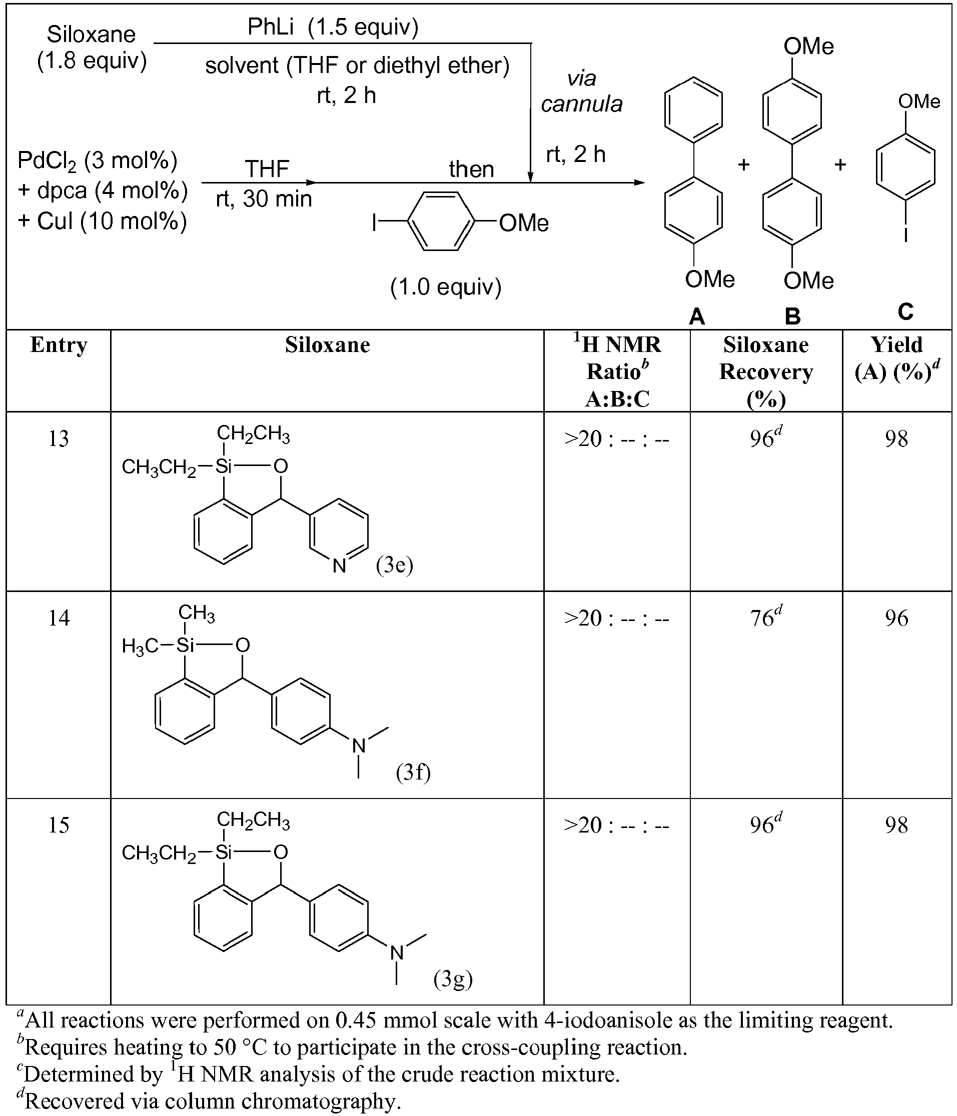

Methods can also be accomplished without copper in the catalyst system. In such embodiments, the catalyst system comprises a palladium-based catalysts, for example, PdCl₂ or Pd(OAc)₂. These embodiments can also employ the use of, for example, XPhos to form a palladium complex catalyst system. An example of such a method is set forth in Scheme 9.

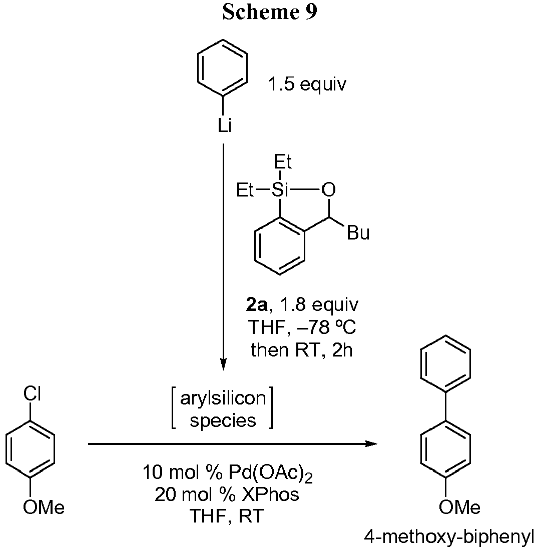

Polymeric siloxane compounds are within the scope of the invention. As can be seen in Scheme 10 (wherein n is about 150 to about 300, preferably about 200 or 250), the siloxane motif may be incorporated into a polymer *via* ring-opening metathesis polymerization (ROMP). For example, according to Scheme 10, treatment of commercially available 5-norbornene-2-carboxaldehyde (a mixture of endo- and exo-isomers) with phenylmagnesium bromide generated the benzylic alcohol. Subsequent ortho-lithiation with n-BuLi, followed by trapping with Me₂SiHCl and treatment with H₂O provided a mixture of benzyl alcohol and siloxane (observed by ¹H-NMR), which was treated with catalytic KOtBu to furnish complete conversion to the desired siloxane monomer. This sequence can be performed on multigram-scale. ROMP (ring opening metathesis polymerization) of this monomer was then achieved smoothly with the first generation Grubbs catalyst.

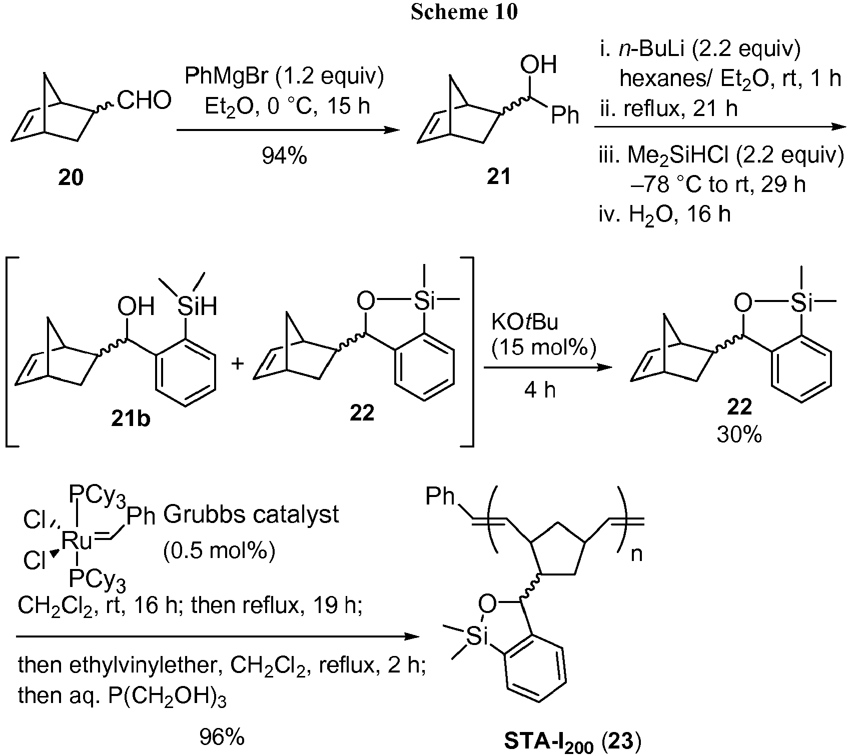

The polymer can be obtained in near quantitative yield without using crosslinking units or co-polymerization agents. As such, the loading of the polymer with siloxane units should be nearly identical to the molarity of the monomer, *e.g*., 3.9 mmol/g for the example set forth in Scheme 9, with each polymer chain having a relative length of 200-mers. The number of repeating siloxane units on each polymer chain can be adjusted by changing the amount of Grubbs catalyst used during the polymerization process.

These polymers of the invention are generally soluble in common organic solvents such as those used in the cross-coupling reaction.

The silicon transfer reagent may also be incorporated into a solid support, for example a resin, using "click chemistry," which is understood by those in the art to include, for example, the reaction of an alkyne and an azide to produce a 1,2,3-triazole. *See* Scheme 11. In preferred embodiments of the invention, the reaction is between an alkyne-capped resin, such as those known in the art, and an azido-siloxane, which can be prepared according to known methods. (Scheme 11). In these embodiments, the compounds of formula I can be tethered to a resin via a triazoyl linker. These embodiments of the inventions may facilitate removed of the transfer reagent from the reaction mixture by mechanical means such as filtration. Additionally, the transfer agent may be regenerated and reused.

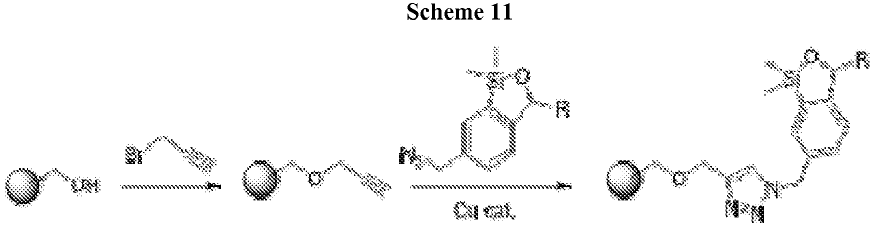

To further exemplify the invention, STA-I₂₀₀, one embodiment of the invention, was used to mediate the cross-coupling of phenyl lithium and 1-iodoanisole. See Scheme 12.

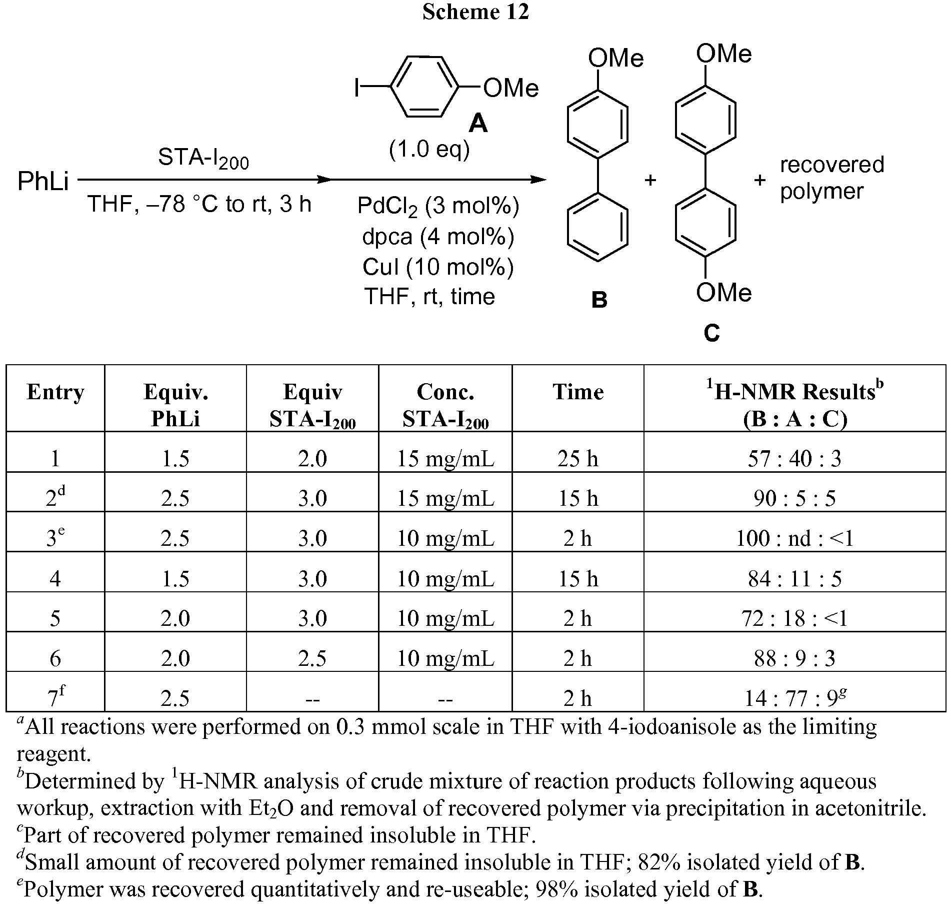

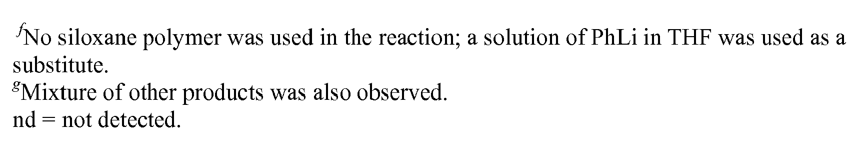

The polymeric siloxanes of the invention have utility in, for example, the cross-coupling between ary organolithiums and aryl or alkenyl iodides. Examples of such cross-coupling reactions are set forth in Scheme 13.

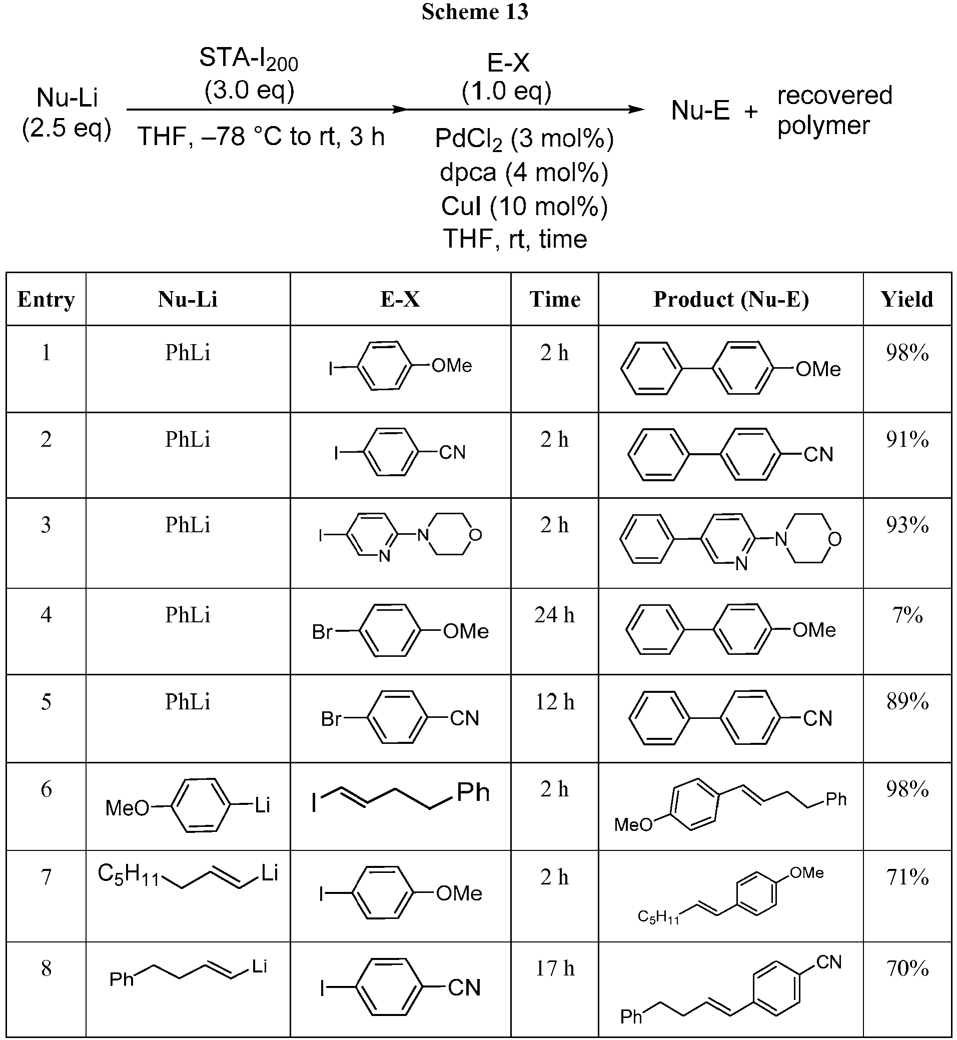

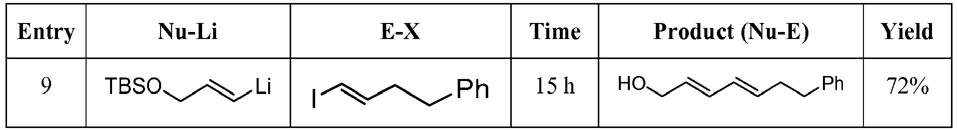

Polymeric siloxanes of the invention are also recyclable and retain activity through multiple cross-coupling reaction cycles, using the same nucleophile for each transformation. *See, e.g.,* Scheme 14.

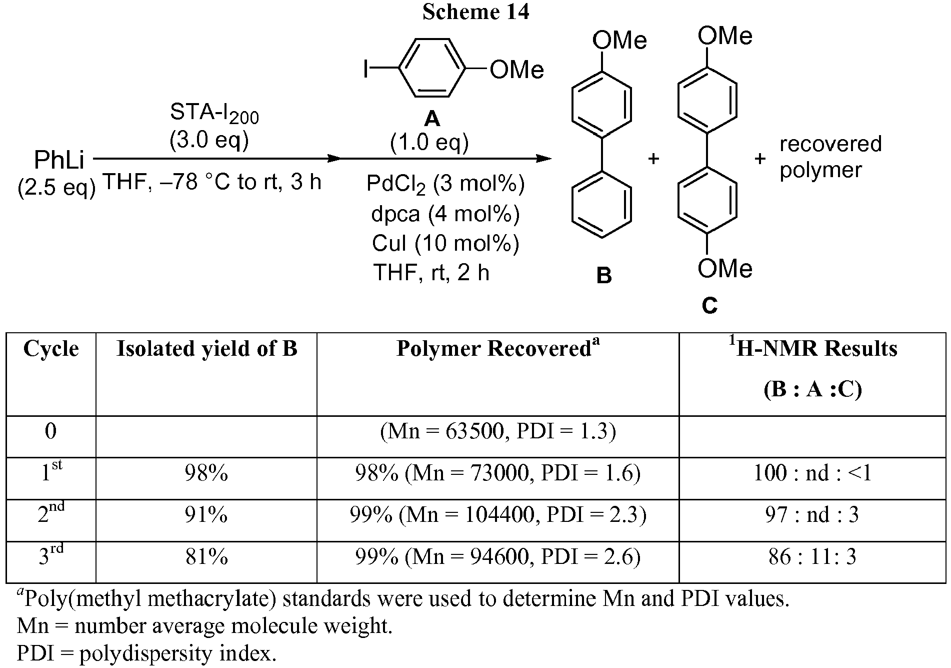

Polymeric siloxanes of the invention can also transfer different nucleophiles in repeated cycles. *See, e.g.,* Scheme 15.

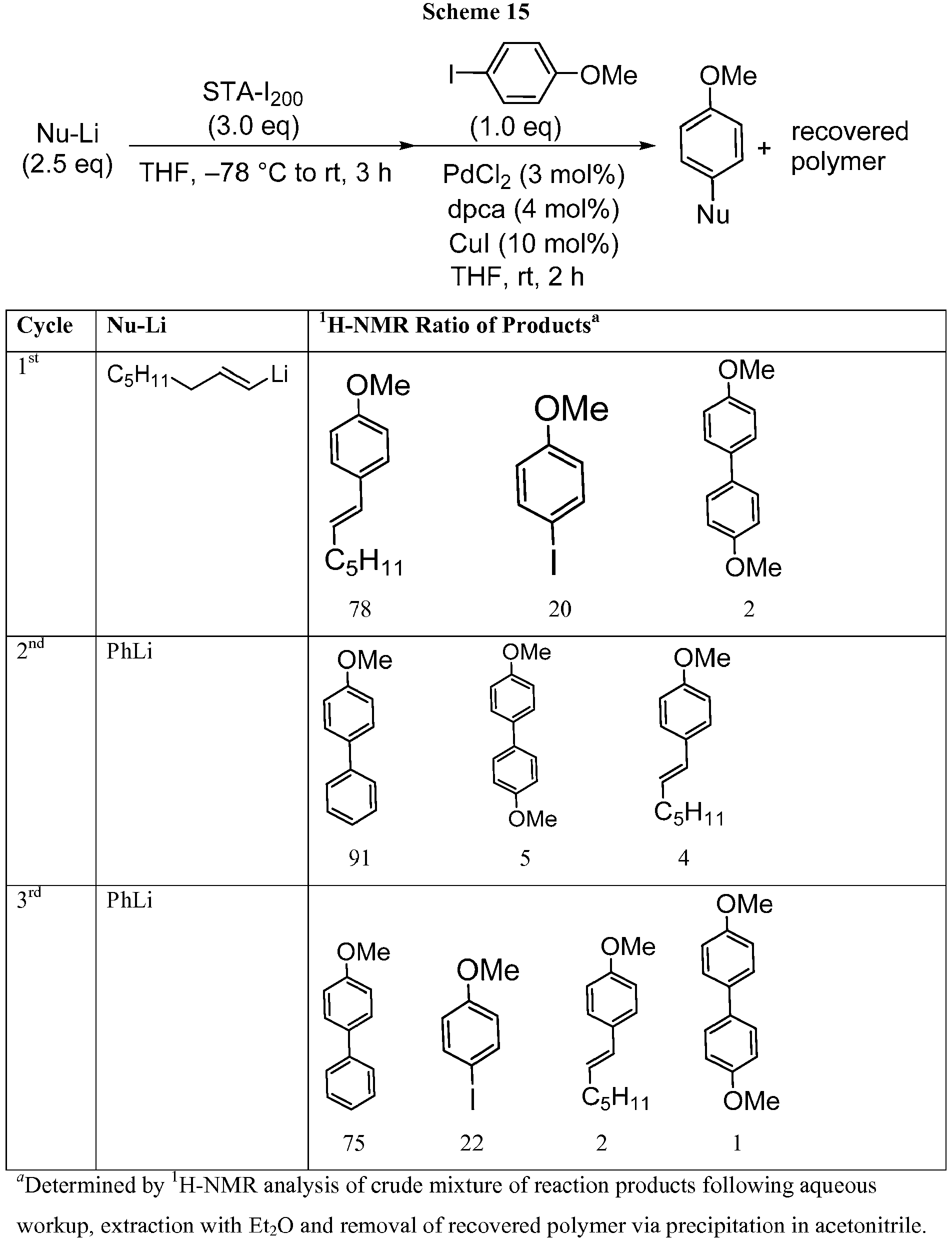

Also described are methods of forming carbon-nitrogen bonds using the siloxanes of the invention. Examples of such methods are set forth in Scheme 16.

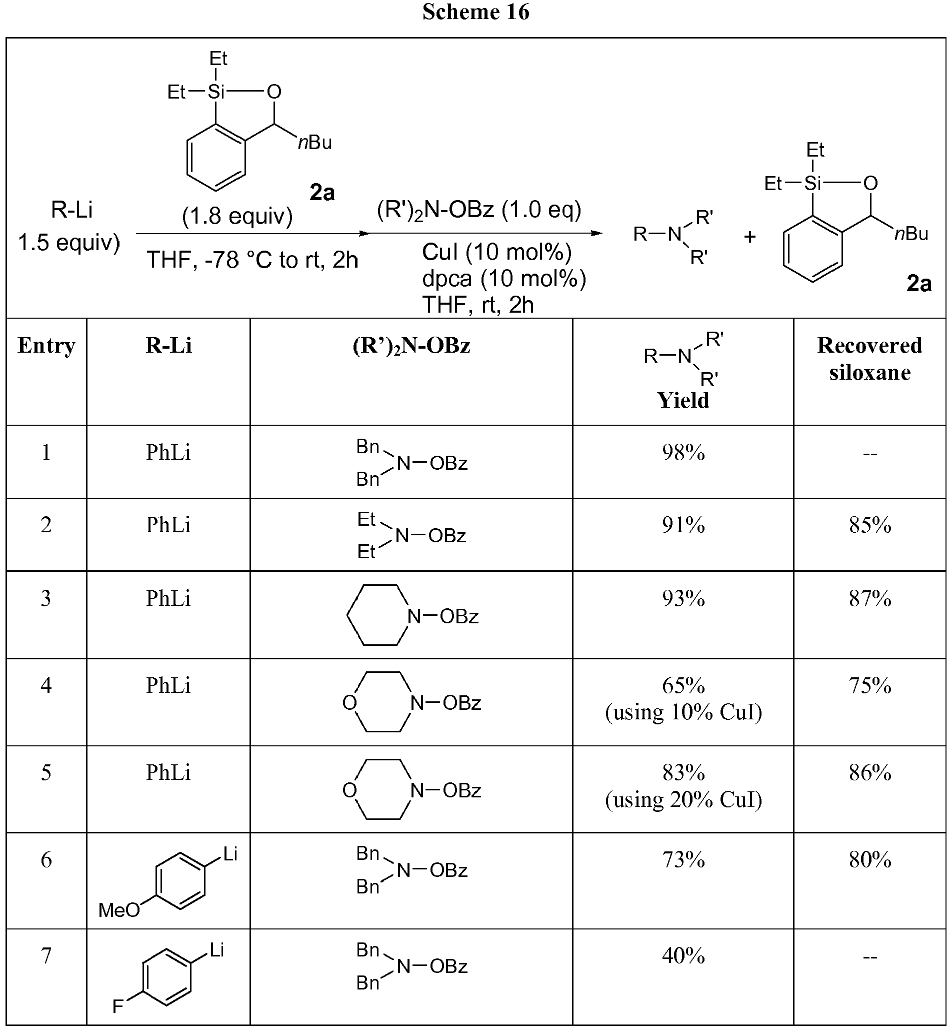

The following examples are only illustrative and are not meant to limit the invention.

### EXAMPLES

**General.** All moisture-sensitive reactions were performed using syringe-septum cap techniques under an inert atmosphere of N₂. All glassware was flame dried or dried in an oven (140 °C) for at least 4 h prior to use. Reactions were magnetically stirred unless otherwise stated. Tetrahydrofuran (THF), dichloromethane (CH₂Cl₂) and diethyl ether (Et₂O) were dried by passage through alumina in a Pure Solve™ PS-400 solvent purification system. Unless otherwise stated, solvents and reagents were used as received. Analytical thin layer chromatography was performed on pre-coated silica gel 60 F-254 plates (particle size 40-55 micron, 230-400 mesh) and visualized by a uv lamp or by staining with PMA (2 g phosphomolybdic acid dissolved in 20 mL absolute ethanol), KMnO₄ (1.5 g of KMnO₄, 10 g of K₂CO₃ and 2.5 mL of 5% aq. NaOH in 150 mL H₂O), or CAM (4.8 g of (NH₄)₆Mo₇O₂₄·4H₂O and 0.2 g of Ce(SO₄)₂ in 100 mL of a 3.5 N H₂SO₄ solution) stain. Column chromatography was performed using silica gel (Silacycle Silaflash® P60, 40-63 micron particle size, 230-300 mesh) and compressed air pressure with commercial grade solvents. Yields refer to chromatographically and spectroscopically pure compounds, unless otherwise stated. NMR spectra were recorded at 500 MHz/125 MHz (¹H NMR/¹³C NMR) on a Bruker Avance III 500 MHz spectrometer at 300 K. Chemical shifts are reported in parts per million with the residual solvent peak as an internal standard. ¹H NMR spectra are tabulated as follows: chemical shift, multiplicity (s=singlet, d=doublet, t=triplet, q=quartet, qn=quintet, dd=doublet of doublets, ddd= doublet of doublet of doublets, dddd= doublet of doublet of doublet of doublets, dt= doublet of triplets, m=multiplet, b=broad), coupling constant and integration. ¹³C NMR spectra are tabulated by observed peak. Melting points were determined using a Thomas-Hoover capillary melting point apparatus and are uncorrected. Infrared spectra were measured on a Jasco FT/IR 480 plus spectrometer. High-resolution mass spectra (HRMS) were obtained at the University of Pennsylvania on a Waters GCT Premier spectrometer. Single crystal X-ray structures were determined at the University of Pennsylvania. X-ray intensity data were collected on a Rigaku Mercury CCD or Bruker APEXII CCD area detector employing graphite-monochromated Mo-Ka radiation (1 = 0.71073 Å) at a temperature of 143(1) K.

### Preparation of water-washed silica gel for column chromatography (where specified):

Silica gel was suspended in H₂O and the slurry mixture was then packed into a prepared column. The obtained H₂O-washed silica gel packed column was then rinsed with 2 column volumes of acetone, 1 column volume of EtOAc and 2 column volumes of hexanes, successively. The obtained column was then ready for use.

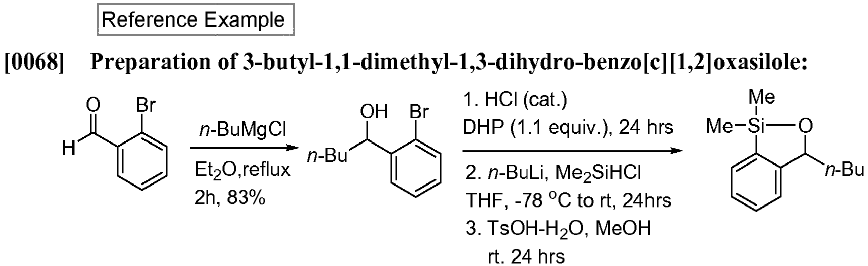

*Synthesis of 1-(2-bromophenyl)pentan-1-ol*: Following the procedure described by Wagner (P. J. Wagner and E. J. Siebert, J. Am. Chem. Soc, 1981, 103, 7329), *n*-BuMgCl (2.0 M in Et₂O, 14.3 mL, 38.1 mmol) was added slowly to a vigorously stirred solution of 2-bromobenzaldehyde (6.4 g, 34.6 mmol) in Et₂O (50 mL) at rt. The resulting suspension was heated to reflux for 2 h and cooled to rt. The reaction mixture was quenched, using extreme caution, by slow addition of aqueous 1 N HCl solution. The organic layer was collected and the aqueous layer was extracted using Et₂O (2 x 60 mL). The combined organic layers were washed with brine, dried over MgSO₄ and concentrated *in vacuo.* The crude product was purified by flash chromatography on silica gel (1:10, EtOAc/Hexane) to afford the desired alcohol (6.9 g, 83%) as light yellow oil: ¹H NMR (500 MHz, CDCl₃) δ 7.55 (dd, *J* = 7.5, 1.0 Hz, 1H), 7.52 (dd, *J* = 8.0, 1.0 Hz, 1H), 7.34 (td, *J* = 7.5, 1.0 Hz, 1H), 7.12 (td, *J* = 8.0, 1.5 Hz, 1H), 5.07 (dd, *J* = 8.0, 4.5 Hz, 1H), 2.08 (OH, 1H), 1.80 (m, 1H), 1.69 (m, 1H), 1.51 (m, 1H), 1.38 (m, 3H), 0.94 (t, *J* = 7.5 Hz, 3H).; ¹³C NMR (125 MHz, CDCl₃) δ 144.1, 132.8, 128.9, 127.5, 122.2, 73.1, 37.6, 28.2, 22.7, 14.2.

Following Hiyama's procedure (Y. Nakao, H. Imanaka, A. K. Sahoo, A. Yada, T. Hiyama, J. Am. Chem. Soc. 2005, 127, 6952), the alcohol (6.9 g, 28.5 mmol) obtained above was dissolved in 3,4-dihydro-2*H*-pyran (3.0 g, 31.4 mmol) at rt. A drop of concentrated HCl (37.5%, about 10 µL) was added and the resulting mixture was stirred for 24 h at rt. The reaction mixture was diluted with diethyl ether (20 mL) and quenched by addition of aqueous NaHCO₃ (10 mL). The organic layer was collected and the aqueous layer was extracted using Et₂O (2 x 20 mL). The combined organic layers were washed with brine, dried over MgSO₄ and concentrated *in vacuo.* The crude product was purified by short flash chromatography on silica gel (1:10, EtOAc/Hexane) to afford a colorless oil (7.9 g, 24.2 mmol, 85%), which was dissolved in dry THF (60 mL) and cooled to -78 °C. *n*-Butyllithium (2.0 M in hexane, 13.3 mL, 26.6 mmol) was added dropwise and the resulting solution was allowed to warm to -30°C over 1 hr and stirred for an additional 30 min at -30 °C. The solution was then cooled to -78 °C and chlorodimethylsilane (Me₂SiHCl, 4.6 g, 48.4 mmol) was added dropwise over 10 minutes. The resulting reaction mixture was allowed to warm to rt and was stirred overnight. The reaction mixture was quenched by addition of saturated aqueous NaHCO₃ (30 mL) and extracted with Et₂O (3 x 50 mL). The combined organic layers were washed with brine, dried with MgSO₄ and concentrated *in vacuo.* The crude product was purified by short flash chromatography on silica gel (1:10, EtOAc/Hexane) to afford the desired silylated product (6.22 g, 20.3 mmol, 84%) as a colorless oil. The silylated product was then dissolved in MeOH (40 mL) and *p*-TsOH•H₂O (0.08 g, 0.4 mmol) was added. The resulting solution was stirred for 24 hrs at rt and concentrated *in vacuo.* Distillation of the crude product under vacuum afforded 1-oxa-2-silacyclopentane (±)-2 (3.89 g, 17.7 mmol, 87%) as a colorless oil: IR (neat, cm⁻¹) 3059, 2958, 2927, 2858, 1594, 1443, 1250, 1080, 919, 865, 830, 789, 744.; ¹H NMR (500 MHz, CDCl₃) δ 7.56 (d, *J* = 7.0 Hz, 1H), 7.41 (td, *J* = 7.5, 1.0 Hz, 1H), 7.29 (t, *J* = 7.0 Hz, 1H), 7.22 (d, *J* = 7.5 Hz, 1H), 5.26 (dd, *J* = 7.5, 3.5 Hz, 1H), 1.93 (m, 1H), 1.62 (m, 1H), 1.50-1.20 (m, 4H), 0.90 (t, *J* = 7.5 Hz, 3H), 0.41 (s, 3H), 0.38 (s, 3H); ¹³C NMR (125 MHz, CDCl₃) δ 153.5, 135.8, 131.0, 129.7, 127.0, 122.4, 81.9, 38.9, 27.5, 23.0. 14.2, 1.5, 0.8; HRMS (ES⁺) *m*/*z* (M+H)⁺: Calcd for C₁₃H₂₁OSi: 221.1362, found: 221.1367.

### Reference Example

### Preparation of Silicon-Based Cross Coupling Agents

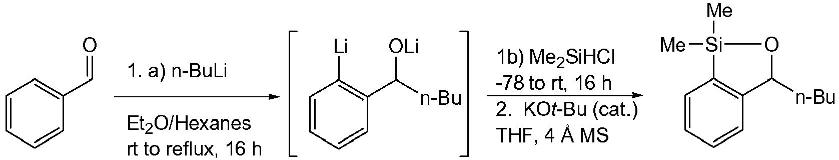

To a solution of benzaldehyde (5.1 mL, 50 mmol) in a mixture of hexanes (250 mL) and Et₂O (200 mL) under an atmosphere of N₂ at room temperature was added a solution of *n*-butyllithium in hexanes (110 mmol) dropwise, maintaining the reaction mixture at room temperature using a water bath. The resulting solution was heated to reflux for 16 h, and the brown mixture was cooled to room temperature, then to -78 °C using an acetone/CO₂(s) bath. To this solution was added R₁(R₂)SiHCl (110 mmol), and the resulting pale yellow slurry was allowed to warm to room temperature and stirred for 16 h. The reaction mixture was diluted with 0.5 M aq. KHCO₃ (100 mL) and extracted with Et₂O (3 x 50 mL). The combined organic layers were washed with brine, dried (MgSO₄), filtered and concentrated under reduced pressure to afford an oil.

The crude oil was taken up in THF (500 mL) in a 2 L flask, and 4 Å molecular sieves were added. To the stirred mixture was added KO*t*-Bu (280 mg, 2.5 mmol), and vigorous gas evolution resulted. Once gas production had ceased, the turbid mixture was diluted with H₂O (100 mL), and the aqueous phase was extracted with Et₂O (3 x 50 mL). The organic extracts were dried (MgSO₄), filtered and concentrated. The resulting yellow oil was purified by chromatography on SiO₂ (100% Hex then 5% Et₂O/Hex). In some cases, the title compound was purified a second time by bulb-to-bulb distillation under vacuum to remove co-eluting impurities.

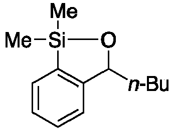

### Reference Example

**3-Butyl-1,1-dimethyl-1,3-dihydrobenzo[*c*][1,2]oxasilole (1).** To a cooled solution of benzaldehyde (21.0 g, 198 mmol, 1.00 equiv) in hexanes (733 mL, pre-dried over MgSO₄) and Et₂O (587 mL) at 0 °C was added *n*-BuLi (224 mL, 1.94 M in hexanes, 435 mmol, 2.20 equiv) dropwise. The reaction mixture was allowed to warm to room temperature and was stirred for 30 minutes. The flask was then fitted with a reflux condenser and the reaction mixture was heated to reflux (75 °C) for 16 h. A dark solution resulted and the reaction mixture was allowed to cool to room temperature. The reflux condenser was removed and the reaction mixture was cooled to -78 °C and Me₂SiHCl (48.4 mL, 435 mmol, 2.20 equiv) was then added dropwise. The reaction mixture was allowed to slowly warm to room temperature and stirred for 8 h. The resulting pale yellow slurry was then quenched with H₂O (300 mL) and the aqueous phase extracted with Et₂O (3 × 100 mL). The combined organic layers were washed with brine, dried (MgSO₄), filtered and concentrated under reduced pressure.

The resulting crude orange oil was taken up in THF (200 mL) at room temperature and 4 Å molecular sieves (3.00 g) and KO*t*-Bu (1.10 g, 9.90 mmol, 0.05 equiv) were added as a single portion and vigorous evolution of H₂ was observed. The reaction mixture was allowed to stir at room temperature for 5 h, quenched with H₂O (50 mL) and the aqueous phase extracted with Et₂O (3 × 100 mL). The combined organic layers were washed with brine, dried (MgSO₄), filtered and concentrated under reduced pressure. Flash chromatography on water-washed silica (100 % hexanes then 1% EtOAc in hexanes) followed by Kugelrohr distillation (85-95 °C, 0.05 mmHg) provided **1** (22.7 g, 103 mmol, 52% yield) as a colorless oil. Analytical data matches that which has been previously reported:⁷ **R***_{f}*0.35 (1% EtOAc in hexanes); **¹H NMR** (500 MHz, CDCl₃) δ 7.56 (d, *J* = 7.0 Hz, 1H), 7.41 (td, *J* = 7.5, 1.0 Hz, 1H), 7.29 (t, *J* = 7.0 Hz, 1H), 7.22 (d, *J* = 7.5 Hz, 1H), 5.26 (dd, *J* = 7.5, 3.5 Hz, 1H), 1.93 (m, 1H), 1.62 (m, 1H), 1.50-1.20 (m, 4H), 0.90 (t, *J* = 7.5 Hz, 3H), 0.41 (s, 3H), 0.38 (s, 3H); **¹³C NMR** (125 MHz, CDCl₃) δ 153.5, 135.8, 131.0, 129.7, 127.0, 122.4, 81.9, 38.9, 27.5, 23.0. 14.2, 1.5, 0.8; **IR** (neat) 3059 (s), 2958 (m), 2927 (m), 2858 (m), 1594 (s), 1443 (s), 1250 (s), 1080 (s), 919 (m), 865 (s), 830 (s), 789 (s), 744 (s) cm⁻¹; **HRMS** (ES+) *m*/*z* calcd for C₁₃H₂₁OSi [M+H]⁺ 221.1362, found 221.1367.

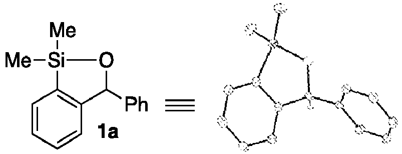

### Reference Example

**1,1-Dimethyl-3-phenyl-1,3-dihydrobenzo[*c*][1,2]oxasilole (1a):** Phenylmagnesium bromide (10.4 mL, 3.00 M in Et₂O, 31.3 mmol, 1.20 equiv) was added dropwise to a vigorously stirred solution of 2-bromobenzaldehyde (4.82 g, 26.1 mmol, 1.00 equiv) in Et₂O (75 mL) at 0 °C. The reaction mixture was stirred at room temperature for 12 h, quenched with sat. aq. NH₄Cl (25 mL) and the aqueous phase extracted with Et₂O (2 x 25 mL). The combined organic layers were washed with brine, dried (MgSO₄), and concentrated under reduced pressure. Flash chromatography on silica (1% Et₂O in hexanes then 10% Et₂O in hexanes) provided the desired (2-bromophenyl)(phenyl)methanol (**S1**) (6.44 g, 24.5 mmol, 94% yield) as a white solid. Analytical data matches that which has been previously reported:⁸ **R*_{f}*** 0.3 (10% EtOAc in hexanes); **¹H NMR** (500 MHz, CDCl₃) δ 7.61-7.52 (m, 2 H), 7.41 (d, *J* = 6.9 Hz, 2 H), 7.37-7.31 (m, 3 H), 7.28 (t, *J* = 7.1 Hz, 1 H), 7.15 (dt, *J* = 1.6, 7.6 Hz, 1 H), 6.21 (d, *J* = 3.8 Hz, 1 H), 2.34 (d, *J* = 4.0 Hz, 1 H); **¹³C NMR** (125 MHz, CDCl₃) δ 142.6, 142.3, 133.0, 129.3, 128.6, 127.9, 127.8, 127.2, 126.7, 122.9, 74.9.

The resulting (2-bromophenyl)(phenyl)methanol (**S1**) (4.29 g, 16.3 mmol, 1.00 equiv) was dissolved in THF (50 mL) and *n*-BuLi (14.7 mL, 2.45 M in hexanes, 35.9 mmol, 2.20 equiv) was added dropwise at -78 °C. The reaction mixture was stirred for 1 h, followed by the addition of Me₂SiHCl (3.90 mL, 35.9 mmol, 2.20 equiv) at -78 °C. The resulting reaction mixture was allowed to warm to room temperature and stirred for 12 h. The reaction mixture was quenched by addition of *t*-BuOH (20 mL) and stirred for 5 h, followed by the addition of H₂O (20 ml) and stirred for another 2 h. The aqueous phase was then extracted with Et₂O (2 x 25 mL) and the combined organic layers were washed with brine, dried (MgSO₄), and concentrated under reduced pressure. Flash chromatography on water-washed silica gel (100% hexanes then 1% Et₂O/Hexanes) provided the desired siloxane **1a** (1.82 g, 26.8 mmol, 46% yield) as a white crystalline solid: **m.p.** 45.5 - 46.5 °C; **R*_{f}***0.3 (5% Et₂O in hexanes); **¹H NMR** (500 MHz, CDCl₃) δ 7.63-7.59 (m, 1 H), 7.36-7.26 (m, 7 H), 7.05-7.01 (m, 1 H), 6.17 (s, 1 H), 0.53 (s, 3 H), 0.45 (s, 3 H); **¹³C NMR** (125 MHz, CDCl₃) δ 152.6, 143.9, 135.3, 130.8, 129.9, 128.7, 127.9, 127.3, 127.3, 123.9, 84.2, 1.4, 0.7; **IR** (CH₂Cl₂) 3057 (m), 2964 (m), 2874 (m), 1447 (m), 1265 (s), 1181 (m), 1136 (m), 1042 (s), 1016 (s), 862 (s), 822 (s), 793 (s), 740 (s), 702 (s), 656 (m) cm⁻¹; **HRMS** (CI⁺) *m*/*z* calculated for C₁₅H₁₇SiO [M+H]⁺ 241.1049, found 241.1034.

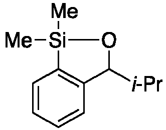

### Reference Example

**3-Isopropyl-1,1-dimethyl-1,3-dihydrobenzo[*c*][1,2]oxasilole (1b):** Phenylmagnesium bromide (11.7 mL, 3.00 M in Et₂O, 35.1 mmol, 1.20 equiv) was added dropwise to a vigorously stirred solution of isobutyraldehyde (2.11 g, 29.3 mmol, 1.00 equiv) in Et₂O (50 mL) at 0 °C. The reaction mixture was warmed to room temperature and stirred for 12 h, then quenched with sat. aq. NH₄Cl (25 mL). The aqueous phase was extracted with Et₂O (2 x 25 mL) and the combined organic layers were washed with brine, dried (MgSO₄) and concentrated under reduced pressure. Flash chromatography on silica, (10% Et₂O in hexanes) provided the desired 2-methyl-1-phenylpropan-1-ol **(S2)** (4.09 g, 27.2 mmol, 93% yield) as a colorless oil. Analytical data matches that which has been previously reported:⁹ **R*_{f}*** 0.15 (10% Et₂O in hexanes); **¹H NMR** (500 MHz, CDCl₃) δ 7.36-7.26 (m, 5 H), 4.37 (dd, *J* = 3.3, 6.8 Hz, 1 H), 2.02-1.91 (m, 1 H), 1.82 (d, *J* = 3.4 Hz, 1 H), 1.01 (d, *J* = 6.7 Hz, 3 H), 0.81 (d, *J* = 6.7 Hz, 3 H); **¹³C NMR** (125 MHz, CDCl₃) δ 143.8, 128.3, 127.6, 126.7, 80.2, 35.4, 19.2, 18.4.

The resulting 2-methyl-1-phenylpropan-1-ol (**S2**) (2.76 g, 18.4 mmol, 1.00 equiv) was dissolved in hexanes (125 ml, pre-dried over MgSO₄) and Et₂O (100 ml) at 0 °C and *n*-BuLi (22.9 ml, 1.77 M in hexanes, 40.5 mmol, 2.20 equiv) was added dropwise. The reaction mixture was allowed to warm to room temperature and was stirred for 30 minutes. The flask was then fitted with a reflux condenser and the system was heated to reflux (75 °C) for 16 h. A dark solution resulted and the reaction mixture was allowed to cool to room temperature. The reflux condenser was removed and the reaction mixture was cooled to -78 °C. Me₂SiHCl (4.40 ml, 40.5 mmol, 2.20 equiv) was added dropwise. The reaction mixture was allowed to slowly warm to room temperature and stirred for 12 h. The slurry was quenched by addition of *t*-BuOH (20 mL) and stirred for 5 h, followed by the addition of H₂O (20 ml) and stirred for another 2 h. The aqueous phase was extracted with Et₂O (2 x 25 mL) and the combined organic layers were washed with brine, dried (MgSO₄), filtered and concentrated under reduced pressure. Flash chromatography on water-washed silica gel (100% hexanes then 1% Et₂O/Hexanes) followed by Kugelrohr distillation (75-80 °C, 0.025 mmHg) provided **1b** (1.48 g, 7.17 mmol, 39% yield) as a colorless oil: **R*_{f}***O.5 (5% Et₂O in hexanes); **¹H NMR** (500 MHz, CDCl₃) δ 7.54 (d, *J* = 7.1 Hz, 1 H), 7.39 (dt, *J* = 1.0, 7.5 Hz, 1 H), 7.29 (t, *J* = 7.2 Hz, 1 H), 7.20 (d, *J* = 7.7 Hz, 1 H), 5.17 (d, *J* = 2.2 Hz, 1 H), 2.12 (dspt, *J* = 2.5, 6.8 Hz, 1 H), 1.17 (d, *J* = 6.9 Hz, 3 H), 0.60 (d, *J* = 6.7 Hz, 3 H), 0.41 (s, 3 H), 0.36 (s, 3 H); **¹³C NMR** (125 MHz, CDCl₃) δ 152.5, 136.4, 130.8, 129.7, 127.0, 122.4, 86.5, 34.6, 20.4, 15.0, 1.0, 0.8; **IR** (neat) 3060 (m), 2963 (s), 2873 (m), 1594 (m), 1469 (m), 1442 (m), 1383 (m), 1365 (m), 1251 (s), 1198 (m), 1137 (m), 1120 (m), 1102 (m), 1067 (s), 1014 (s), 953 (s), 874 (s), 830 (s), 788 (s), 744 (s), 704 (m), 651 (m) cm⁻¹; **HRMS** (CI⁺) *m*/*z* calculated for C₁₁H₁₅SiO [M-Me]⁺ 191.0892, found 191.0893.

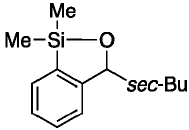

### Reference Example

**3-(*sec*-Butyl)-1,1-dimethyl-1,3-dihydrobenzo[*c*][1,2]oxasilole (1c):** To a cooled solution of benzaldehyde (3.25 g, 30.6 mmol, 1.00 equiv) in hexanes (125 ml, pre-dried over MgSO₄) and Et₂O (100 mL) at 0 °C was added sec-BuLi (26.2 mL, 1.4 M in cyclohexane, 36.7 mmol, 1.20 equiv) dropwise. The reaction mixture was allowed to warm to room temperature and was stirred for 5 h, at which time *n*-BuLi (20.7 mL, 1.77 M in hexanes, 36.7 mmol, 1.20 equiv) was added dropwise. The flask was then fitted with a reflux condenser and the system was heated to reflux (75 °C) for 16 h. A dark solution resulted and the reaction mixture was allowed to cool to room temperature. The reflux condenser was removed and the reaction mixture was cooled to -78 °C and Me₂SiHCl (7.97 ml, 67.3 mmol, 2.20 equiv) was added dropwise. The reaction mixture was allowed to slowly warm to room temperature and stirred for 12 h. The slurry was quenched by addition of *t*-BuOH (20 mL) and stirred for 5 h, followed by the addition of H₂O (20 ml) and stirred for another 2 h. The aqueous phase was extracted with Et₂O (2 x 25 mL) and the combined organic layers were washed with brine, dried (MgSO₄), filtered and concentrated under reduced pressure. Flash chromatography on water-washed silica gel (100% hexanes then 1% Et₂O/Hexanes) followed by Kugelrohr distillation (140-160 °C, 0.01 mmHg) provided **1c,** isolated as a 5:3 ratio of diastereomers by ¹H NMR spectroscopy (2.02 g, 9.17 mmol, 30% yield) as a colorless oil: **R*_{f}*** 0.55 (5% Et₂O in hexanes); *Major diastereoisomer:* **¹H NMR** (500 MHz, CDCl₃) δ 7.54 (d, *J* = 7.1 Hz, 1 H), 7.39 (t, *J* = 7.5 Hz, 1 H), 7.29 (t, *J* = 7.1 Hz, 1 H), 7.21 (d, *J* = 7.7 Hz, 1 H), 5.20 (d, *J=* 2.2 Hz, 1 H), 1.88-1.79 (m, 1 H), 1.13 (d, *J* = 6.9 Hz, 3 H), 1.08-1.01 (m, 2 H), 0.79 (t, *J* = 7.4 Hz, 3 H), 0.40 (s, 3 H), 0.36 (s, 3 H); **¹³C NMR** (125 MHz, CDCl₃) δ 152.3, 136.6, 130.9, 129.6, 126.9, 122.4, 86.7, 41.8, 22.5, 16.9, 12.5, 1.0, 0.8. *Minor diastereoisomer*: **¹H NMR** (500 MHz, CDCl₃) δ 7.54 (d, *J* = 7.1 Hz, 1 H), 7.39 (t, *J* = 7.5 Hz, 1 H), 7.29 (t, *J* = 7.1 Hz, 1 H), 7.19 (d, *J* = 7.7 Hz, 1 H), 5.20 (d, *J* = 1.2 Hz, 1 H), 1.88-1.79 (m, 1 H), 1.74-1.65 (m, 1 H), 1.53-1.43 (m, 1 H), 1.04 (t, *J* = 7.7 Hz, 3 H), 0.55 (d, *J* = 6.7 Hz, 3 H), 0.40 (s, 3 H), 0.36 (s, 3 H); **¹³C NMR** (125 MHz, CDCl₃) δ 152.7, 136.4, 130.8, 129.7, 126.9, 122.2, 84.62, 41.5, 31.9, 27.5, 12.5, 1.0, 0.7; **IR** (neat) 3060 (m), 2962 (s), 2875 (s), 1594 (m), 1443 (s), 1375 (m), 1323 (m), 1250 (s), 1197 (m), 1137 (m), 1107 (s), 1068 (s), 1040 (s), 1014 (s), 960 (s), 875 (s), 826 (s), 789 (s), 746 (s), 705 (m), 653 (m) cm⁻¹; **HRMS** (CI⁺) *m*/*z* calculated for C₁₃H₁₉SiO [M-H]⁺ 221.1362, found 221.1368.

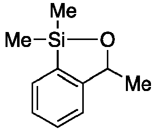

### Reference Example

**1,1,3-Trimethyl-1,3-dihydrobenzo[*c*][1,2]oxasilole (1d):** To a cooled solution of benzaldehyde (2.63 g, 24.8 mmol, 1.00 equiv) in hexanes (125 ml, pre-dried over MgSO₄) and Et₂O (100 ml) at 0 °C was added MeLi (1.36 M in Et₂O, 29.7 mmol, 1.20 equiv) dropwise. The resulting solution was allowed to warm to room temperature and stirred for 5 h, at which time *n*-BuLi (12.1 mL, 2.45 M in hexanes, 29.7 mmol, 1.20 equiv) was added dropwise. The flask was then fitted with a reflux condenser and the system was heated to reflux (75 °C) for 16 h. A dark solution resulted and the reaction mixture was allowed to cool to room temperature. The reflux condenser was removed and the reaction mixture was cooled to -78 °C and Me₂SiHCl (5.20 ml, 54.6 mmol, 2.20 equiv) was added dropwise. The reaction mixture was allowed to slowly warm to room temperature and stirred for 12 h. The reaction mixture was quenched by addition of *t*-BuOH (20 mL) and stirred for 5 h, followed by the addition of H₂O (20 ml) and stirred for another 2 h. The aqueous phase was extracted with Et₂O (2 x 25 mL) and the combined organic layers were washed with brine, dried (MgSO₄), filtered and concentrated under reduced pressure. Flash chromatography on water-washed silica gel (100% hexanes then 1% Et₂O/Hexanes) followed by Kugelrohr distillation (55 °C, 0.01 mmHg) provided **1d** (1.55 g, 8.69 mmol, 35 % yield) as a colorless oil: **R*_{f}***0.45 (5% Et₂O in hexanes); **¹H NMR** (500 MHz, CDCl₃) δ 7.56 (d, *J* = 7.1 Hz, 1 H), 7.40 (dt, *J* = 0.8, 7.5 Hz, 1 H), 7.30 (t, *J* = 7.3 Hz, 1 H), 7.22 (d, *J* = 7.7 Hz, 1 H), 5.34 (q, *J* = 6.5 Hz, 1 H), 1.51 (d, *J* = 6.5 Hz, 3 H), 0.41 (s, 3 H), 0.37 (s, 3 H); **¹³C NMR** (125 MHz, CDCl₃) δ 154.5, 135.2, 131.0, 129.8, 127.0, 122.3, 78.0, 25.4, 1.7, 0.6; **IR** (neat) 3060 (m), 2968 (s), 2924 (m), 2867 (m), 1595 (m), 1444 (s), 1368 (m), 1318 (s), 1251 (s), 1199 (m), 1137 (m), 1086 (s), 1028 (s), 929 (s), 855 (s), 828 (s), 793 (s), 759 (s), 742 (s), 696 (m), 653 (m) cm⁻¹; **HRMS** (CI⁺) *m*/*z* calculated for C₉H₁₁SiO [M-Me]⁺ 163.0579, found 163.0578.

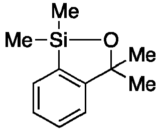

### Reference Example

**1,1,3,3-Tetramethyl-1,3-dihydrobenzo[*c*][1,2]oxasilole (1f):¹** Following a previously reported procedure,¹ methyl 2-bromobenzoate (9.70 g, 45.0 mmol, 1.00 equiv) was dissolved in Et₂O (100 ml) and cooled to 0 °C. Methylmagnesium bromide (99 mL, 1.0 M in Bu₂O, 99.0 mmol, 2.20 equiv) was then added via cannula into the reaction mixture. The resulting solution was heated to reflux (40 °C) for 2 h then cooled to room temperature before being quenched with sat. aq. NH₄Cl (75 mL). The aqueous phase extracted with Et₂O (2 x 50 mL). The combined organic layers were washed with brine, dried (MgSO₄), filtered and concentrated under reduced pressure to afford 2-(2-bromophenyl)propan-2-ol (**S3**) (8.30 g, ca. 38.8 mmol, 1.00 equiv), which was used without further purification.

To the crude alcohol was added conc. HCl (3 drops) and 3,4-dihydro-2H-pyran (**S3**) (4.07 g, 48.5 mmol, 1.20 equiv) and stirred neat at room temperature for 24 h. The mixture was diluted with Et₂O (50 mL), and washed with a sat. aq. NaHCO₃ (25 mL). The aqueous layer was extracted with Et₂O (2 x 50 mL) and the combined organic layers were washed with brine, dried (MgSO₄), filtered and concentrated under reduced pressure to afford 2-((2-(2-bromophenyl)propan-2-yl)oxy)tetrahydro-2*H*-pyran (**S4**) (8.30 g, ca. 38.8 mmol, 1.00 equiv) which was used without further purification.

The crude THP-protected product (**S4**) was dissolved in THF (50 mL) and *n-*BuLi (23.3 mL, 2.00 M in hexanes, 46.6 mmol, 1.20 equiv) was added dropwise at -78 °C. The reaction mixture was stirred for 1 h, followed by the addition of Me₂SiHCl (6.30 mL, 58.2 mmol, 1.50 equiv) at -78 °C. The resulting reaction mixture was allowed to warm to room temperature and stirred for 12 h. The reaction mixture was quenched by addition of H₂O (50 ml). The aqueous phase was then extracted with Et₂O (2 x 30 mL) and the combined organic layers were washed with brine, dried (MgSO₄), filtered and concentrated under reduced pressure to afford dimethyl(2-(2-((tetrahydro-2*H*-pyran-2-yl)oxy)propan-2-yl)phenyl)silane (**S5**) (8.30 g, ca. 38.8 mmol, 1.00 equiv) which was used without further purification.

To this crude material (**S5**) was added MeOH (50 mL) and *p*-toluenesulfonic acid monohydrate (370 mg, 1.94 mmol, 0.05 equiv), and the mixture was stirred at room temperature for 12 h before concentration under reduced pressure. The residue was diluted with Et₂O (25 mL), and washed with a sat. aq. NaHCO₃ (25 mL). The aqueous layer was extracted with Et₂O (2 x 25 mL), and the combined organic layers were washed with brine, dried (MgSO₄), filtered and concentrated under reduced pressure. Kugelrohr distillation (60 °C, 0.01 mmHg) provided **If** (3.27 g, 17.0 mmol, 38% yield from methyl 2-bromobenzoate) as a colorless oil. Analytical data matches that which has been previously reported:² **R*_{f}***0.3 (1% Et₂O in hexanes); **¹H NMR** (500 MHz, CDCl₃) δ 7.52 (dt, *J* = 7.1, 0.9 Hz, 1 H), 7.41 (td, *J* = 7.5, 1.3 Hz, 1 H), 7.30 (dt, *J* = 7.2, 0.9 Hz, 1 H), 7.22 (d, *J* = 7.7 Hz, 1 H), 1.55 (s, 6 H), 0.39 (s, 6 H); **¹³C NMR** (125 MHz, CDCl₃) δ 157.6, 134.4, 130.6, 129.7, 126.6, 122.1, 83.5, 32.1, 1.37.

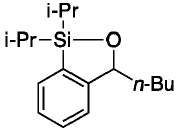

### Reference Example

**3-Butyl-1,1-diisopropyl-1,3-dihydrobenzo[*c*][1,2]oxasilole (2c):** To a cooled solution of benzaldehyde (3.08 g, 29.0 mmol, 1.00 equiv) in hexanes (125 ml, pre-dried over MgSO₄) and Et₂O (100 ml) at 0 °C was added *n*-BuLi (27.8 mL, 2.3 M in hexanes, 63.9 mmol, 2.20 equiv) dropwise. The reaction mixture was allowed to warm to room temperature and was stirred for 30 minutes. The flask was then fitted with a reflux condenser and the system was heated to reflux (75 °C) for 16 h. A dark solution resulted and was allowed to cool to room temperature, the reflux condenser was removed and the reaction mixture was cooled to -78 °C and *i*-Pr₂SiHCl (10.9 ml, 63.8 mmol, 2.20 equiv) was added dropwise. The reaction mixture was allowed to slowly warm to room temperature and stirred for 12 h. The reaction mixture was quenched by addition of 3M HCl in MeOH (40 mL) at 0 °C and stirred for 12 h at room temperature. Water (100 mL) was added and stirred for another 2 h and the aqueous phase extracted with Et₂O (2 x 50 mL). The combined organic layers were washed with brine, dried (MgSO₄), filtered and concentrated under reduced pressure. Flash chromatography on silica (1% Et₂O/Hexanes) provided **2c** (5.94 g, 21.5 mmol, 74% yield) as a colorless oil: **R*_{f}***0.70 (5% Et₂O in hexanes); **¹H NMR** (500 MHz, CDCl₃) δ 7.53 (d, *J* = 7.1 Hz, 1 H), 7.38 (t, *J* = 7.5 Hz, 1 H), 7.27 (t, *J* = 7.1 Hz, 1 H), 7.20 (d, *J* = 7.7 Hz, 1 H), 5.18-5.14 (m, 1 H), 1.94-1.86 (m, 1 H), 1.60-1.48 (m, 3 H), 1.45-1.30 (m, 2 H), 1.25-1.16 (m, 2 H), 1.06 (d, *J* = 6.7 Hz, 3 H), 1.05 (d, *J* = 7.1 Hz, 3 H), 1.00 (d, *J* = 7.5 Hz, 3 H), 0.93 (d, *J* = 7.3 Hz, 3 H), 0.92 (t, *J* = 7.3 Hz, 3 H); **¹³C NMR** (125 MHz, CDCl₃) δ 154.7, 132.4, 132.2, 129.5, 126.7, 122.2, 82.3, 39.1, 28.1, 23.0, 17.6, 17.5, 17.2, 17.1, 14.2, 13.4, 12.9; **IR** (neat) 3059 (m), 3000 (m), 2941 (s), 2864 (s), 1595 (m), 1464 (s), 1443 (m), 1381 (m), 1261 (m), 1111 (m), 1080 (s), 1054 (m), 1012 (m), 988 (m), 973 (m), 917 (s), 880 (s), 846 (m), 831 (m), 816 (m), 749 (s), 716 (s), 668 (s), 648 (m), 612 (m) cm⁻¹; **HRMS** (CI⁺) *m*/*z* calculated for C₁₄H₂₁SiO [M-C₃H₇]⁺ 233.1726, found 233.1726.

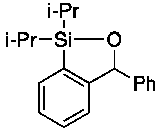

### Reference Example

**1,1-Diisopropyl-3-phenyl-1,3-dihydrobenzo[*c*][1,2]oxasilole (2d):** To the previously prepared (2-bromophenyl)(phenyl)methanol (**S1**) (5.18 g, 19.7 mmol, 1.00 equiv) dissolved in THF (50 mL) was added *n*-BuLi (19.0 mL, 2.28 M in hexanes, 43.3 mmol 2.20 equiv) dropwise at -78 °C. The reaction mixture was stirred for 1 h, followed by the addition of *i*-Pr₂SiHCl (7.39 mL, 43.3 mmol, 2.20 equiv) at -78 °C. The resulting reaction mixture was allowed to warm to room temperature and stirred for 12 h. The slurry was quenched by addition of 3M HCl in MeOH (40 mL) at 0 °C and stirred for 4 h at room temperature. Water (100 mL) was added and stirred for another 2 h and the aqueous phase extracted with Et₂O (2 x 50 mL). The combined organic layers were washed with brine, dried (MgSO₄), filtered and concentrated under reduced pressure. Flash chromatography on silica (1% Et₂O/Hexanes) provided **2d** (4.12 g, 13.9 mmol, 71% yield) as a colorless oil: **R*_{f}***0.45 (5% Et₂O in hexanes); **¹H NMR** (500 MHz, CDCl₃) δ 7.63-7.59 (m, 1 H), 7.37-7.27 (m, 7 H), 6.99 (d, *J* = 6.9 Hz, 1 H), 6.12 (s, 1 H), 1.39-1.24 (m, 2 H), 1.16 (d, *J* = 7.5 Hz, 3 H), 1.13 (d, *J* = 7.5 Hz, 3 H), 1.06 (d, *J* = 5.7 Hz, 3 H), 1.04 (d, *J* = 5.7 Hz, 3 H); **¹³C NMR** (125 MHz, CDCl₃) δ 153.4, 143.8, 132.7, 131.9, 129.8, 128.6, 128.0, 127.8, 127.0, 123.9, 84.6, 17.7, 17.2, 13.6, 13.3; **IR** (neat) 3061 (m), 3031 (m), 3000 (m), 2942 (s), 2891 (m), 2864 (s), 1592 (m), 1494 (m), 1463 (s), 1442 (s), 1383 (m), 1263 (m), 1182 (m), 1133 (m), 1077 (m), 1065 (s), 1038 (s), 1014 (s), 988 (s), 919 (m), 881 (m), 815 (s), 747 (s), 732 (s), 713 (s), 698 (s), 669 (s), 633 (m) cm⁻¹; **HRMS** (CI⁺) *m*/*z* calculated for C₁₆H₁₇SiO [M-C₃H₇]⁺ 253.1049, found 253.1056.

**Preparation of Et₂SiHCl from commercially available Et₂SiH₂:⁴** Anhydrous CuCl₂ (96.5 g, 714 mmol, 2.10 equiv) was dried under vacuum at 200 °C (utilizing a sand bath) in a 2000 mL two-necked round-bottomed flask (RBF) for 12 hours. Upon cooling to room temperature, CuI (3.24 g, 17.0 mmol, 0.05 equiv), Et₂O (680 mL ∼ 0.5 M) and Et₂SiH₂ (30.0 g, 340 mmol, 1.00 equiv) were successively added and the resulting slurry stirred at room temperature for 43 hours. After 8 hours, it was noted that the reaction went from brown to light grey in color with black precipitate forming. After 43 hours, the reaction mixture was filtered under N₂ atmosphere and the flask rinsed with Et₂O (2 x 50 mL). The Et₂O was distilled (35 °C) and the reaction mixture transferred to a 100 mL flame-dried RBF via syringe, carefully leaving behind any remaining Cu salts. Kugelrohr distillation (95-100 °C) of this crude reaction mixture under N₂ provided clean Et₂SiHCl (32.2 g, 263 mmol, 77% yield) whose analytical data matches that which has been previously reported.⁴

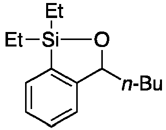

### Reference Example

**3-Butyl-1,1-diethyl-1,3-dihydrobenzo[*c*][1,2]oxasilole (2a):** To a cooled solution of benzaldehyde (10.0 g, 94.2 mmol, 1.00 equiv) in hexanes (500 mL, pre-dried over MgSO₄) and Et₂O (400 mL) at 0 °C was added *n*-BuLi (86.4 mL, 2.40 M in hexanes, 2.20 equiv) dropwise. The reaction mixture was allowed to warm to room temperature and was stirred for 30 minutes. The flask was then fitted with a reflux condenser and the system was heated to reflux (75 °C) for 16 h. A dark solution resulted and the reaction mixture was allowed to cool to room temperature. The reflux condenser was removed and the reaction mixture was cooled to -78 °C and Et₂SiHCl (28.9 mL, 207 mmol, 2.20 equiv) was added dropwise. The reaction mixture was allowed to slowly warm to room temperature and stirred for 8 h. The resulting pale yellow slurry was then quenched with H₂O (300 mL) with vigorous evolution of H₂ gas observed. The reaction mixture was allowed to stir for 5 h and the aqueous layer extracted with Et₂O (3 × 100 mL). The combined organic layers were washed with brine, dried (MgSO₄), filtered and concentrated under reduced pressure. Flash chromatography on silica (100 % hexanes to 1% EtOAc in hexanes) provided 2a (6.2 g, 25.0 mmol, 53% yield) as a colorless oil: **R*_{f}***0.45 (1% EtOAc in hexanes); **¹H NMR** (500 MHz, CDCl₃) δ 7.54 (d, *J* = 7.1 Hz, 1H), 7.39 (td, *J* = 7.5, 1.0 Hz, 1H), 7.28 (t, *J* = 7.2 Hz, 1H), 7.22 (d, *J* = 7.5 Hz, 1H), 5.22 (dd, *J* = 8.0, 3.3 Hz, 1H), 1.92 (m, 1H), 1.59 (m, 1H), 1.52-1.30 (m, 4H), 0.99 (t, *J* = 7.7 Hz, 3H), 0.94-0.88 (m, 6H), 0.88-0.78 (m, 4H); **¹³C NMR** (125 MHz, CDCl₃) δ 154.2, 133.7, 131.6, 129.6, 126.8, 122.3, 82.12, 39.1, 27.8, 22.9, 14.2, 7.38, 7.16, 6.88, 6.60; **IR** (neat) 3058 (m), 2956 (s), 2932 (m), 2874 (m), 1459 (s), 1080 (s), 1013 (m), 916 (s), 741 (s) cm⁻¹; **HRMS** (CI⁺) *m*/*z* calculated for C₁₅H₂₄OSi [M]⁺ 248.1596, found 248.1593.

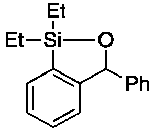

### Reference Example

**1,1-Diethyl-3-phenyl-1,3-dihydrobenzo[*c*][1,2]oxasilole (2b):** To the previously prepared (2-bromophenyl)(phenyl)methanol (**S1**) (4.16 g, 15.8 mmol, 1.00 equiv) dissolved in THF (120 mL) was added *n*-BuLi (14.6 mL, 2.39 M in hexanes, 34.8 mmol, 2.20 equiv) dropwise at -78 °C. The reaction mixture was stirred for 1 h, followed by the addition of Et₂SiHCl (4.84 mL, 34.8 mmol, 2.20 equiv) at -78 °C. The resulting pale yellow slurry was then quenched with H₂O (300 mL) with vigorous evolution of H₂ gas observed. The reaction mixture was allowed to stir for 5 h and the aqueous phase was extracted with Et₂O (2 x 50 mL), the combined organic layers were washed with brine, dried (MgSO₄), filtered and concentrated under reduced pressure. Flash chromatography on silica (100% hexanes to 5% Et₂O in hexanes) provided **2b** (1.74 g, 6.48 mmol, 41% yield) as a colorless oil: **R***_{f}*0.45 (5% Et₂O in hexanes); **¹H NMR** (500 MHz, CDCl₃) δ 7.63-7.60 (m, 1 H), 7.36-7.27 (m, 7 H), 7.02 (d, *J* = 7.1 Hz, 1 H), 6.16 (s, 1 H), 1.08 (t, *J* = 7.9 Hz, 3 H), 1.02-0.95 (m, 5 H), 0.95-0.82 (m, 2 H); **¹³C NMR** (125 MHz, CDCl₃) δ 153.2, 143.9, 133.6, 131.4, 129.9, 128.6, 128.0, 127.5, 127.1, 123.9, 84.4, 7.4, 7.1, 7.0, 6.7; **IR** (neat) 3059 (m), 2999 (m), 2956 (s), 2878 (s), 1595 (m), 1486 (m), 1451 (s), 1412 (m), 1343 (w), 1265 (m), 1236 (m), 1182 (m), 1133 (m), 1012 (bs), 964 (m), 926 (m), 871 (m), 817 (s), 739 (bs), 661 (m), 629 (m) cm⁻¹; **HRMS** (CI⁺) *m*/*z* calculated for C₁₇H₂₁SiO [M+H]⁺ 269.1362, found 269.1366.

### Reference Example

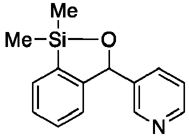

**3-(1,1-Dimethyl-1,3-dihydrobenzo[*c*][1,2]oxasilol-3-yl)pyridine (3d):** To a cooled solution of Et₂O (420 mL) containing *n*-BuLi (48.7 mL, 2.00 M in hexanes, 1.30 equiv) at -78 °C was added 3-bromopyridine (20.0 g, 127 mmol, 1.20 equiv) dropwise. The resulting yellow slurry was allowed to stir at -78 °C for 30 min. 2-Bromobenzaldehyde (19.5 g, 106 mmol, 1.00 equiv) was added dropwise and the reaction mixture was allowed to stir for 5 h at -78 °C. The reaction was then warmed to 0 °C and quenched with 3M aq. HCl (100 mL). The organic layer was washed with another portion of 3M aq. HCl (50 mL). The acidic aqueous layers were collected and neutralized to pH 8-9 with 1M aq. NaOH (350 mL), producing a white turbid mixture that was extracted with Et₂O (3 × 150 mL). The combined organic layers were dried (MgSO₄), filtered and concentrated under reduced pressure. Flash chromatography on silica (0.5% to 1% to 5% MeOH in CH₂Cl₂) provided (2-bromophenyl)(pyridin-3-yl)methanol **(S6)** (23.6 g, 89.4 mmol, 85% yield) as an off-white crystalline solid: Analytical data matches that which has been previously reported:¹⁰ **R***_{f}*0.35 (5% MeOH in CH₂Cl₂); **¹H NMR** (500 MHz, CDCl₃) δ 8.57 (d, *J* = 1.8 Hz, 1H), 8.41 (dd, *J* = 4.7, 1.1 Hz, 1H), 7.69 (td, *J* = 7.6, 1.6 Hz, 1H), 7.62 (dd, *J* = 7.8, 1.2 Hz, 1H), 7.53 (dd, *J* = 7.9, 1.0 Hz, 1H), 7.36 (t, *J* = 7.5 Hz, 1 H), 7.23 (dd, *J* = 7.8, 4.8 Hz, 1H), 7.16 (td, *J* = 7.5, 1.5 Hz, 1H), 6.21 (s, 1 H), 3.93 (s, 1 H); **¹³C NMR** (125 MHz, CDCl₃) δ 148.9, 148.7, 142.1, 138.2, 134.9, 133.1, 129.6, 128.5, 128.1, 123.6, 122.7, 72.7.

To a cooled solution of (2-bromophenyl)(pyridin-3-yl)methanol **(S6)** (7.00 g, 26.5 mmol, 1.00 equiv) in a mixture (1:1) of THF (100 mL) and Et₂O (100 mL) at -78 °C was added *n*-BuLi (29.2 mL, 2.00 M in hexanes, 2.20 equiv) dropwise. The resulting dark yellow slurry was allowed to stir at -78 °C for 30 min and Me₂SiHCl (6.50 mL, 58.3 mmol, 2.20 equiv) was added dropwise. The reaction mixture was allowed to slowly warm to room temperature and stirred for 12 h. The resulting orange slurry was then quenched with H₂O (100 mL) with vigorous evolution of H₂ gas observed. The reaction mixture was allowed to stir for 5 h and extracted with Et₂O (3 × 50 mL). The combined organic layers were collected and washed with 1M aq. HCl (3 ×50 mL). The acidic aqueous layers were collected and neutralized to pH 8-9 with 1M aq. NaOH (150 mL) producing a white turbid mixture that was extracted with Et₂O (3 × 100 mL). The combined organic layers were dried (MgSO₄), filtered and concentrated under reduced pressure. Flash chromatography on silica (0.5% to 1% to 5% MeOH in CH₂Cl₂) followed by Kugelrohr distillation (135 - 150 °C, 0.01 mmHg) provided **3d** (3.14 g, 13.0 mmol, 49% yield) as an off-white amorphous solid: **R***_{f}*0.2 (5% MeOH in CH₂Cl₂); **¹H NMR** (500 MHz, CDCl₃) δ 8.61 (d, *J* = 1.7 Hz, 1H), 8.53 (dd, *J* = 4.7, 1.5 Hz, 1H), 7.63 (m, 1 H), 7.53 (dt, *J* = 7.8, 1.8 Hz, 1H), 7.33 (m, 2 H), 7.25 (m, 1 H), 7.00 (m, 1 H), 6.20 (s, 1 H), 0.53 (s, 3 H), 0.46 (s, 3 H); **¹³C NMR** (125 MHz, CDCl₃) δ 151.4, 149.3, 148.8, 139.3, 135.2, 134.7, 131.0, 130.1, 127.6, 81.7, 1.23, 0.56; **IR** (neat) 3057 (m), 2959 (s), 1662 (m), 1579 (m), 1475 (s), 1427 (s), 1252 (s), 1181 (s), 1136 (s), 1051 (m), 860 (m), 822 (s), 791 (s), 750 (s), 713 (s) cm⁻¹; **HRMS** (ES⁺) *m*/*z* calculated for C₁₄H₁₆NOSi [M+H]⁺ 242.1001, found 242.0993.

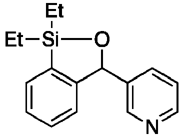

### Reference Example

**3-(1,1-Diethyl-1,3-dihydrobenzo[c][1,2]oxasilol-3-yl)pyridine (3e):** To a cooled solution of (2-bromophenyl)(pyridin-3-yl)methanol **(S6)** (7.00 g, 26.5 mmol, 1.00 equiv) in a mixture (1 : 1) of dry THF (100 mL) and dry Et₂O (100 mL) at -78 °C was added *n-*BuLi (26.5 mL, 2.20 M in hexanes, 2.20 equiv) dropwise. The resulting dark yellow slurry was allowed to stir at -78 °C for 30 min and Et₂SiHCl (8.15 mL, 58.3 mmol, 2.20 equiv) was added dropwise. The reaction mixture was allowed to slowly warm to room temperature and stirred for 12 h. The resulting red-brown solution was then quenched with H₂O (100 mL) with vigorous evolution of H₂ gas observed. The reaction mixture was allowed to stir for 5 h and extracted with Et₂O (3 × 50 mL). The combined organic layers were collected and washed with 1M aq. HCl (3 ×50 mL). The acidic aqueous layers were collected and neutralized to pH 8-9 with 1M aq. NaOH (150 mL) producing a white turbid mixture that was extracted with Et₂O (3 × 100 mL). The combined organic layers were dried (MgSO₄), filtered and concentrated under reduced pressure. Flash chromatography on silica (0.5% to 1% to 3% MeOH in CH₂Cl₂) provided **3e** (3.6 g, 13.4 mmol, 51% yield) as pale yellow oil: **R***_{f}*0.45 (5% MeOH in CH₂Cl₂); **¹H NMR** (500 MHz, CDCl₃) δ 8.62 (d, *J* = 1.0 Hz, 1H), 8.53 (dd, *J* = 4.6, 1.2 Hz, 1H), 7.62 (m, 1 H), 7.53 (dt, *J* = 7.7, 1.6 Hz, 1H), 7.33 (m, 2 H), 7.23 (m, 1 H), 6.99 (d, *J* = 6.6 Hz, 1H), 6.19 (s, 1 H), 1.05 (m, 3 H), 1.0-0.88 (m, 7 H); **¹³C NMR** (125 MHz, CDCl₃) δ 152.0, 149.4, 149.2, 139.3, 134.8, 133.6, 131.5, 130.1, 127.5, 123.7, 123.6, 81.9, 7.25, 7.00, 6.87, 6.51; **IR** (neat) 3057 (m), 2957 (m), 2876 (s), 2942 (s), 1580 (s), 1425 (m), 1235 (s), 1183 (s), 1134 (s), 1050 (m), 1017 (m), 817 (m), 744 (s) cm⁻¹; **HRMS** (ES⁺) *m*/*z* calculated for C₁₆H₂₀NOSi [M+H]⁺ 270.1314, found 270.1314.

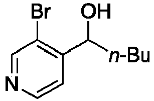

### Reference Example

**1-(3-Bromopyridin-4-yl)pentan-1-ol (S7):** To a cooled THF solution (315 mL) of LDA, prepared from diisopopylamine (15.6 mL, 110 mmol, 1.10 equiv) and *n*-BuLi (42.4 mL, 2.50 M in hexanes, 106 mmol, 1.05 equiv), 3-bromopyridine (15.8 g, 100 mmol, 1.00 equiv) was added dropwise as a solution in THF (16.6 mL) at -78 °C. The resulting solution temperature was further lowered to -100 °C and stirred for 10 min at this temperature before a THF solution (47.6 mL) of pentanal (21.4 mL, 200 mmol, 2.00 equiv) was added dropwise over 10 min. The reaction mixture was stirred at -100 °C for 1 h then warmed to -20 °C over 20 min before it was quenched with sat. aq. NH₄Cl (150 mL). The aqueous phase was extracted with EtOAc (3 x 75 mL). The combined organic layers were washed with brine, dried (MgSO₄) and concentrated under reduced pressure. Flash chromatography on silica (10% to 20% EtOAc in hexanes) provided 1-(3-bromopyridin-4-yl)pentan-1-ol **(S7)** (17.5 g, 71.7 mmol, 72% yield) as a yellow oil: **R***_{f}*0.1 (30% EtOAc in hexanes); **¹H NMR** (500 MHz, CDCl₃) δ 8.56 (s, 1 H), 8.43 (d, *J* = 5.0 Hz, 1H), 7.50 (d, *J* = 5.0 Hz, 1H), 4.98 (dd, *J* = 8.0 Hz, 3.3 Hz, 1 H), 3.03 (bs, 1 H), 1.76 (m, 1 H), 1.60 (m, 1 H), 1.51-1.31 (m, 4 H), 0.90 (t, *J* = 7.2 Hz, 3 H); **¹³C NMR** (125 MHz, CDCl₃) δ 153.4, 151.6, 148.5, 122.3, 120.1, 72.0, 37.0, 27.9, 22.6, 14.1; **IR** (neat) 3272 (bs), 2956 (m), 2929 (m), 2859 (s), 1585 (m), 1463 (s), 1401 (m), 1082 (m), 1021 (s), 845 (s) cm⁻¹; **HRMS** (CI⁺) *m*/*z* calculated for C₁₀H₁₅BrNO [M+H]⁺ 244.0259, found 244.0259.

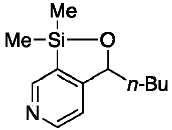

### Reference Example

**3-Butyl-1,1-dimethyl-1,3-dihydro-[1,2]oxasilolo[3,4-c]pyridine (3a):** 1-(3-bromopyridin-4-yl)pentan-1-ol **(S7)** (1.00 g, 4.10 mmol, 1.00 equiv) was dissolved in THF (27.4 mL) and *n*-BuLi (4.4 mL, 2.05 M in hexanes, 9.02 mmol, 2.20 equiv) was added dropwise at -78 °C. The reaction mixture was stirred for 30 min, followed by the addition of Me₂SiHCl (0.98 mL, 9.02 mmol, 2.20 equiv) at -78 °C. The resulting reaction mixture was allowed to warm to room temperature and was stirred for 12 h. The reaction mixture was then quenched with H₂O (20 mL) with evolution of H₂ gas observed. The reaction mixture was allowed to stir for 5 h and extracted with EtOAc (3 × 20 mL). The combined organic layers were collected and washed with 1M aq. HCl (3 × 40 mL). The acidic aqueous layers were collected and neutralized to pH 8-9 with 1M aq. NaOH (120 mL) producing a white turbid mixture that was extracted with Et₂O (3 × 75 mL). The combined organic layers were dried (MgSO₄), filtered and concentrated under reduced pressure. Flash chromatography on silica (50% EtOAc in hexanes) followed by Kugelrohr distillation (120 °C, 0.01 mmHg) provided **3a** (281 mg, 1.27 mmol, 31% yield) as an off-white amorphous solid: **R***_{f}*O.3 (70% EtOAc in hexanes); **¹H NMR** (500MHz, CDCl₃) δ 8.76 (bs, 1 H), 8.56 (d, *J* = 5.0 Hz, 1H), 7.13 (d, *J* = 5.2 Hz, 1H), 5.20 (dd, *J* = 7.5 Hz, *J* = 3.5 Hz, 1 H), 1.90 (m, 1 H), 1.59 (m, 1 H), 1.45-1.29 (m, 4 H), 0.89 (t, *J* = 7.0 Hz, 3 H), 0.44 (s, 3 H), 0.41 (s, 3 H); **¹³C NMR** (125 MHz, CDCl₃) δ 152.6, 143.9, 135.3, 130.8, 129.9, 128.7, 127.9, 127.3, 127.3, 123.9, 84.2, 1.4, 0.7; **IR** (CH₂Cl₂) 3057 (m), 2964 (m), 2874 (m), 1447 (m), 1265 (s), 1181 (m), 1136 (m), 1042 (s), 1016 (s), 862 (s), 822 (s), 793 (s), 740 (s), 702 (s), 656 (m) cm⁻¹; **HRMS** (CI⁺) *m*/*z* calculated for C₁₂H₂₀NOSi [M+H]⁺ 222.1236, found 222.1233.

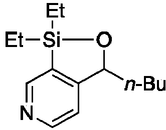

### Reference Example

**3-Butyl-1,1-diethyl-1,3-dihydro-[1,2]oxasilolo[3,4-*c*]pyridine (3b):** 1-(3-Bromopyridin-4-yl)pentan-1-ol **(S7)** (1.00 g, 4.10 mmol, 1.00 equiv) was dissolved in THF (27.4 mL) and *n*-BuLi (4.4 mL, 2.05 M in hexanes, 9.02 mmol, 2.20 equiv) was added dropwise at -78 °C. The reaction mixture was stirred for 30 min, followed by the addition of Et₂SiHCl (1.12 mL, 9.02 mmol, 2.20 equiv) at -78 °C. The resulting reaction mixture was allowed to warm to room temperature and stirred for 12 h. The slurry was quenched with H₂O (20 mL) with vigorous evolution of H₂ gas observed. The solution was allowed to stir for 5 h and extracted with EtOAc (3 × 20 mL). The combined organic layers were collected and washed with 1M aq. HCl (3 × 40 mL). The acidic aqueous layers were collected and neutralized to pH 8-9 with 1M aq. NaOH (120 mL) producing a white turbid mixture that was extracted with Et₂O (3 × 75 mL). The combined organic layers were dried (MgSO₄), filtered and concentrated under reduced pressure. Flash chromatography on silica (30% EtOAc in hexanes) followed by Kugelrohr distillation (130 °C, 0.01 mmHg) provided **3b** (540 mg, 2.17 mmol, 43% yield) as a yellow oil: **R***_{f}*0.2 (40% EtOAc in hexanes); **¹H NMR** (500 MHz, CDCl₃) δ 8.69 (bs, 1 H), 8.50 (d, *J* = 5.0 Hz, 1H), 7.09 (d, *J* = 5.0 Hz, 1H), 5.12 (dd, *J* = 7.8 Hz, *J* = 3.4 Hz, 1 H), 1.84 (m, 1 H), 1.51 (m, 1 H), 1.44-1.24 (m, 4 H), 0.93 (t, *J* = 7.5 Hz, 3 H), 0.88-0.77 (m, 10 H); **¹³C NMR** (125 MHz, CDCl₃) δ 162.9, 152.6, 149.7, 129.0, 117.7, 81.3, 38.0, 27.4, 22.6, 13.9, 7.09, 6.93, 6.55, 6.26; **IR** (CH₂Cl₂) 3057 (m), 2964 (m), 2874 (m), 1447 (m), 1265 (s), 1181 (m), 1136 (m), 1042 (s), 1016 (s), 862 (s), 822 (s), 793 (s), 740 (s), 702 (s), 656 (m) cm⁻¹; **HRMS** (CI⁺) *m*/*z* calculated for C₁₄H₂₄NOSi [M+H]⁺ 250.1549, found 250.1547.

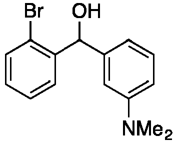

**(2-Bromophenyl)(3-(dimethylamino)phenyl)methanol (S8):** In a two-necked 200 mL round-bottom flask, Mg turnings (5.30 g, 216 mmol, 10.0 equiv) were flame-dried under vacuum. Upon cooling to room temperature, THF (55 mL), 3-bromo-*N,N-*dimethylaniline (6.50 g, 32.4 mmol, 1.50 equiv) as a solution in THF (55 mL) and a crystal of I₂ were added. The flask was fitted with a reflux condenser and the solution vigorously stirred. Once the resulting exotherm had subsided, the reaction mixture was heated to reflux (70 °C) for 1 h. This Grignard reagent was then allowed to cool to room temperature and added to a cooled solution of 2-bromobenzaldehyde (4.00 g, 21.6 mmol, 1.00 equiv) in THF (32 mL) at 0 °C via cannula over 10 min. The reaction mixture was then allowed to reach room temperature and stirred for 12 h and quenched with sat. aq. NH₄Cl (75 mL). The aqueous phase was then extracted with EtOAc (3 x 50 mL). The combined organic layers were washed with brine, dried (Na₂SO₄) and concentrated under reduced pressure. Flash chromatography on silica (1% to 5% EtOAc in hexanes) provided the desired (2-bromophenyl)(3-(dimethylamino)phenyl)methanol **(S8)** (6.36 g, 20.8 mmol, 96% yield) a yellow viscous oil: **R***_{f}*O.4 (20% EtOAc in hexanes); **¹H NMR** (500 MHz, CDCl₃) δ 7.59 (dd, *J* = 7.7, 1.6 Hz, 1 H), 7.53 (dd, *J* = 7.9, 1.1 Hz, 1 H), 7.33 (td, *J* = 7.5, 1.0 Hz, 1 H), 7.20 (t, *J* = 7.9, 1 H), 7.13 (td, *J* = 7.7, 1.6 Hz, 1 H), 6.84 (bs, 1 H), 6.72 (d, *J* = 7.5, 1 H), 6.66 (dd, *J* = 8.2, 2.2 Hz, 1 H), 6.16 (s, 1 H), 2.93 (s, 6 H), 2.38 (bs, 1 H); **¹³C NMR** (125 MHz, CDCl₃) δ 150.8, 143.3, 142.9, 132.9, 129.3, 129.1, 128.7, 127.8, 123.1, 115.3, 112.2, 111.4, 75.3, 40.8; **IR** (neat) 3392 (bs), 2917 (s), 2848 (s), 1604 (s), 1498 (s), 1437 (s), 1353 (s), 1018 (m), 996 (s), 744 (s) cm⁻¹; **HRMS** (ES⁺) *m*/*z* calculated for C₁₅H₁₇NOBr [M+H]⁺ 306.0494, found 306.0492.

### Reference Example

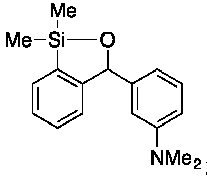

**3-(1,1-Dimethyl-1,3-dihydrobenzo[*c*][1,2]oxasilol-3-yl)-*N,N* dimethylaniline (3c):** (2-Bromophenyl)(3-(dimethylamino)phenyl)methanol **(S8)** (6.90 g, 22.5 mmol, 1.00 equiv) was dissolved in THF (90 mL) and *n*-BuLi (20.8 mL, 2.40 M in hexanes, 50.0 mmol, 2.20 equiv) was added dropwise at -78 °C. The reaction mixture was stirred for 45 min, followed by the addition of Me₂SiHCl (5.60 mL, 50.0 mmol, 2.20 equiv) at -78 °C. The resulting reaction mixture was allowed to warm to room temperature and stirred for 12 h. The slurry was quenched with H₂O (75 mL) with vigorous evolution of H₂ gas observed. The reaction mixture was allowed to stir for 5 h and extracted with EtOAc (3 × 50 mL). The combined organic layers were collected and washed with 3M aq. HCl (3 × 50 mL). The acidic aqueous layers were collected and neutralized to pH 8-9 with 1M aq. NaOH producing a white turbid mixture that was extracted with Et₂O (3 × 75 mL). The combined organic layers were dried (MgSO₄), filtered and concentrated under reduced pressure. Flash chromatography on silica (5% to 10% EtOAc in hexanes) followed by Kugelrohr distillation (150 °C, 0.01 mmHg) provided **3c** (2.8 g, 9.88 mmol, 44% yield) as an orange viscous oil: **R***_{f}*0.1 (40% EtOAc in hexanes); **¹H NMR** (500 MHz, CDCl₃) δ 7.63 (dd, *J* = 6.2,1.6 Hz, 1 H), 7.33 (m, 2 H), 7.22 (t, *J* = 7.6 Hz, 1 H), 7.13 (d, *J* = 7.4 Hz, 1 H), 6.71-6.62 (m, 3 H), 6.15 (s, 1 H), 2.94 (s, 6 H), 0.56 (s, 3 H), 0.48 (s, 3 H); **¹³C NMR** (125 MHz, CDCl₃) δ 152.8, 150.88, 144.71, 135.04, 130.6, 129.9, 129.3, 127.3, 127.1, 123.8, 115.6, 112.2, 111.6, 84.7, 40.7, 1.51, 0.65; **IR** (neat) 2953 (bs), 2874 (bs), 2802 (s), 1605 (s), 1499 (s), 1440 (s), 1353 (s), 1251 (s), 1135 (s), 1066 (s), 1043 (s), 995 (s), 870 (s), 789 (s), 743 (s) cm⁻¹; **HRMS** (ES⁺) *m*/*z* calculated for C₁₇H₂₂NOSi [M+H]⁺ 284.1471, found 284.1476.

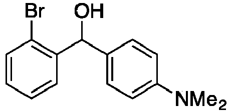

**(2-Bromophenyl)(4-(dimethylamino)phenyl)methanol (S9):** In a two-necked 500 mL round-bottom flask, Mg turnings (10.5 g, 432 mmol, 10.0 equiv) were flame-dried under vacuum. Upon cooling to room temperature, THF (72 mL), 4-bromo-*N,N-*dimethylaniline (12.9 g, 64.9 mmol, 1.50 equiv) as a solution in THF (72 mL) and a crystal of I₂ were added. The flask was fitted with a reflux condenser and the solution was vigorously stirred. Once the resulting exotherm had subsided, the reaction mixture was heated to reflux (70 °C) for 1 h. This Grignard reagent was then allowed to cool to room temperature and added to a cooled solution of 2-bromobenzaldehyde (8.00 g, 43.2 mmol, 1.00 equiv) in THF (87 mL) at 0°C via cannula over 10 min. The reaction was then allowed to reach room temperature and stirred for 12 h and quenched with sat. aq. NH₄Cl (100 mL). The aqueous phase was then extracted with EtOAc (3 x 50 mL). The combined organic layers were washed with brine, dried (Na₂SO₄) and concentrated under reduced pressure. Flash chromatography on silica (3% to 10% EtOAc in hexanes) provided of the desired (2-bromophenyl)(4-(dimethylamino)phenyl)methanol **(S9)** (12.6 g, 41.2 mmol, 95% yield) as a blue crystalline solid: **R***_{f}*0.2 (10% EtOAc in hexanes); **¹H NMR** (500MHz, CDCl₃) δ 7.71 (dd, *J* = 7.7, 1.5 Hz, 1 H), 7.53 (dd, *J* = 7.9, 1.0 Hz, 1 H), 7.37 (td, *J* = 7.6, 1.0 Hz, 1 H), 7.24 (m, 2 H), 7.14 (td, *J* = 7.7, 1.5 Hz, 1 H), 6.69 (m, 2 H), 6.07 (s, 1 H), 2.94 (s, 6 H), 2.58 (bs, 1 H); **¹³C NMR** (125 MHz, CDCl₃) δ 150.3, 143.2, 132.9, 130.3, 128.8, 128.6, 128.4, 128.3, 127.7, 122.8, 112.8, 112.6, 112.3, 74.8, 40.7; **IR** (neat) 3387 (bs), 2887 (m), 2802 (s), 1613 (s), 1521 (s), 1352 (s), 1162 (s), 1017 (m), 810 (s), 758 (s), 741 (s) cm⁻¹; **HRMS** (ES⁺) *m*/*z* calculated for C₁₅H₁₇NOBr [M+H]⁺ 306.0494, found 306.0497.

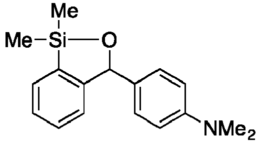

### Reference Example

**4-(1,1-Dimethyl-1,3-dihydrobenzo[*c*][1,2]oxasilol-3-yl)-*N,N*-dimethylaniline (3f):** (2-Bromophenyl)(4-(dimethylamino)phenyl)methanol **(S9)** (2.33 g, 7.61 mmol, 1.00 equiv) was dissolved in THF (25 mL) and *n*-BuLi (7.6 mL, 2.20 M in hexanes, 16.7 mmol, 2.20 equiv) was added dropwise at -78 °C. The reaction mixture was stirred for 45 min, followed by the addition of Me₂SiHCl (2.00 mL, 16.7 mmol, 2.20 equiv) at -78 °C. The resulting reaction mixture was allowed to warm to room temperature and stirred for 12 h. The slurry was quenched with H₂O (50 mL) with vigorous evolution of H₂ gas observed. The reaction mixture was allowed to stir for 5 h and extracted with EtOAc (3 × 25 mL). The combined organic layers were collected and washed with 3M aq. HCl (3 × 25 mL). The acidic aqueous layers were collected and neutralized to pH 8-9 with 1M aq. NaOH producing a white turbid mixture that was extracted with Et₂O (3 × 75 mL). The combined organic layers were dried (MgSO₄), filtered and concentrated under reduced pressure. Flash chromatography on silica (5% Et₂O in hexanes) provided **3f** (795 mg, 2.81 mmol, 37% yield) as an yellow crystalline solid: **R*_{f}*** 0.1 (25% EtOAc in hexanes); **¹H NMR** (500 MHz, CDCl₃) δ 7.63 (dd, *J* = 6.0, 1.8 Hz, 1 H), 7.33 (m, 2 H), 7.14 (m, 2 H), 7.06 (m, 1 H), 6.72 (m, 2 H), 6.14 (s, 1 H), 2.95 (s, 6 H), 0.52 (s, 3 H), 0.46 (s, 3 H); **¹³C NMR** (125 MHz, CDCl₃) δ 153.3, 150.5, 135.5, 132.1, 130.6, 129.8, 128.5, 128.3, 127.1, 124.1, 112.7, 84.2, 40.8, 1.57, 0.66; **IR** (neat) 3419 (bs), 2921 (m), 1615 (s), 1523 (s), 1443 (s), 1348 (s), 1251 (s), 1163 (s), 1134 (s), 861 (s), 822 (s), 789 (s), 743 (s) cm⁻¹; **HRMS** (CI⁺) *m*/*z* calculated for C₁₇H₂₂NOSi [M+H]⁺ 284.1471, found 284.1459.

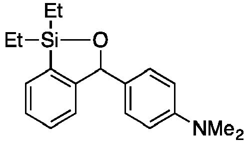

### Reference Example

**4-(1,1-Diethyl-1,3-dihydrobenzo[*c*][1,2]oxasilol-3-yl)-*N,N-*dimethylaniline (3g):** (2-Bromophenyl)(4-(dimethylamino)phenyl)methanol **(S9)** (7.00 g, 22.9 mmol, 1.00 equiv) was dissolved in THF (230 mL), *n*-BuLi (22.9 mL, 2.20 M in hexanes, 50.4 mmol, 2.20 equiv) was added dropwise at -78 °C. The reaction mixture was stirred for 45 min, followed by the addition of Et₂SiHCl (7.00 mL, 50.4 mmol, 2.20 equiv) at -78 °C. The resulting reaction mixture was allowed to warm to room temperature and stirred for 12 h. The slurry was quenched with H₂O (100 mL) with vigorous evolution of H₂ gas observed. The reaction mixture was allowed to stir for 5 h and extracted with EtOAc (3 × 50 mL). The combined organic layers were collected and washed with 3M aq. HCl (3 × 50 mL). The acidic aqueous layers were collected and neutralized to pH 8-9 with 1M aq. NaOH (300 mL) producing a white turbid mixture that was extracted with Et₂O (3 × 100 mL). The combined organic layers were dried (MgSO₄), filtered and concentrated under reduced pressure. Flash chromatography on silica (5% EtOAc in hexanes) provided **3g** (3.6 g, 11.6 mmol, 51% yield) as an yellow oil: **R***_{f}*O.3 (15% EtOAc in hexanes); **¹H NMR** (500 MHz, CDCl₃) δ 7.64 (dd, *J* = 6.0, 1.5 Hz, 1 H), 7.37-7.30 (m, 2 H), 7.17-7.15 (m, 2 H), 7.06 (m, 1 H), 6.74- 6.70 (m, 2 H), 6.15 (s, 1 H), 2.95 (s, 6 H), 1.08 (m, 3 H), 1.04-0.88 (m, 7 H); ^{**1**3}C **NMR** (125 MHz, CDCl₃) δ 153.8, 150.4, 133.8, 132.0, 131.2, 129.7, 128.7, 126.8, 124.1, 112.5, 84.3, 40.7, 7.43, 7.11, 6.99, 6.65; **IR** (neat) 2955 (m), 2875 (m), 2800 (s), 1614 (s), 1523 (s), 1444 (m), 1348 (s), 1230 (s), 1162 (s), 1132 (s), 997 (m), 949 (s), 823 (m), 723 (m) cm⁻¹; **HRMS** (ES⁺) *m*/*z* calculated for C₁₉H₂₆NOSi [M+H]⁺ 312.1784, found 312.1783.

**General Procedure A:** To a cooled solution of siloxane (0.81 mmol, 1.8 equiv) in dry Et₂O (1.2 mL) at room temperature was added a solution of PhLi in Bu₂O (0.68 mmol, 1.5 equiv) and allowed to stir for 2 h. After 1.5 h had elapsed following PhLi addition, in a separate flask were combined PdCl₂ (2.50 mg, 0.014 mmol, 0.03 equiv), CuI (8.60 mg, 0.045 mmol, 0.1 equiv) and dpca (6.7 mg, 0.018 mmol, 0.04 equiv) in dry THF (1 mL) at room temperature and stirred for 30 min. The aryl halide (0.45 mmol, 1.0 equiv) was added to the orange slurry, followed by addition of the siloxane/PhLi reaction mixture by cannula (flask rinsed with 0.5 mL of THF). After 2 h at room temperature, the reaction mixture was diluted with Et₂O (2 mL) and quenched according to the siloxane used in the reaction; **1, 1a-f, 2a-d** quenched with sat. aq. NH₄Cl (5 mL); **3a-b** and **3d-e** quenched with 1M aq. HCl (5 mL); **3c** and **3f-g** quenched with 3M aq. HCl (5 mL).

For siloxanes **1, 1a-f, 2a-d** the aqueous layer was extracted with Et₂O (3 × 5 mL) and the combined organic layers were dried (MgSO₄), filtered, and concentrated under reduced pressure. Flash chromatography provided the cross-coupled product and the recovered siloxane (where possible as with siloxanes **2a-d).**

For siloxanes **3a-g,** the organic layer was washed with either 1M or 3M aq. HCl (3 × 5 mL) (according to siloxane, see above), and the acidic aqueous layers were collected. The organic layer was then washed with sat. aq. NaHCO₃ (5 mL), dried (MgSO₄), filtered, and concentrated under reduced pressure. Flash chromatography provided the cross-coupled product. The acidic aqueous layer was then basified to pH 8-9 with 1M aq. NaOH, producing a white turbid mixture that was then extracted with Et₂O 3 × 50 mL). The combined organic layers were dried (MgSO₄), filtered, and concentrated under reduced pressure to provide the recovered siloxane.

### Reference Example

### Preparation of fluorinated siloxanes.

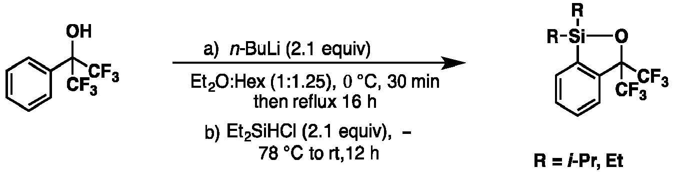

**Procedure:** To a cooled solution of (Hexafluoro-2-hydroxyisopropyl)benzene (3.00 g, 12.3 mmol, 1.00 equiv) in hexanes (75 mL, pre-dried over MgSO₄) and Et₂O (60 mL) at 0 °C was added *n*-BuLi (12.6 mL, 2.15 M in hexanes, 27.1 mmol, 2.20 equiv) dropwise under N₂. The reaction mixture was warmed to room temperature and stirred for 30 minutes. The flask was then fitted with a reflux condenser and the reaction mixture was heated to reflux (70 °C) for 16 h. A dark brown solution resulted and the reaction mixture was allowed to cool to room temperature. The reflux condenser was removed and the reaction mixture was cooled to -78 °C before either *i*-Pr₂SiHCl (4.60 mL, 27.06 mmol, 2.20 equiv) or Et₂SiHCl was added dropwise under N₂. The reaction mixture was allowed to slowly warm to room temperature and stirred for 12 h. The resulting pale yellow slurry was then quenched with H₂O (75 mL) and the aqueous phase extracted with Et₂O (3 × 40 mL). The combined organic layers were washed with brine, dried (MgSO₄), filtered and concentrated under reduced pressure. Flash chromatography (100% hexanes) provided 2.76 g (63% yield) of the desired diisopropyl siloxane as a white crystalline solid (m.p. 39-41 °C).

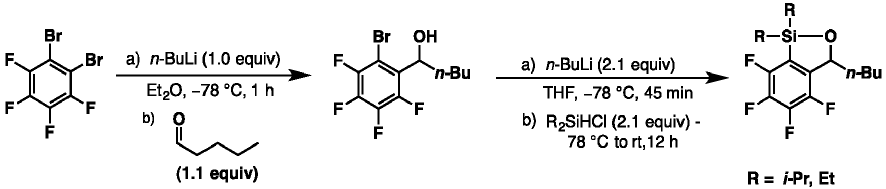

**Procedure:** To a cooled solution of 1,2-Dibromotetrafluorobenzene (5.00 g, 16.2 mmol, 1.00 equiv) in Et₂O (162 mL) at -78 °C was added *n*-BuLi (7.75 mL, 2.10 M in hexanes, 16.2 mmol, 1.00 equiv) dropwise over 30 min under N₂. The reaction mixture was stirred for another 30 minutes before pentanal (1.90 mL, 17.9 mmol, 1.10 equiv) was added dropwise. The reaction mixture was stirred for 5 h at -78 °C before it was warmed to 0 °C and quenched with H₂O (75 mL). The aqueous phase extracted with Et₂O (3 × 30 mL). The combined organic layers were washed with brine, dried (MgSO₄), filtered and concentrated under reduced pressure. Flash chromatography (2% to 5% ethyl acetate in hexanes) provided 4.34 g (85% yield) of the perfluorinated bromo benzylic alcohol.

The resulting perfluorinated bromo benzylic alcohol (332 mg, 1.05 mmol, 1.00 equiv) was dissolved in THF (5.5 mL) and *n*-BuLi (1.1 mL, 2.10 M in hexanes, 2.21 mmol, 2.10 equiv) was added dropwise at -78 °C. The reaction mixture was stirred for 45 min followed by the addition of either iPr₂SiHCl (0.40 mL, 2.32 mmol, 2.20 equiv) or Et₂SiHCl at -78 °C. The resulting reaction mixture was allowed to warm to room temperature and stirred for 12 h. The reaction mixture was quenched by addition of H₂O (20 ml) and stirred for 1 h. The aqueous phase was then extracted with Et₂O (2 x 15 mL) and the combined organic layers were washed with brine, dried (MgSO₄), and concentrated under reduced pressure. Flash chromatography (100% hexanes) provided the desired siloxane (223 mg, 61% yield) as a light yellow oil.

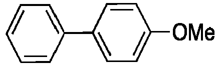

**4-Methoxy-1,1'-bipheny (S10):** Following General Procedure A, the product was purified by chromatography on SiO₂ (1% EtOAc in hexanes) to afford **S7** (80.0 mg, 0.43 mmol, 96% with siloxane **2a;** 81.0 mg, 0.44 mmol, 98% with siloxane **2b;** 81.0 mg, 0.44 mmol, 98% with siloxane **3e;** 81.0 mg, 0.44 mmol, 98% with siloxane **3g)** as a colorless solid. Analytical data matches that which has been previously reported for **S10**:¹¹ **R***_{f}*O.5 (1% EtOAc in hexanes); **¹H NMR** (500 MHz, CDCl₃) δ 7.57-7.53 (m, 4 H), 7.42 (t, *J* = 7.6 Hz, 2 H), 7.31 (t, *J* = 7.4 Hz, 1 H), 6.99 (d, *J* = 8.7 Hz, 2 H), 3.86 (s, 3 H); **¹³C NMR** (125 MHz, CDCl₃) δ 159.3, 141.0, 133.9, 128.9, 128.3, 126.9, 126.8, 114.3, 55.5.

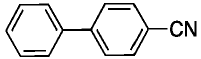

**[1,1'-Biphenyl]-4-carbonitrile (S11):** Following General Procedure A, the product was purified by chromatography on SiO₂ (1% EtOAc in hexanes) to afford **S8** (74.0 mg, 0.41 mmol, 92% with siloxane **2a;** 73.0 mg, 0.40 mmol, 91% with siloxane **2b;** 77.0 mg, 0.43 mmol, 96% with siloxane **3e;** 75.0 mg, 0.42 mmol, 94% with siloxane **3g** as a colorless solid. Analytical data matches that which has been previously reported for **S11**:¹² **R***_{f}*O.3 (1% EtOAc in hexanes); **¹H NMR** (500 MHz, CDCl₃) 7.73 (d, *J* = 8.3 Hz, 2 H), 7.69 (d, *J* = 8.4 Hz, 2 H), 7.59 (d, *J* = 7.4 Hz, 2 H), 7.49 (t, *J* = 7.2 Hz, 2 H), 7.43 (t, *J* = 7.3 Hz, 1 H); **¹³C NMR** (125 MHz, CDCl₃) δ 145.8, 139.3, 132.8, 129.3, 128.8, 127.9, 127.4, 119.1, 111.1.

**General Procedure B:** To a solution of alkenyl iodide or aryl iodide (0.68 mmol, 1.50 equiv) in Et₂O (1 mL) at -78 °C was added *t*-BuLi in pentane (1.35 mmol, 3.00 equiv), and a white or yellow (depending on alkenyl iodide or aryl idodide) slurry developed. The reaction mixture was allowed to stir for 40 min at -78 °C and 20 min at room temperature, at which time a solution of siloxane (0.81 mmol, 1.80 equiv) in THF (0.5 mL + 0.2 mL rinse) was added and allowed to stir at room temperature for 2 h. After 1.5 h had elapsed following siloxane addition, in a separate flask were combined PdCl₂ (2.50 mg, 0.014 mmol, 0.03 equiv), CuI (8.60 mg, 0.045 mmol, 0.1 equiv) and dpca (6.7 mg, 0.018 mmol, 0.04 equiv) in dry THF (1 mL) at room temperature and stirred for 30 min. The alkenyl halide or aryl iodide (0.45 mmol, 1.00 equiv) was added as a solution in THF (0.3 mL) to the orange slurry, immediately followed by addition of the siloxane reaction mixture by cannula (flask rinsed with 0.5 mL of THF). After 2-12 h at room temperature, the reaction mixture was diluted with Et₂O (2 mL) and quenched according to the siloxane used in the reaction; **1, 1a-f, 2a-d** quenched with sat. aq. NH₄Cl (5 mL); **3a-b** and **3d-e** quenched with 1M aq. HCl (5 mL); **3c** and **3f-g** quenched with 3M aq. HCl (5 mL).

For siloxanes **1, 1a-f, 2a-d,** the aqueous layer was extracted with Et₂O (3 × 5 mL) and the combined organic layers were dried (MgSO₄), filtered, and concentrated under reduced pressure. Flash chromatography provided the cross-coupled product and the recovered siloxane (where possible as with siloxanes **2a-d).**

For siloxanes **3a-g,** the organic layer was washed with either 1M or 3M aq. HCl (3 × 5 mL) (according to siloxane, see above), and the acidic aqueous layers were collected. The organic layer was then washed with sat. aq. NaHCO₃ (5 mL), dried (MgSO₄), filtered, and concentrated under reduced pressure. Flash chromatography provided the cross-coupled product. The acidic aqueous layer was then basified to pH 8-9 with 1M aq. NaOH, producing a white turbid mixture which was then extracted with Et₂O 3 × 50 mL). The combined organic layers were dried (MgSO₄), filtered, and concentrated under reduced pressure to provide the recovered siloxane.

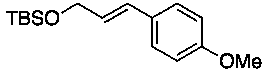

**(*E*)-*tert*-Butyl((3-(4-methoxyphenyl)allyl)oxy)dimethylsilane (S12):** Following General Procedure B, the product was purified by chromatography on SiO₂ (1% EtOAc in hexanes) to afford **S9** in >20:1 E/Z ratio as a colorless oil (110 mg, 0.40 mmol, 88% with siloxane **2a;** 115 mg, 0.41 mmol, 92% with siloxane **2b;** 124 mg, 0.44 mmol, 99% with siloxane **3e;** 121 mg, 0.44 mmol, 97% with siloxane **3g).** Analytical data matches that which has been previously reported for **S12:**¹³ **R***_{f}*O.3 (1% EtOAc in hexanes); **¹H NMR** (500 MHz, CDCl₃) δ 7.31 (d, *J* = 8.8 Hz, 2 H), 6.85 (d, *J* = 8.8 Hz, 2 H), 6.53 (d, *J* = 16.0 Hz, 1 H), 6.15 (dt, *J* = 5.2, 16.0 Hz, 1 H), 4.33 (d, *J* = 5.3 Hz, 2 H), 3.81 (s, 3 H), 0.94 (s, 9 H), 0.11 (s, 6 H); **¹³C NMR** (125 MHz, CDCl₃) δ 159.2, 130.1, 129.3, 127.7, 127.1, 114.07, 64.2, 55.4, 26.1, 18.6, -4.97.

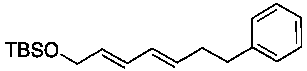

***tert*-Butyldimethyl(((2*E*,4*E*)-7-phenylhepta-2,4-dien-1-yl)oxy)silane (S13):** Following General Procedure B, the product was purified by chromatography on SiO₂ (1% EtOAc in hexanes) to afford **S13** in >20:1 E/Z ratio as a colorless oil (125 mg, 0.41 mmol, 92% with siloxane **2a;** 124 mg, 0.41 mmol, 91% with siloxane **2b;** 131 mg, 0.43 mmol, 96% with siloxane **3e;** 135 mg, 0.44 mmol, 99% with siloxane **3g): R***_{f}*0.3 (1% EtOAc in hexanes); **¹H NMR** (500 MHz, CDCl₃) δ 7.31-7.25 (m, 2 H), 7.21-7.17 (m, 3 H), 6.19 (dd, *J* = 10.6, 15.0 Hz, 1 H), 6.08 (dd, *J* = 10.6, 15.0 Hz, 1 H), 5.75-5.62 (m, 2H), 4.21 (d, *J* = 5.5 Hz, 2 H), 2.72 (t, *J* = 7.5 Hz, 2 H), 2.41 (q, *J* = 7.5 Hz, 2 H), 0.93 (s, 9 H), 0.83 (s, 6 H); **¹³C NMR** (125 MHz, CDCl₃) δ 141.9, 133.4, 132.4, 130.8, 130.4, 130.3, 129.5, 128.6, 128.5, 126.0, 63.8, 63.7, 35.9, 34.6, 29.9, 26.1, 18.6, -5.01. **IR** (neat) 3026 (m), 2954 (m), 2930 (s), 2856 (s) 1468 (m), 1254 (s), 1109 (m), 1066 (bs), 988 (s), 837 (s), 776 (s) cm⁻¹; **HRMS** (CI⁺) *m*/*z* calculated for C₁₅H₂₁OSi [M-C₄H₉]⁺ 245.1362, found 245.1368.

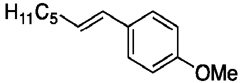

**(*E*)-1-(Hept-1-en-1-yl)-4-methoxybenzene (S14):** Following General Procedure B, the product was purified by chromatography on SiO₂ (1% EtOAc in hexanes) to afford **S14** in >20:1 E/Z ratio as a colorless oil (89.0 mg, 0.44 mmol, 97% with siloxane **2a;** 88.0 mg, 0.43 mmol, 96% with siloxane **2b;** 87.0 mg, 0.43 mmol, 95% with siloxane **3e;** 87.0 mg, 0.42 mmol, 94% with siloxane **3g)** as a colorless solid. Analytical data matches that which has been previously reported for **S14**:¹⁴ **R***_{f}*0.3 (1% EtOAc in hexanes); **¹H NMR** (500 MHz, CDCl₃) δ 7.28 (dd, *J* = 2.0, 6.5 Hz, 2 H), 6.84 (dd, *J* = 2.1, 6.6 Hz, 2 H), 6.32 (d, *J* = 15.7 Hz, 1 H), 6.09 (dt, *J* = 7.2, 15.8 Hz, 1 H), 3.80 (s, 3 H), 2.18 (qd, *J* = 1.3, 7.3 Hz, 2 H), 1.46 (qn, *J* = 7.2 Hz, 2 H), 1.40-1.37 (m, 4 H), 0.90 (t, *J* = 7.1 Hz, 3 H); **¹³C NMR** (125 MHz, CDCl₃) δ 158.6, 131.2, 129.6, 129.5, 127.1, 114.2, 55.7, 33.5, 31.4, 29.5, 22.7, 14.1.

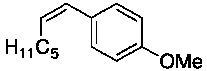

**(*Z*)-1-(Hept-1-en-1-yl)-4-methoxybenzene (S15):** Following General Procedure A, the product was purified by chromatography on SiO₂ (1% EtOAc in hexanes) to afford **S15** in >20:1 Z/E ratio as a colorless oil (80.0 mg, 0.39 mmol, 87% with siloxane **2a;** 82.0 mg, 0.40 mmol, 89 % with siloxane **2b;** 88.0 mg, 0.43 mmol, 96% with siloxane **3e,** 87.0 mg, 0.42 mmol, 94% with siloxane **3g).** Analytical data matches that which has been previously reported for **S15** (Denmark, S. E.; Sweis, R. F. J. Am. Chem. Soc. 2001, 123, 6439). **R***_{f}*O.3 (1% EtOAc in hexanes); **¹H NMR** (500 MHz, CDCl₃) δ 7.23 (d, *J* = 8.6 Hz, 2 H), 6.87 (d, *J* = 8.8 Hz, 2 H), 6.33 (d, *J* = 11.7 Hz, 1 H), 5.55 (dt, *J* = 7.3, 11.6 Hz, 1 H), 3.82 (s, 3 H), 2.29 (qd, *J* = 1.3, 7.3 Hz, 2 H), 1.45 (qn, *J* = 7.6 Hz, 2 H), 1.36-1.28 (m, 4 H), 0.90 (t, *J* = 7.2 Hz, 3 H); **¹³C NMR** (125 MHz, CDCl₃) δ 158.6, 131.7, 130.5, 130.1, 128.4, 113.5, 55.1, 31.4, 29.7, 28.9, 22.6, 14.2.

### Preparation of Siloxane Polymers

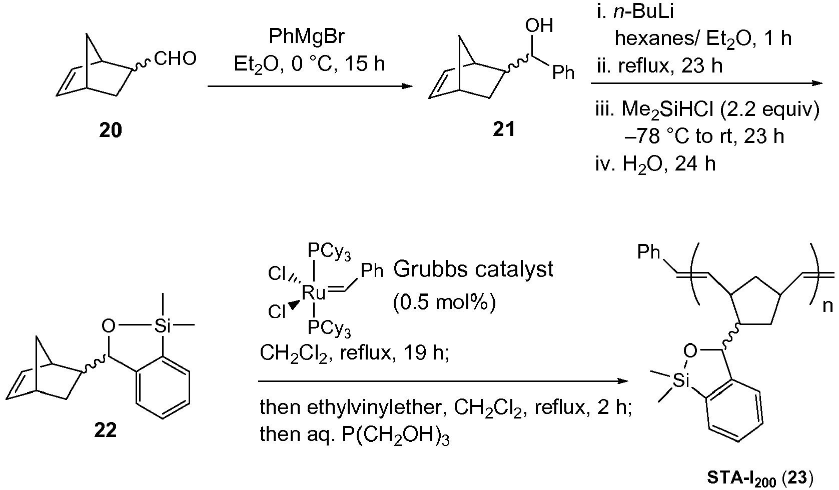

To a stirred solution of aldehyde **20** (5.42 g, 44.4 mmol, mixture of *endo* and *exo* isomers) in THF (200 mL) at 0 °C was added solution of PhMgBr in Et₂O (17.8 mL, 3.00 M, 53.3 mmol) dropwise. The solution was stirred for 15 h at room temperature and then diluted with sat. aq. NH₄Cl (50 mL). The aqueous phase was extracted with Et₂O and the combined organic layers were washed with brine, dried with MgSO₄, filtered and concentrated under reduced pressure. The crude product was purified by column chromatography on SiO₂ (5% Et₂O/ hexanes) to afford **21** (7.72 g, 87%, mixture of 4 diastereomers) as a pale yellow oil.

To a stirred solution of alcohol **21** (6.83 g, 34.1 mmol) in hexanes (125 mL, dried over MgSO₄) and Et₂O (100 mL) at 0 °C was added *n*-BuLi in hexanes 35.8 mL, 2.10 M, 75.1 mmol) dropwise. The solution was stirred at room temperature for 1 h, at which time the solution was heated to reflux for 23 h. The solution was then cooled to -78 °C and Me₂SiHCl (8.16 mL, 75.1 mmol) was added dropwise. The solution was warmed to room temperature, and after 23 h, H₂O (100 mL) was added and the biphasic mixture was stirred for another 24 h. The aqueous phase was extracted with hexanes and the combined organic layers were washed with brine, dried with MgSO₄, filtered and concentrated under reduced pressure. The crude product was purified by column chromatography on SiO₂ (0-1% Et₂O/hexanes; the column was slurry-packed using SiO₂ in H₂O, which was then washed with acetone, EtOAc, and hexanes, successively, prior to chromatographic separation), followed by Kugelrohr distillation at 120-130 °C (0.025 mmHg) to afford **22** as a colorless oil.

Grubbs' 1^{st} generation catalyst (6.1 mg, 7.4 µmol) was dissolved in CH₂Cl₂ (0.5 mL) and the solution was stirred for 30 min. The catalyst solution was then introduced via cannula to another flask containing **22** (378 mg, 1.48 mmol) in CH₂Cl₂ (1 mL), and the resulting solution was then stirred at room temperature. After 17 h, the reaction mixture was heated to reflux. After 19 h, the reaction was quenched with ethylvinylether (0.5 mL) in CH₂Cl₂ (1.5 mL), and heating of the mixture at 50 °C was continued for another 2 h. The solution was then cooled to room temperature and diluted with CH₂Cl₂ (5 mL). Tris(hydroxymethyl)phosphine (53.9 mg, 0.3 eq.) in H₂O (10 mL) was added to the solution and the biphasic mixture was stirred vigorously for 15 min. The aqueous phase was extracted with CH₂Cl₂ and the combined organic layers were washed with brine, dried with MgSO₄, filtered and concentrated under reduced pressure. The obtained white solid was washed extensively with MeCN and dried under vacuum to afford **23** (363 mg, 96%, Mₙ = 73689, PDI = 1.272) as a white solid. n is about 250.

### Polymer-Mediated Cross-Coupling

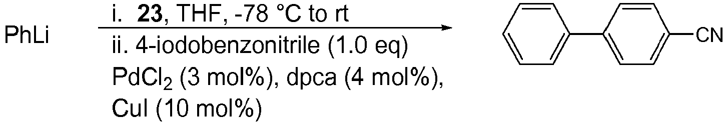

To a stirred solution of polymer **23** (196 mg, see above) in THF (4 mL) at -78 °C was added PhLi in dibutylether (479 µL, 1.23 M, 0.575 mmol) dropwise, followed by THF (6 mL). The reaction mixture was stirred for 3 h at room temperature and a colorless slurry resulted. A mixture of 4-iodobenzonitrile (87.7 mg, 0.383 mmol), PdCl₂ (2.0 mg, 0.011 mmol, 3 mol%), CuI (7.3 mg, 0.038 mmol,), and dpca (N-[2-(Diphenylphosphino)benzylidene]cyclohexylamine, 5.7 mg, 0.015 mmol) was added to the flask in a single portion and the slurry was stirred for 2 d at room temperature. The reaction mixture was diluted with sat. aq. NH₄Cl (5 ml), and the aqueous phase was extracted with Et₂O. The combined organic layers were washed with brine, dried with MgSO₄, filtered and concentrated under reduced pressure. The resulting residue was washed extensively with acetonitrile, and the washings were combined and concentrated to obtain the crude product, which was purified by chromatography on SiO₂ (1% Et₂O/hexanes) to afford the product (26.1 mg, 0.146 mmol, 38%) as a white solid.

### Alternative procedure for polymer-mediated cross-coupling

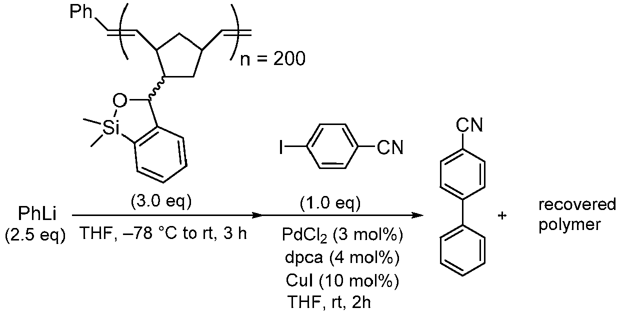

To a cooled solution of siloxane polymer in THF (0.193 g, 0.752 mmol, 3.0 equiv, 10 mg/mL) at -78 °C was added PhLi in Bu₂O (348 µL, 1.8 M, 0.627 mmol, 2.5 equiv) dropwise. The reaction mixture was allowed to warm to rt and was stirred for 3 h, and a white, cloudy solution developed. A solid mixture of PdCl₂ (1.3 mg, 7.5 µmol, 0.03 equiv), CuI (4.8 mg, 0.025 mmol, 0.1 equiv), and dpca (3.7 mg, 0.01 mmol, 0.04 equiv) was combined and added to the reaction flask, followed by addition of 4-iodobenzonitrile (56.8 g, 0.248 mmol, 1.0 equiv). The obtained reaction mixture was stirred vigorously at rt. Care should be taken so that all reactants are submerged in THF and no solid is deposited on the side of the flask. After 2 h, the reaction mixture was quenched with sat. aq. NH₄Cl (5 mL), followed by addition of d.i. H₂O (5 mL). The organic layer was collected and the aqueous layer was extracted with Et₂O (2 x 10 mL). The combined organic layers were washed with brine, dried with MgSO₄, and concentrated *in vacuo* to 1-2 mL in volume. The obtained concentrated solution was added dropwise into a vigorously stirred solution of CH₃CN (250 mL). The precipitated polymer was filtered and the supernatant was concentrated *in vacuo* to provide the crude product. Following removal from the supernatant, the polymer was re-dissolved in DCM (20 mL) and filter through a glass fritted funnel to remove insoluble particles, if there is any. The obtained solution was then concentrated *in vacuo* to provide the recovered polymer (192 mg, 99 % recovery). The crude product was purified by chromatography on SiO₂ (2 % Et₂O/hexanes) to afford 4-cyanobiphenyl (40.3 mg, 0.225 mmol, 91 %) as a colorless solid.

### General Cross-Coupling Reaction Procedure:

To a cooled solution of siloxane (117 mg, 0.805 mmol) in dry THF (1.0 mL) at - 78 °C was added a solution of PhLi in Bu₂O (0.67 mmol). The reaction mixture was allowed to warm to room temperature and was stirred for 1 h. After 30 min had elapsed following PhLi addition, in a separate flask were combined PdCl₂ (2.4 mg, 0.013 mmol), CuI (8.6 mg, 0.045 mmol) and dpca (6.8 mg, 0.018 mmol) in dry THF (1.0 mL) at room temperature and stirred for 30 min. The aryl halide (0.45 mmol) was added to the orange slurry, followed by cannulation of the siloxane/PhLi reaction mixture (flask rinsed with 0.5 mL THF). After 2 h at room temperature, the reaction mixture was diluted with Et₂O (2 mL), quenched with sat. aq. NH₄Cl (5 mL) and extracted with Et₂O (3 x 5 mL). The combined organic layers were dried (MgSO₄), filtered and concentrated under reduced pressure. The cross-coupled product was purified using column chromatography on SiO₂.

### Siloxane-mediated cross-coupling of 4-chloroanisole and phenyllithium using palladium

To a small pear-shaped flask containing a stirring solution of siloxane **2a** (223.6 mg, 0.5 mmol, 1.8 equiv) and THF (0.35 mL) at -78 °C under inert atmosphere, was added PhLi (1.75 M in dibutyl ether, 0.43 mL, 0.75 mmol, 1.5 equiv) dropwise via syringe. The resulting yellow solution (viscous when cold) was allowed to reach room temperature and stirred for 2 h (nucleophile solution). Another flask containing Pd(OAc)₂ (11.2 mg, 0.05 mmol, 10 mol %) and XPhos (47.7 mg, 0.1 mmol, 20 mol %) under nitrogen atmosphere was charged with THF (2 mL). This mixture was stirred at room temperature for 20 minutes, turning dark red (catalyst solution). Then, to a round-bottomed flask containing 4-chloroanisole (71.0 mg, 0.5 mmol, 1.0 equiv) under nitrogen atmosphere, were added sequentially catalyst solution and nucleophile solution via syringe and the resulting mixture was stirred at room temperature overnight. The solution was concentrated *in vacuo* and filtered through a short plug of silica gel eluting with ether. The filtrate was again concentrated *in vacuo* and subjected to column chromatography eluting with ether/hexanes (0-5%) to afford 4-methoxy-biphenyl (69 mg, 75% yield) with 87% purity as determined by ¹H-NMR. Impurities include: biphenyl and residual siloxane.

### Alternative procedure for siloxane-mediated cross-coupling of 4-chloroanisole and phenyllithium.

To a small pear-shaped flask containing a stirring solution of siloxane **2a** (447.2 mg, 1.8 mmol, 1.8 equiv) and THF (0.7 mL) at -78 °C under inert atmosphere, was added PhLi (1.75 M in dibutyl ether, 0.86 mL, 1.5 mmol, 1.5 equiv) dropwise via syringe. The resulting yellow solution (viscous when cold) was allowed to reach room temperature and stirred for 2 h (nucleophile solution). Another flask containing Pd(OAc)₂ (22.4 mg, 0.1 mmol, 10 mol %) and XPhos (95.4 mg, 0.2 mmol, 20 mol %) under nitrogen atmosphere was charged with THF (2 mL). This mixture was stirred at room temperature for 20 minutes, turning dark red (catalyst solution). Then, to a round-bottomed flask containing 4-chloroanisole (142.6 mg, 1.0 mmol, 1.0 equiv) under nitrogen atmosphere, were added sequentially catalyst solution via syringe and nucleophile solution via cannula and the resulting mixture was stirred at room temperature overnight. The solution was concentrated *in vacuo* and the residue was directly subjected to column chromatography eluting with dichloromethane/hexanes (1-2%) to afford the product (127.1 mg, 69% yield).

### Carbon-nitrogen bond formation

### Reference Example

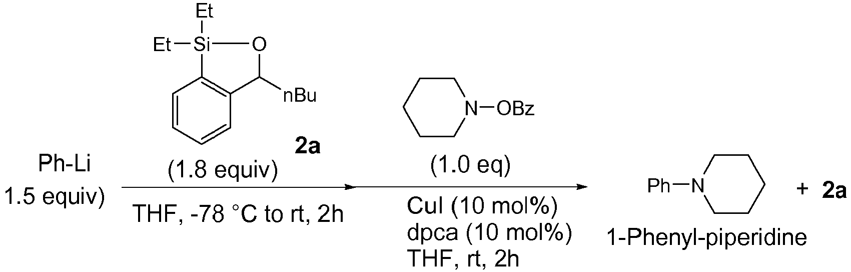

To a cooled solution of siloxane **2a** (112.5 mg, 0.453 mmol, 1.8 equiv) in 4 mL THF at -78 °C was added PhLi in Bu₂O (210 µL, 0.377 mmol, 1.8 M, 1.5 equiv), dropwise. The reaction mixture was allowed to warm to room temperature (rt) and was stirred for 2 h. A solid mixture of CuI (4.8 mg, 0.025 mmol, 0.1 equiv) and dpca (9.3 mg, 0.025 mmol, 0.1 equiv) was combined and added to the reaction flask, followed by addition of piperidines-1-yl benzoate (51.7 mg, 0.252 mmol, 1.0 equiv). The obtained reaction mixture was stirred at rt. After 2 h, the reaction mixture was quenched with sat. aq. NH₄Cl (5 mL), followed by addition of d.i. H₂O (5 ml). The organic layer was collected and the aqueous layer was extracted with Et₂O (2 x 10 mL). The combined organic layers were washed with brine, dried with MgSO₄, and concentrated in vacuo. Flash chromatography on silica gel (2% Et₂O/hexanes) afforded 1-phenyl-piperidine (37.7 mg, 0.234 mmol, 93%) as a colorless oil, and recovered siloxane **2a** (97.9 mg, 0.394 mmol, 87%).

### Polymer-mediated carbon-nitrogen cross-coupling

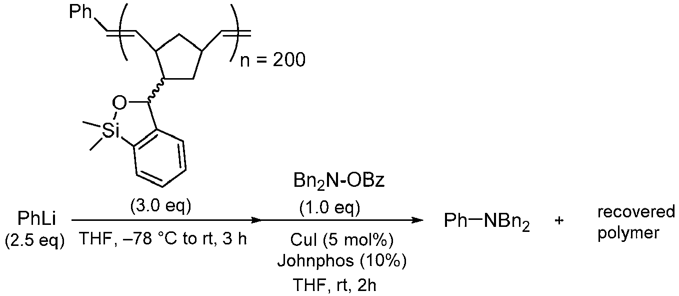

To a cooled solution of siloxane polymer in THF (0.131 g, 0.512 mmol, 3.0 equiv, 10 mg/mL) at -78 °C was added PhLi in Bu₂O (237 µL, 1.8 M, 0.627 mmol, 2.5 equiv) dropwise. The reaction mixture was allowed to warm to rt and was stirred for 3 h, and a white, cloudy solution developed. A solid mixture of CuI (1.6 mg, 0.00855 mmol, 0.05 equiv), and Johnphos (5.1 mg, 0.0171 mmol, 0.1 equiv) was combined and added to the reaction flask, followed by addition of O-benzoyl-N,N-dibenzylhydroxylamine (54.3 mg, 0.171 mmol, 1.0 equiv). The obtained reaction mixture was stirred vigorously at rt. Care should be taken so that all reactants are submerged in THF and no solid is deposited on the side of the flask. After 2 h, the reaction mixture was quenched with sat. aq. NH₄Cl (5 mL), followed by addition of d.i. H₂O (5 mL). The organic layer was collected and the aqueous layer was extracted with Et₂O (2 x 10 mL). The combined organic layers were washed with brine, dried with MgSO₄, and concentrated *in vacuo* to 1-2 mL in volume. The obtained concentrated solution was added dropwise into a vigorously stirred solution of CH₃CN (250 mL). The precipitated polymer was filtered and the supernatant was concentrated *in vacuo* to provide the crude product. Following removal from the supernatant, the polymer was re-dissolved in DCM (20 mL) and filter through a glass fritted funnel to remove insoluble particles, if there is any. The obtained solution was then concentrated *in vacuo* to provide the recovered polymer (114.2 mg, 87 % recovery). The crude product was purified by chromatography on SiO₂ (1 % Et₂O/ hexanes) to afford N,N-dibenzylaniline (40.8 mg, 0.149 mmol, 87 %) as a colorless solid.

### References:

¹ Nakao, Y.; Imanaka, H.; Chen, J.; Yada, A.; Hiyama, T. J. Organomet. Chem. 2007, 692, 585.
² Nakao, Y.; Takeda, M.; Matsumoto, T.; Hiyama, T. Angew. Chem. Int. Ed. 2010, 49, 4447.
³ Spino, C.; Gund, V. G.; Nadeau, C. J. Comb. Chem. 2005, 7, 345.
⁴ (a) Kunai, A.; Kawakami, T.; Toyoda, K.; Ishikawa, M. Organometallics, 1992, 11, 2708. (b) Kunai, A.; Ohshita, J. J. Orgnomet. Chem. 2003, 686, 3.
⁵ Huang, Z.; Negishi, E. Org. Lett. 2006, 8, 3675.
⁶ Wang, Z.; Denmark, S. E. Org. Synth. 2005, 81, 42.
⁷ Smith, A. B., III; Tong, R.; Kim, W.-S.; Maio, W. M, Angew. Chem. Int. Ed. 2011, 50, 8904.
⁸ Lin, S., Lu, X. J. Org. Chem. 2007, 72, 9757.
⁹ Bastug, G., Dierick, S., Lebreux, F., Marko, I. E. Org. Lett. 2012, 14, 1306.
¹⁰ Harrowven, D.C.; Sutton, B.J.; Coulton, S. Org. Biomol. Chem. 2003, 1, 4047.
¹¹ Manolikakes, G.; Knochel, P. Angew. Chem. Int. Ed. 2009, 48, 205.
¹² Kobayashi, O.; Uraguchi, D.; Yamakawa, T. Org. Lett. 2009, 11, 2679.
¹³ Seki, M.; Mori, K. Eur. J. Org. Chem. 1999, 2965.
¹⁴ Denmark, S. E.; Sweis, R. F. J. Am. Chem. Soc. 2001, 123, 6439.
¹⁵ Smith, A.B., III et al., J. Am. Chem. Soc. 2012, 134, 4533-4536.
¹⁶ Son, E.-C., e tal., Bull. Chem. Soc. Jpn. 2006, 79, 492.
¹⁷ Smith, A.B., III et al. Angew. Chem. Int. Ed. 2011, 50, 8904-8907.

## Claims

1. A compound of formula I: 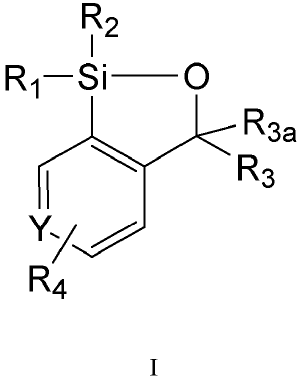 wherein
Y is CH or N;
R₁ and R₂ are independently methyl, propyl, or isopropyl, optionally wherein R₁ and R₂ are each methyl or isopropyl;
R₃ is
aryl substituted with one or more nitro, diC₁₋₆alkylamino, C₁₋₆alkoxy, or C₁₋₆alkyl;
heteroaryl optionally substituted with one or more nitro, diC₁₋₆alkylamino, C₁₋₆alkoxy, or C₁₋₆alkyl;
C₁₋₁₀ straight or branched-chain alkyl optionally substituted with one halogen, nitro, C₁₋₆alkoxy, or aryl;
a polymer; or
a resin support;
R₃ₐ is H or CF₃; and
at least one R₄, wherein each R₄ is independently hydrogen, halogen, nitro, C₁₋₆alkoxy, C₁₋₆alkyl, aryl, or a resin support;
and wherein R₃ is a polymer or a resin support, and/or R₄ is a resin support.

2. The compound of claim 1, wherein R₃ is C₁₋₆ or C₁₋₄ straight or branched-chain alkyl optionally substituted with one or more C₁₋₆alkoxy.

3. The compound of claim 1, wherein R₃ is C₁₋₄ straight or branched-chain alkyl, optionally wherein R₃ is n-butyl, isobutyl, or tert-butyl and preferably wherein R₃ is n-butyl.

4. The compound of claim 1, wherein R₃ is C₁₋₄ straight or branched-chain alkyl substituted with one or more halogen, optionally wherein R₃ is -CF₃.

5. The compound of claims 1, wherein R₃ is phenyl substituted with one or more nitro, diC₁₋₆alkyl amino, C₁₋₆alkoxy, or C₁₋₆alkyl.

6. The compound of claim 1, wherein R₃ is pyridyl or a polymer.

7. The compound of claim 1, wherein R₄ is hydrogen, C₁₋₆alkoxy, C₁₋₆alkyl, or aryl and preferably wherein R₄ is hydrogen.

8. The compound of any one of the preceding claims that is 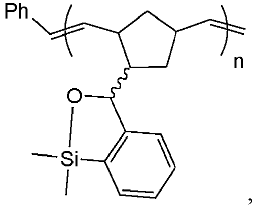 wherein n is about 150 to about 300, preferably about 200, 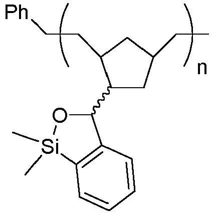 wherein n is 20 to 200,

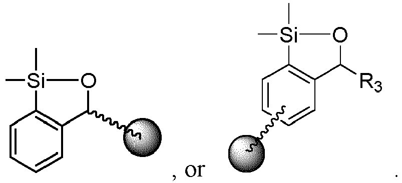

9. A method of cross-coupling a compound of formula NuLi with a compound of formula E-X to form a compound of formula Nu-E comprising
contacting the compound of formula NuLi with the compound of formula E-X in the presence of a compound choosen from the group comprising
a compound of formula I: 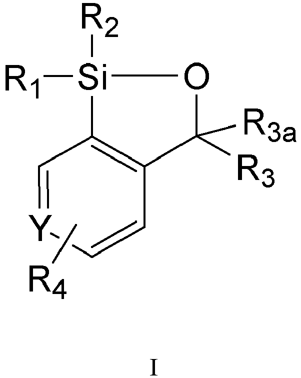 wherein
Y is CH or N;
R₁ and R₂ are independently C₁₋₁₀ straight or branched-chain alkyl optionally substituted with one or more halogen, nitro, C₁₋₆alkoxy, or aryl;
R₃ is H;
aryl optionally substituted with one or more nitro, diC₁₋₆alkylamino, C₁₋₆alkoxy, or C₁₋₆alkyl;
heteroaryl optionally substituted with one or more nitro, diC₁₋₆alkylamino, C₁₋₆alkoxy, or C₁₋₆alkyl;
C₁₋₁₀ straight or branched-chain alkyl optionally substituted with one or more halogen, nitro, C₁₋₆alkoxy, or aryl;
a polymer; or
a resin support;
R₃ₐ is H or C₁₋₆alkyl optionally substituted with one or more halogen; and
at least one R₄, wherein each R₄ is independently hydrogen, halogen, nitro, C₁₋₆alkoxy, C₁₋₆alkyl, aryl, or a resin support,
wherein R₃ is a polymer or a resin support, and/or R₄ is a resin support; catalyst or catalyst system, and an ethereal solvent;
for a time and under conditions sufficient to produce the compound of formula Nu-E; wherein Nu is an aryl compound or an alkenyl compound; and wherein E is an aryl compound or an alkenyl compound and X is iodo, chloro, or bromo, or wherein E is a disubstituted amine and X is -O-benzoyl.

10. The method of claim 9, wherein the solvent is tetrahydrofuran.

11. The method of claim 9, wherein the catalyst system comprises palladium, copper, or a mixture thereof, preferably wherein the catalyst system comprises PdCl₂, dpca, and CuI, dpca and CuI, CuI and Johnphos or Pd(OAc)₂ and XPhos.

12. The method of any one of claims 9 to 11, wherein the compound of formula 1 is 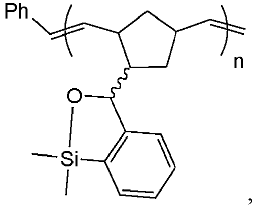 wherein n is about 150 to about 300, preferably about 200, 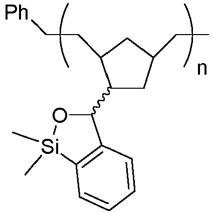 wherein n is 20 to 200,

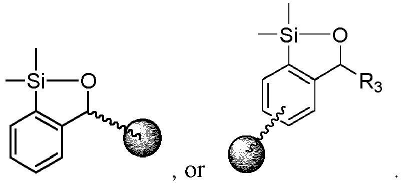

## Patentansprüche

1. Verbindung der Formel I: 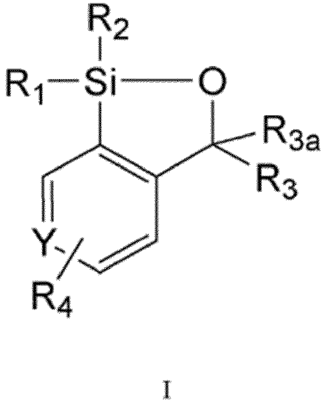 wobei
Y CH oder N ist,
R₁ und R₂ unabhängig Methyl, Propyl oder Isopropyl sind, wobei wahlweise R₁ und R₂ jedes Methyl oder Isopropyl ist,
R₃
Aryl, das mit einem oder mehreren Nitro, Di-C₁₋₆-Alkylamino, C₁₋₆-Alkoxy oder C₁-₆-Alkyl substituiert ist;
Heteroaryl, das mit einem oder mehreren Nitro, Di-C₁-₆-Alkylamino, C₁₋₆-Alkoxy oder C₁-₆-Alkyl substituiert ist;,
gerad- oder verzweigtkettiges C₁-₁₀-Alkyl, das wahlweise mit einem Halogen, Nitro, C₁₋₆-Alkoxy oder Aryl substituiert ist;
ein Polymer; oder
ein Harzträger ist,
R₃ₐ H oder CF₃ und
mindestens ein R₄ ist, wobei jedes R₄ unabhängig Wasserstoff, Halogen, Nitro, C₁-₆-Alkoxy, C₁-₆-Alkyl, Aryl oder ein Harzträger ist,
und wobei R₃ ein Polymer oder ein Harzträger ist; und/oder R₄ ein Harzträger ist.

2. Verbindung nach Anspruch 1, wobei R₃ ein gerad- oder verzweigtkettiges C₁-₆- oder C₁-₄-Alkyl ist, das wahlweise mit einem oder mehreren C₁-₆-Alkoxy substituiert ist.

3. Verbindung nach Anspruch 1, wobei R₃ ein gerad- oder verzweigtkettiges C₁-₄-Alkyl ist, wobei wahlweise R₃ n-Butyl, Isobutyl oder tert-Butyl ist und wobei R₃ bevorzugt n-Butyl ist.

4. Verbindung nach Anspruch 1, wobei R₃ ein gerad- oder verzweigtkettiges C₁-₄-Alkyl ist, das mit einem oder mehreren Halogenen substituiert ist, wobei wahlweise R₃ -CF₃ ist.

5. Verbindung nach Anspruch 1, wobei R₃ Phenyl ist, das mit einem oder mehreren Nitro, Di-C₁-₆-Alkylamino, C₁-₆-Alkoxy oder C₁-₆-Alkyl substituiert ist

6. Verbindung nach Anspruch 1, wobei R₃ Pyridyl oder ein Polymer ist.

7. Verbindung nach Anspruch 1, wobei R₄
Wasserstoff, C₁₋₆-Alkoxy, C₁-₆-Alkyl oder Aryl ist und wobei R₄ bevorzugt Wasserstoff ist.

8. Verbindung nach einem der vorhergehenden Ansprüche, die 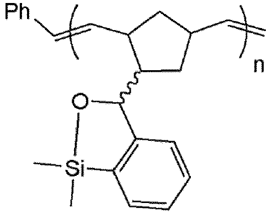 ist, wobei n etwa 150 bis etwa 300, bevorzugt etwa 200 beträgt, 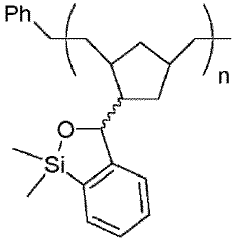 wobei n 20 bis 200 beträgt

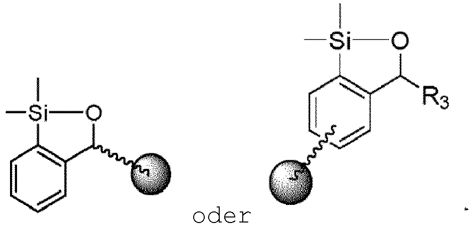

9. Verfahren zum Quervernetzen einer Verbindung der Formel NuLi mit einer Verbindung der Formel E-X, um eine Verbindung der Formel Nu-E zu bilden, umfassend
Kontaktieren der Verbindung der Formel NuLi mit der Verbindung der Formel E-X in Gegenwart einer Verbindung ausgewählt aus der Gruppe umfassend eine Verbindung der Formel I: 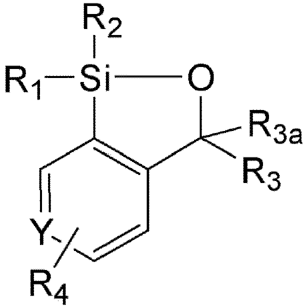 wobei
Y CH oder N ist,
R₁ und R₂ unabhängig gerad- oder verzweigtkettiges C₁-₁₀-Alkyl sind, das wahlweise mit einem oder mehreren von Halogen, Nitro, C₁₋₆-Alkoxy oder Aryl substituiert ist;
R₃ H;
Aryl, das wahlweise mit einem oder mehreren Nitro, Di-C₁-₆-Alkylamino, C₁₋₆-Alkoxy oder C₁-₆-Alkyl substituiert ist;
Heteroaryl, das wahlweise mit einem oder mehreren Nitro, Di-C₁-₆-Alkylamino, C₁₋₆-Alkoxy oder C₁-₆-Alkyl substituiert ist;,
gerad- oder verzweigtkettiges C₁-₁₀-Alkyl, das wahlweise mit einem oder mehreren Halogen, Nitro, C₁-₆-Alkoxy oder Aryl substituiert ist;
ein Polymer; oder
ein Harzträger ist,
R₃ₐ H oder C₁-₆-Alkyl ist, das wahlweise mit einem oder mehreren Halogenen substituiert ist; und
mindestens ein R₄ ist, wobei jedes R₄ unabhängig Wasserstoff, Halogen, Nitro, C₁-₆-Alkoxy, C₁-₆-Alkyl, Aryl oder ein Harzträger ist,
wobei R₃ ein Polymer oder ein Harzträger ist; und/oder R₄ ein Harzträger ist; eines Katalysators oder Katalysatorsystems und eines ätherischen Lösungsmittels;
für eine Zeitspanne und unter Bedingungen, die zum Herstellen der Verbindung der Formel Nu-E ausreichen;
wobei Nu eine Arylverbindung oder eine Alkenylverbindung ist; und wobei E eine Arylverbindung oder eine Alkenylverbindung ist und X Iod, Chlor oder Brom ist oder wobei E ein disubstituiertes Amin ist und X -O-Benzoyl ist.

10. Verfahren nach Anspruch 9, wobei das Lösungsmittel Tetrahydrofuran ist.

11. Verfahren nach Anspruch 9, wobei das Katalysatorsystem Palladium, Kupfer oder eine Mischung davon umfasst, wobei bevorzugt das Katalysatorsystem PdCl₂, dpca und CuI, dpca und CuI, CuI und Johnphos oder Pd(OAc)₂ und XPhos umfasst.

12. Verfahren nach einem der Ansprüche 9 bis 11, wobei die Verbindung der Formel 1 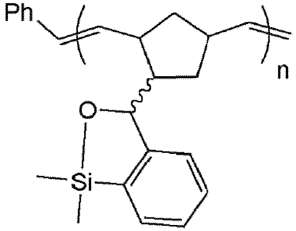 ist, wobei n etwa 150 bis etwa 300, bevorzugt etwa 200 beträgt 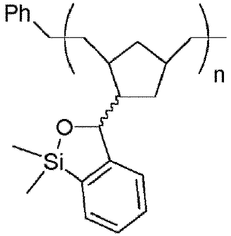 wobei n 20 bis 200 beträgt,

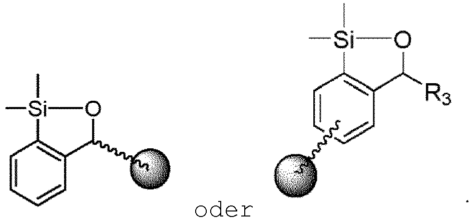

## Revendications

1. Composé de formule I : 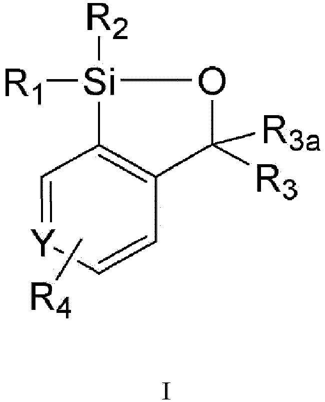 dans lequel
Y est CH ou N ;
R₁ et R₂ sont indépendamment un méthyle, un propyle, ou un isopropyle, facultativement dans lequel R₁ et R₂ sont chacun un méthyle ou un isopropyle ;
R₃ est
un aryle substitué par un ou plusieurs nitro, di(alkyl en C₁-₆) amino, alcoxy en C₁-₆, ou alkyle en C₁-₆ ;
un hétéroaryle facultativement substitué par un ou plusieurs nitro, di (alkyl en C₁-₆) amino, alcoxy en C₁-₆, ou alkyle en C₁-₆ ;
un alkyle en C₁₋₁₀ linéaire ou ramifié facultativement substitué par un halogène, un nitro, un alcoxy en C₁-₆, ou un aryle ;
un polymère ; ou
un support de résine ;
R₃ₐ est H ou CF₃ ; et
au moins un R₄, dans lequel chaque R₄ est indépendamment un hydrogène, un halogène, un nitro, un alcoxy en C₁-₆, un alkyle en C₁-₆, un aryle, ou un support de résine ;
et dans lequel R₃ est un polymère ou un support de résine, et/ou R₄ est un support de résine.

2. Composé selon la revendication 1, dans lequel R₃ est un alkyle linéaire ou ramifié en C₁-₆ ou C₁-₄ facultativement substitué par un ou plusieurs alcoxy en C₁-₆.

3. Composé selon la revendication 1, dans lequel R₃ est un alkyle linéaire ou ramifié en C₁-₄, facultativement dans lequel R₃ est un n-butyle, un isobutyle, ou un tert-butyle et de préférence dans lequel R₃ est un n-butyle.

4. Composé selon la revendication 1, dans lequel R₃ est un alkyle linéaire ou ramifié en C₁-₄ substitué par un ou plusieurs halogènes, facultativement dans lequel R₃ est -CF₃.

5. Composé selon la revendication 1, dans lequel R₃ est un phényl substitué par un ou plusieurs nitro, di(alkyl en C₁₋₆) amino, alcoxy en C₁-₆, ou alkyle en C₁-₆.

6. Composé selon la revendication 1, dans lequel R₃ est un pyridyle ou un polymère.

7. Composé selon la revendication 1, dans lequel R₄ est un hydrogène, un alcoxy en C₁-₆, un alkyle en C₁-₆, ou un aryle et de préférence dans lequel R₄ est un hydrogène.

8. Composé selon l'une quelconque des revendications précédentes qui est 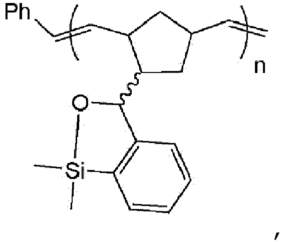 dans lequel n est d'environ 150 à environ 300, de préférence d'environ 200, 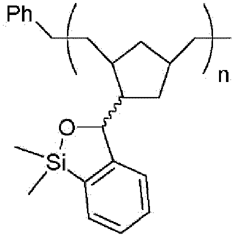 dans lequel n est de 20 à 200,

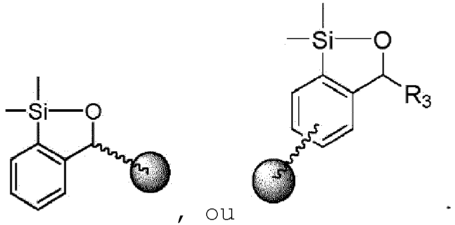

9. Procédé de couplage croisé d'un composé de formule NuLi avec un composé de formule E-X pour former un composé de formule Nu-E comprenant
la mise en contact du composé de formule NuLi avec le composé de formule E-X en présence d'un composé choisi dans le groupe comprenant un composé de formule I : 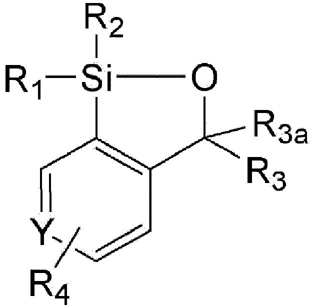 dans lequel
Y est CH ou N ;
R₁ et R₂ sont indépendamment un alkyle en C₁₋₁₀ linéaire ou ramifié facultativement substitué par un ou plusieurs halogène, nitro, alcoxy en C₁-₆ ou aryle ;
R₃ est H ;
un aryle facultativement substitué par un ou plusieurs nitro, di (alkyl en C₁-₆) amino, alcoxy en C₁-₆, ou alkyle en C₁₋₆ ;
un hétéroaryle facultativement substitué par un ou plusieurs nitro, di (alkyl en C₁-₆) amino, alcoxy en C₁-₆, ou alkyle en C₁₋₆ ;
un alkyle en C₁₋₁₀ linéaire ou ramifié facultativement substitué par un ou plusieurs halogène, nitro, alcoxy en C₁-₆, ou aryle ;
un polymère ; ou
un support de résine ;
R₃ₐ est H ou un alkyle en C₁-₆ facultativement substitué par un ou plusieurs halogènes ; et
au moins un R₄, dans lequel chaque R₄ est indépendamment un hydrogène, un halogène, un nitro, un alcoxy en C₁-₆, un alkyle en C₁-₆, un aryle, ou un support de résine ;
dans lequel R₃ est un polymère ou un support de résine, et/ou R₄ est un support de résine ; un catalyseur ou un système catalytique, et un solvant éthéré ;
pour une durée et dans des conditions suffisantes pour produire le composé de formule Nu-E ; dans lequel Nu est un composé aryle ou un composé alcényle ; et dans lequel E est un composé aryle ou un composé alcényle et X est un iodo, un chloro, ou un bromo, ou dans lequel E est une amine disubstituée et X est un -O-benzoyle.

10. Procédé selon la revendication 9, dans lequel le solvant est le tétrahydrofurane.

11. Procédé selon la revendication 9, dans lequel le système catalytique comprend du palladium, du cuivre, ou un mélange de ceux-ci, de préférence dans lequel le système de catalytique comprend les PdCl₂, dpca, et CuI, dpca et CuI, CuI et Johnphos ou Pd(OAc)₂ et XPhos.

12. Procédé selon l'une quelconque des revendications 9 à 11, dans lequel le composé de formule 1 est 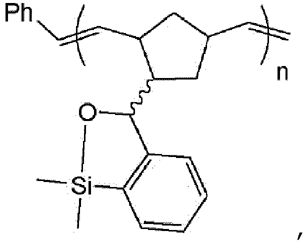 dans lequel n est d'environ 150 à environ 300, de préférence d'environ 200, 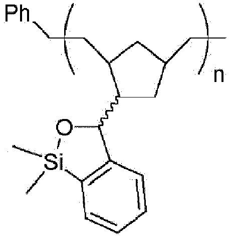 dans lequel n est de 20 à 200,
